# EUROPEAN PATENT SPECIFICATION

(11) **EP 0 321 274 B1**
(45) Date of publication and mention of the grant of the patent: **30.03.1994**
(21) Application number: 88311927.3
(22) Date of filing: 16.12.1988
(51) Int. Cl.: C07D 213/59, C07D 213/61, C07D 215/14, C07D 217/16, C07D 239/26, C07D 401/12, C07D 213/50, C07D 213/53, C07D 215/12, A61K 31/44, A61K 31/47

(54) **Derivatives of thioformamide**
Thioformamidderivate
Dérivés du thioformamide

(30) Priority: 18.12.1987 GB 8729521
(43) Date of publication of application: 21.06.1989
(73) Proprietor: RHONE-POULENC RORER LIMITED, West Malling, Kent ME19 4TA (GB)
(72) Inventor: Cook, David Charles, Dagenham Essex, RM10 7XS (GB); Hart, Terance William, Dagenham Essex, RM10 7XS (GB); McLay, Iain McFarlane, Dagenham Essex, RM10 7XS (GB); Palfreyman, Malcolm Norman, Dagenham Essex, RM10 7XS (GB); Walsh, Roger John Aitchison, Dagenham Essex, RM10 7XS (GB)
(74) Representative: Bentham, Stephen

(56) References cited:
- GB-A- 1 351 024
- CHEMICAL ABSTRACTS, vol. 104, no. 5, February 3, 1986, Columbus, Ohio, USA; H. SINGH et al.: "Synthesis of N-allyl-2-oxo-cycloalkanecarbothioamides", page 520, column 2, abstract-no. 33 782a

## Description

This invention relates to new therapeutically useful thioformamide derivatives, to processes for their preparation and to pharmaceutical compositions containing them.

The new thioformamide derivatives of the present invention are those compounds of the general formula (I) hereinafter depicted, wherein R represents a straight- or branched-chain alkyl radical containing from 1 to 4 carbon atoms, Het represents an aromatic heterocyclic radical containing one or two nitrogen atoms (optionally substituted by a straight- or branched-chain alkyl or alkoxy radical containing 1 to 4 carbon atoms or by a halogen atom), selected from pyrid-3-yl, isoquinolin-4-yl, tetrahydroquinolin-3-yl, quinolin-3-yl, pyridazin-4-yl, pyrimid-5-yl, thiazol-5-yl, thieno[2,3-b]pyridin-5-yl, pyrazin-2-yl, indol-3-yl and thieno[3,2-b]pyridin-6-yl, Y represents an ethylene or preferably methylene radical, or preferably a valency bond, and X represents a carbonyl or hydroxymethylene group or a group of the formula:
〉C=NOR¹, 〉C=NN(R¹)₂ or 〉C=NN(R¹)CON(R¹)₂ in which the symbols R¹, which may be the same or different, each represents the hydrogen atom or preferably a straight- or branched-chain alkyl radical containing from 1 to 4 carbon atoms which is unsubstituted or substituted by one or more substituents selected from C₂₋₄-alkenyl, carboxy, C₂₋₅-alkoxycarbonyl, hydroxy, C₁₋₄-alkoxy, carbamoyl (unsubstituted or substituted by one or two C₁₋₄-alkyl groups), amino, C₁₋₄-alkylamino and di-C₁₋₄-alkylamino groups (e.g. R¹ may represent a t.butyl or 2,3-dihydroxypropyl radical), or preferably represents a benzyl, phenethyl, 1-naphthylmethyl, 2-naphthylmethyl or pyrid-3-ylmethyl radical each of which may be substituted on the ring by one or more halogen atoms or hydroxy, C₁₋₄-alkyl, C₁₋₄-alkoxy (alkoxy being unsubstituted or substituted as defined for alkyl groups represented by R¹), cyano, nitro, trifluoromethyl, carboxy, C₁₋₄-alkylamino, C₂₋₅-alkanoylamino or C₂₋₅-alkoxycarbonyl groups or two R¹ substituents on the same nitrogen atom may together form a straight or branched chain alkylene radical containing from 4 to 6 carbon atoms in the chain which is unsubstituted or substituted as defined for alkyl radicals represented by R¹ (e.g. 1-methoxymethyltetramethylene) and pharmaceutically acceptable salts thereof.

Preferably X represents the carbonyl group or a group of formula 〉C=NOR¹ as hereinbefore defined.

Preferably Het represents 3-pyridyl, 6-chloropyrid-3-yl, 5-bromopyrid-3-yl, 3-quinolinyl, 4-isoquinolinyl or 5-pyrimidyl.

The presence of a hydroxy group on the ring creates an asymmetry in the molecule which, in association with the adjacent asymmetric carbon atom, leads to 4 stereoisomers which, optionally, can be separated into 2 racemic pairs. The racemic pair and its enantiomers of the general formula (II) in which R, Het and Y are as hereinbefore defined, i.e. the compounds in which the hydroxy group is in the trans position relative to the group -CSNHR are preferred.

Furthermore, in certain cases the substituents R and R¹ contribute to stereoisomerism. All such forms are embraced by the present invention.

Particular compounds of the present invention are as follows:-
A (±)-N-methyl-2-oxo-1-(3-pyridyl)cyclohexanecarbothioamide
B (±)-N-methyl-2-oxo-1-(3-quinolinyl)cyclohexanecarbothioamide
C (±)-N-methyl-2-oxo-1-(3-pyridyl)cycloheptanecarbothioamide
D (±)-trans-N-methyl-2-hydroxy-1-(3-pyridyl)cyclohexanecarbothioamide
E (±)-cis-N-methyl-2-hydroxy-1-(3-pyridyl)cyclohexanecarbothioamide
F (±)-anti-N-methyl-2-hydroximino-1-(3-pyridyl)cyclohexanecarbothioamide
G (±)-anti-N-methyl-2-methoxyimino-1-(3-pyridyl)cyclohexanecarbothioamide
H (±)-anti-N-methyl-2-benzyloxyimino-1-(3-pyridyl)cyclohexanecarbothioamide
I (±)-anti-N-methyl-2-hydroxyimino-1-(3-quinolinyl)cyclohexanecarbothioamide
J (2S)-anti-2-(methoxymethyl)-1-[2'-(3-quinolinyl)-2'-methylthiocarbamoylcyclohexylideneamino]pyrrolidine
K (2S)-anti-2-(methoxymethyl)-1-[2'-(3-pyridyl)-2'-methylthiocarbamoylcyclohexylideneamino]pyrrolidine,
L (±)-anti-N-methyl-2-(2-dimethylaminoethoxyimino)-1-(3-pyridyl)cyclohexanecarbothioamide,
M (±)-anti-N-methyl-2-(2-aminoethoxyimino)-1-(3-pyridyl) cyclohexanecarbothioamide
N (±)-anti-N-methyl-2-methoxycarbonylmethoxyimino-1-(3-pyridyl)cyclohexanecarbothioamide
O (±)-anti-N-methyl-2-carbamoylmethoxyimino-1-(3-pyridyl)cyclohexanecarbothioamide,
P (±)-anti-N-methyl-2-(2,3-dihydroxypropoxyimino)-1-(3-pyridyl)cyclohexanecarbothioamide
Q (±)-anti-N-methyl-2-(2-hydroxyethoxyimino)-1-(3-pyridyl)cyclohexanecarbothioamide
R (±)-N-methyl-2-oxo-1-(6-chloropyrid-3-yl)cyclohexanecarbothioamide
S (±)-N-methyl-2-oxo-1-(5-bromopyrid-3-yl)cyclohexanecarbothioamide
T (±)-N-ethyl-2-oxo-1-(3-pyridyl)cyclohexanecarbothioamide
U (±)-N-ethyl-2-oxo-1-(3-quinolinyl)cyclohexanecarbothioamide
V (±)-anti-N-methyl-2-dimethylhydrazono-1-(3-quinolinyl)cyclohexanecarbothioamide
W (±)-anti-N-methyl-2-diethylhydrazono-1-(3-quinolinyl) cyclohexanecarbothioamide
X (±)-anti-N-methyl-2-benzyloxyimino-1-(3-quinolinyl)cyclohexanecarbothioamide
Y (±)-anti-N-methyl-2-methoxyimino-1-(3-quinolinyl)cyclohexanecarbothioamide
Z (±)-anti-N-methyl-2-ethoxyimino-1-(3-quinolinyl)cyclohexanecarbothioamide
AA (±)-anti-N-methyl-2-butoxyimino-1-(3-quinolinyl)cyclohexanecarbothioamide
AB (±)-anti-N-methyl-2-(3-isopropylamino-2-hydroxypropoxyimino)-1-(3-quinolinyl)cyclohexanecarbothioamide
AC (±)-anti-N-methyl-2-t.butoxyimino-1-(3-quinolinyl)cyclohexanecarbothioamide
AD (±)-anti-N-methyl-2-prop-2-enoxyimino-1-(3-quinolinyl)cyclohexanecarbothioamide
AE (±)-anti-N-methyl-2-naphth-2-ylmethoxyimino-1-(3-quinolinyl)cyclohexanecarbothioamide
AF (±)-anti-N-methyl-2-phenethyloxyimino-1-(3-quinolinyl)cyclohexanecarbothioamide
AG (±)-anti-N-methyl-2-naphth-1-ylmethoxyimino-1-(3-quinolinyl)cyclohexanecarbothioamide
AH (±)-anti-N-methyl-2-(3-t.butylamino-2-hydroxypropoxyimino-1-(3-quinolinyl)cyclohexanecarbothioamide
AI (±)-anti-N-methyl-2-isopropoxyimino-1-(3-quinolinyl)cyclohexanecarbothioamide
AJ (±)-anti-N-methyl-2-(4-hydroxybenzyloxyimino-1-(3-quinolinyl)cyclohexanecarbothioamide
AK (±)-anti-N-methyl-2-(4-fluorobenzyloxyimino)-1-(3-quinolinyl)cyclohexanecarbothioamide
AL (±)-anti-N-methyl-2-(4-fluorobenzyloxyimino)-1-(3-pyridyl)cyclohexanecarbothioamide
AM (±)-anti-N-methyl-2-(2,3,4,5,6-pentafluorobenzyloxyimino)-1-(3-pyridyl)cyclohexanecarbothioamide
AN (±)-anti-N-methyl-2-isopropoxyimino-1-(3-pyridyl)cyclohexanecarbothioamide
AO (±)-anti-N-methyl-2-t.butoxyimino-1-(3-pyridyl)cyclohexanecarbothioamide
AP (±)-anti-N-methyl-2-[4-(3-t.butylamino-2-hydroxypropoxy)benzyloxyimino]-1-(3-pyridyl)cyclohexanecarbothioamide
AQ (±)-anti-N-methyl-2-(4-hydroxybenzyloxyimino)-1-(3-pyridyl)cyclohexanecarbothioamide
AR (±)-anti-N-methyl-2-(3-fluorobenzyloxyimino)-1-(3-pyridyl)cyclohexanecarbothioamide
AS (±)-anti-N-methyl-2-(2-fluorobenzyloxyimino)-1-(3-pyridyl)cyclohexanecarbothioamide
AT (±)-anti-N-methyl-2-(3-pyridylmethoxyimino)-1-(3-pyridyl)cyclohexanecarbothioamide
AU (±)-anti-N-methyl-2-(4-nitrobenzyloxyimino)-1-(3-pyridyl)cyclohexanecarbothioamide,
AV (±)-anti-N-methyl-2-(4-cyanobenzyloxyimino)-1-(3-pyridyl)cyclohexanecarbothioamide
AW (±)-anti-N-methyl-2-(3,4-difluorobenzyloxyimino)-1-(3-pyridyl)cyclohexanecarbothioamide
AX (±)-anti-N-methyl-2-(4-methoxybenzyloxyimino)-1-(3-pyridyl)cyclohexanecarbothioamide
AY (±)-anti-N-methyl-2-(2-trifluoromethylbenzyloxyimino)-1-(3-pyridyl)cyclohexanecarbothioamide
AZ (±)-N-methyl-2-oxo-1-(4-isoquinolinyl)cyclohexanecarbothioamide
BA (±)-anti-N-methyl-2-benzyloxyimino-1-(4-isoquinolinyl)-cyclohexanecarbothioamide
BB (±)-N-methyl-2-oxo-1-(5-pyrimidyl)cyclohexanecarbothioamide
BC (±)-N-methyl-2-oxo-1-(3-pyridyl)cyclopentanecarbothioamide
BD (±)-anti-N-methyl-2-(4-fluorobenzyloxyimino)-1-(3-pyridyl)cyclopentanecarbothioamide
BE (±)-anti-N-methyl-2-methoxyimino-1-(3-pyridyl)cyclopentanecarbothioamide
BF (±)-anti-N-methyl-2-benzyloxyimino-1-(3-pyridyl)cyclopentanecarbothioamide.
as well as their enantiomeric and diastereoisomeric and syn forms, where they exist.

The letters A to BF are allocated to the compounds for easy reference later in the specification, e.g. in the Table and in the Examples.

The compounds have valuable pharmacological properties, in particular properties which are indicative of utility in the treatment and/or prophylaxis of disorders associated with:-
(1) vascular smooth muscle contraction including hypertension and other cardiovascular disorders such as congestive heart failure, and conditions associated with tissue ischaemia such as angina, peripheral vascular disease and cerebrovascular disease;
(2) respiratory smooth muscle contraction including reversible airways obstruction and asthma;
(3) contraction of smooth muscle of gastro- intestinal tract, urinary bladder and uterus, including peptic ulcers, irritable bowel syndrome and diverticular disease; irritable bladder syndrome; and premature labour.

The compounds also have utility in the inhibition of head hair loss associated with male pattern baldness, by topical application.

For example, compounds of general formula (I) were submitted to:-

### Vaso-relaxant Activity Tests.

The test methods used were adapted from those described by Winslow et al [Eur.J.Pharmacol., 131, 219-228 (1986)] and Karaki [J.Pharmacol. Methods, 18, 1-21 (1987)] for differentiating vaso-relaxant activity.

### Test A : Activity against contractions induced by low K⁺ concentrations in the isolated rat aorta

Thoracic aorta was removed from rats and transverse strips, denuded of endothelium, were suspended in a bath containing Krebs solution. The tension was recorded and a contraction induced by addition of 20 mM K⁺ (potassium ion) to the bathing solution. The test compound was added to the bath as a solution in increasing cumulative concentration. The concentration in the bathing solution of the test compound which reduced the K⁺-induced contraction by 90% was determined and expressed in µM as the effective concentration (EC₉₀), given in Table I.

### Test B : Activity against contractions induced by high K⁺ concentrations in isolated rat aorta

The test method was as in Test A except that contractions were induced by addition of 60 mM K⁺ to the bathing solution. The cumulative addition of solutions of the test compound was conducted and the concentration in the bath reducing the K⁺-induced contraction by 90% was greater than 30µM for Compounds A, B and T, and much greater than 30µM for Compounds D, E and G.

**Table I**

| Compound | Activity Test A EC₉₀ µM |
|---|---|
| A | 0.8 |
| B | 0.07 |
| D | 7.5 |
| E | 24 |
| G | 0.3 |
| H | 0.01 |
| J | 0.3 |
| R | 0.24 |
| S | 0.4 |
| T | 0.3 |

In in vivo tests in the anaesthetised guinea pig, performed according to the method of Dixon W.E. and Brodie T.G., J. Physiol 29, pp 97-173 (1903), Compound BD affected histamine-induced bronchospasm and blood pressure as follows:-

| | | % maximum bronchospasm | B.P. | |
|---|---|---|---|---|
| | | | kPa | (mmHg) |
| | Control | 80 | 5.6 | (42) |
| Compound BD | 100µg/ml | 76 | 6.5 | (49) |
| | 300µg/ml | 46 | 5.7 | (43) |
| | 1000µg/ml | 22 | 5.3 | (40) |

A nebulised aerosol generated from an aqueous solution having a given concentration was administered for 1 minute to the anaesthetised guinea pigs.

The compounds of general formula (I) can be prepared by the application or adaptation of known methods, for example as hereinafter identified. By the term "known methods" as used in this specification is meant methods heretofore used or described in the literature.

According to a feature of the present invention, the compounds of general formula (I) wherein X represents the carbonyl group or a group of formula 〉C=NOR¹ or 〉C=NN(R¹)₂ wherein R¹ is as hereinbefore defined may be prepared by the reaction of a compound of general formula (III) wherein X' represents the carbonyl group or a group of formula 〉C=NOR¹ or 〉C=NN(R¹)₂ as hereinbefore defined and Het and Y are as hereinbefore defined with an isothiocyanate of the general formula:

R-N=C=S (IV)

wherein R represents a straight- or branched-chain alkyl radical containing 1 to 4 carbon atoms.

The reaction is generally carried out in an anhydrous inert organic solvent such as tetrahydrofuran, dimethylformamide or hexamethylphosphoramide, or a mixture of these solvents, at a temperature from -80°C to +50°C, in the presence of an organic base such as potassium tert.-butoxide or an organo-lithium derivative such as butyllithium, or of sodium hydride.

According to a feature of the present invention, the compounds of general formula (I) wherein X represents the hydroxymethylene group may be prepared by the reduction of a compound of general formula (I) wherein X represents the carbonyl group.

The reduction is generally carried out in an inert organic solvent such as methanol or dimethylsulphoxide, or a mixture of these solvents at a temperature from -20°C to +50°C, using an alkali metal borohydride, e.g. sodium borohydride.

According to a feature of the present invention, the compounds of general formula (I) wherein X represents a group of the formula: 〉C=NOR¹, 〉C=NN(R¹)₂ or 〉C=NN(R¹)CON(R¹)₂ as hereinbefore defined may be prepared by the reaction of a compound of general formula (I) wherein X represents the carbonyl group with a compound of the general formula:

NH₂OR¹ (Va)

NH₂N(R¹)₂ (Vb)

or

NH₂N(R¹)CON(R¹)₂ (Vc)

wherein R¹ is as hereinbefore defined or with an acid addition salt (preferably the hydrochloride) thereof.

The reaction is generally carried out in the presence of an inorganic base, e.g. sodium carbonate or sodium acetate in an inert organic solvent, e.g. ethanol, or an organic base, e.g. pyridine, which may serve as the solvent, in an inert organic solvent at a temperature from 0°C to 120°C.

A stereoselective synthesis may be performed for example as hereinafter described in Reference Examples in which a mixture of enantiomers of general formula (III) wherein X' represents the carbonyl group is reacted with a chiral auxiliary agent, e.g. (S)-1-amino-2-methoxymethylpyrrolidine, before being reacted with a compound of general formula (IV) as hereinbefore described followed by the removal of the chiral auxiliary agent.

According to a feature of the invention, the thioformamide derivatives of general formula (I) wherein R represents the methyl radical and X represents the carbonyl group or a group of the formula 〉C=NOR¹ or 〉C=NN(R¹)₂ as hereinbefore defined may be prepared by the process which comprises reacting methylamine with a dithioester of the general formula (VI) wherein the symbols Het, X' and Y are as hereinbefore defined, and R' represents a straight- or branched-chain alkyl radical containing 1 to 4 carbon atoms, or a benzyl or carboxymethyl radical.

In general, the reaction is carried out with an excess of methylamine, without a solvent or in an organic solvent such as an aromatic hydrocarbon, an ether or an alcohol of low molecular weight, or a mixture of these solvents, at a temperature from 20° to 130°C, optionally under pressure.

It is particularly advantageous for the thiol formed during the reaction to be fixed in the form of a heavy metal salt using a thiol acceptor such as mercuric chloride.

The dithioester of general formula (VI) can be obtained by the following methods:
(1) By reaction of a strong base with a compound of the general formula (III) (wherein Het, X' and Y as hereinbefore defined), followed by reacting the resulting product with carbon disulphide and then with a compound of the general formula:

   R'-Z (VII)

   wherein R' is as hereinbefore defined, and Z represents a halogen atom, preferably a chlorine, bromine or iodine atom, or a reactive ester radical, preferably a mesyloxy or tosyloxy radical.

The reaction is generally carried out in an ether such as tetrahydrofuran, to which hexamethylphosphoramide has generally been added, at a temperature between -20° and +50°C.

It is particularly advantageous to employ potassium tert.-butoxide as the strong base. Alternatively the organo-lithium derivatives described above may be employed.

It will be understood that it may be desirable to change one or more of the substituents at an appropriate stage during the synthesis of the compounds of the invention, for example, the compounds of general formula (I) wherein R¹ contains a phenyl group substituted by a alkoxycarbonyl group may be alternatively prepared from the corresponding compounds of general formula (I) wherein R¹ contains a phenyl group substituted by a carboxy group by the application or adaptation of known methods for such conversion.

The thioformamide derivatives of general formula (I) obtained by the aforedescribed processes can be purified by the usual physical methods, in particular crystallisation and chromatography, especially to resolve mixtures of enantiomers using a chiral column.

Compounds of general formula (III) may be prepared by the application or adaptation of known methods for example as hereinafter described in Reference Examples.

Compounds of general formula (III) wherein X represents a group of the formula: 〉C=NOR¹ or 〉C=NN(R¹)₂ as hereinbefore defined may be prepared by the reaction of a compound of general formula (III) wherein X represents the carbonyl group with a compound of the general formula:

NH₂OR¹ (Va)

or

NH₂N(R¹)₂ (Vb)

wherein R¹ is as hereinbefore defined or with an acid addition salt thereof in a similar manner to that hereinbefore described for the preparation of the corresponding compounds of general formula (I).

Compounds of general formula (III) wherein X represents the carbonyl group may be prepared from the corresponding 2-methoxy-1-(Het)-cycloalkanol, by known methods.

Alternatively compounds of general formula (III) wherein X represents the carbonyl group and Y represents an ethylene or methylene radical may be prepared from the corresponding 1-[(Het)bromomethyl] cyclohexanol or 1-[(Het)bromomethyl]-cyclopentanol respectively by known methods.

The compounds of general formula (III) wherein Y, Het and X' are as hereinbefore defined with the proviso that Y is not a direct bond when Het represents optionally substituted pyrazin-2-yl and Y does not represent methylene when Het represents optionally substituted indol-3-yl and their processes of preparation form further features of the present invention.

Compounds of general formula (V) may be prepared by known methods.

By the term "pharmaceutically acceptable salts" as used in this specification is meant salts the anions or cations of which are relatively innocuous to the animal organism when used in therapeutic doses so that the beneficial pharmaceutical properties of the parent compounds of general formula (I) capable of forming salts are not vitiated by side-effects ascribable to those anions or cations.

As well as being useful in themselves as active compounds, acid addition salts of the compounds of general formula (I) capable of forming such salts are useful for the purposes of purification of the parent compounds of general formula (I), for example by exploitation of the solubility differences between the salts and the parent compounds, by techniques well known to those skilled in art. The parent compounds of general formula (I) can be regenerated from their acid addition salts by known methods, for example by treatment with an alkali, e.g. aqueous sodium bicarbonate solution or aqueous ammonia solution.

Suitable acid addition salts for use in pharmaceuticals may be selected from salts derived from inorganic acids, for example hydrochlorides, hydrobromides, phosphates, sulphates and nitrates, and organic acids, for example oxalates, lactates, tartrates, acetates, salicylates, citrates, propionates, succinates, fumarates, maleates, methylene-bis-β-hydroxynaphthoates, gentisates and di-p-toluoyltartrates.

As well as being useful in themselves as active compounds, salts of the compounds of general formula (I) capable of forming salts with bases are useful for the purposes of purification of the parent compounds of general formula (I), for example by exploitation of the solubility differences between the salts and the parent compounds, by techniques well known to those skilled in the art.

Suitable salts with bases include alkali metal (e.g. sodium and potassium), alkaline earth metal (e.g. calcium and magnesium), ammonium and amine (e.g. diethanolamine, triethanolamine, octylamine, morpholine and dioctylmethylamine) salts.

The following Examples and Reference Examples illustrate the preparation of compounds according to the present invention.

Unless stated otherwise, all the spectra were recorded at 200 MHz in deuterochloroform; the chemical shifts are expressed in ppm relative to the tetramethylsilane signal. The abbreviations used in the following text are as follows:
- s: = singlet
- d: = doublet
- t: = triplet
- m: = multiplet
- c: = unresolved bands
- dd: = doublet of doublets
- dt: = doublet of triplets
- ddd: = doublet of doublets of doublets
- dddd: = doublet of doublets of doublets of doublets
- qq: = quartet of quartets

### EXAMPLE 1

### Compound A

A vigorously stirred solution of (±)-2-(3-pyridyl)-cyclohexanone (5.3 g, 30 mmol) in anhydrous tetrahydrofuran (50 ml) under argon at -15°C was treated with potassium t.-butoxide (3.36g, 30 mmol).

After 60 minutes at 0°C, a solution of methyl isothiocyanate (2.4 g, 33 mmol) in anhydrous tetrahydrofuran (10 ml) was added during 5 minutes. After 2.5 hours at 0°C the solution was warmed to 20°C and then poured into a saturated aqueous brine solution (250 ml). The mixture was extracted with ethyl acetate (50 ml) and then with chloroform (3 x 50 ml). The combined organic extracts were dried over sodium sulphate and then concentrated in vacuo (30°C; 1.9 kPa (14 mmHg)).

The crude product (7.9 g) was recrystallised from methanol to give (±)-N-methyl-2-oxo-1-(3-pyridyl)cyclohexanecarbothioamide (4.8 g, 19 mmol), m.p. 188-190°C. N.M.R. (CDCl₃) 1.62-2.06 (m, 4H); 2.42-2.60 (m, 2H); 2.60-2.82 (m, 1H); 2.84-3.06 (m 1H), 3.16-3.2 (d, 3H); 7.24-7.34 (ddd, 1H); 7.6-7.68 (ddd, 1H); 8.43-8.47 (d, 1H); 8.48-8.54 (dd, 1H); 8.90-9.2 (broad singlet, 1H). Calculated for C₁₃H₁₆N₂OS; C, 62.9%; H, 6.5%; N, 11.3%; S, 12.9%. Found C, 62.9%; H, 6.6%; N, 11.3%; S, 13.1%.

### REFERENCE EXAMPLE 1

A solution of (±)-trans-1-[(3-pyridyl)bromomethyl] cyclopentanol (10.24 g, 40 mmol) in anhydrous tetrahydrofuran (500 ml) at 0°C was treated, dropwise during 30 minutes, with a solution of silver perchlorate (9.9 g, 48 mmol) in anhydrous tetrahydrofuran (50 ml). After 60 minutes at 0°C the mixture was poured into a mixture of saturated aqueous brine solution (500 ml) and 10% w/v aqueous sodium bicarbonate solution (500 ml). The resulting mixture was filtered and then extracted with ethyl acetate (2 x 500 ml). The combined organic extracts were washed with brine and then dried over sodium sulphate. Concentration in vacuo (30°C; 1.9 kPa (14 mmHg)) afforded a crude oil which was recrystallised from cyclohexane (120 ml) to give (±)-2-(3-pyridyl)cyclohexanone (6.7 g, 38 mmol), m.p. 78-80°C. N.M.R. (CDCl₃) 1.72-2.12 (m, 4H); 2.12-2.40 (m, 2H); 2.40-2.64 (m, 2H); 3.56-3.72 (dd, 1H); 7.22-7.32 (m, 1H); 7.44-7.54 (ddd, 1H); 8.34-8.42 (dd, 1H); 8.46-8.54 (dd, 1H).

### REFERENCE EXAMPLE 2

A solution of 3-cyclopentylidenemethylpyridine (62.2 g, 0.39 mol) in acetone (600 ml) and water (100 ml) was treated with a solution of concentrated sulphuric acid (18.9 g, 0.19 mol) in water (100 ml) at 5°C. The ice-cold solution was treated with 1,3-dibromo-5,5-dimethylhydantoin (55 g, 0.19 mol) during 20 minutes. After 3.5 hours at 0°C the mixture was treated with sodium bicarbonate (33.6 g, 0.4 mol) followed by water (2 l) and then extracted with ethyl acetate (2 x 500 ml). The organic phase was removed and washed with 10% w/v aqueous sodium bicarbonate solution (500 ml) followed by water (200 ml) and brine (200 ml).

The crude extract was dried over sodium sulphate and then filtered through a column of flash silica gel (10 cm x 2.4 cm diameter). After concentration in vacuo (20°C, 1.9 kPa (14 mmHg)) the dark oil crystallised on standing to give (±)-trans-1-[(3-pyridyl)bromomethyl]cyclopentanol (56 g, 0.22 mol) melting point 92-94°C.

Calculated for C₁₁H₁₄BrNO, C, 51.6; H, 5.5; Br, 31.2; N, 5.5%. Found C, 51.9; H, 5.6; Br, 30.6; N, 5.5%.

N.M.R. (CDCl₃) ; 1.36-2.06 (c, 8H); 2.32-2.46 (broad singlet, 1 H); 5.02 (s, 1H); 7.24-7.34 (ddd, 1H); 8.0-8.1 (ddd, 1H); 8.52-8.56 (dd, 1H); 8.62-8.66 (d, 1H).

### REFERENCE EXAMPLE 3

A suspension of cyclopentyltriphenylphosphonium bromide (226 g, 0.55 mol) in anhydrous tetrahydrofuran (1000 ml) at 2°C was treated with vigorous stirring under an atmosphere of argon, with potassium t-butoxide (61.7 g, 0.55 mol). The dark red mixture was stirred at 5°C for 80 minutes and then treated with pyridine-3-carbaldehyde (58.9 g, 0.55 mol) during a period of 20 minutes. The reaction mixture was stirred at 0°C for 2 hours and then at 20°C for 18 hours. The tetrahydrofuran was removed in vacuo (30°C; 1.9 kPa (14 mmHg)) and the residue extracted with pentane (2 x 500 ml). After treatment with decolourising charcoal (5 g) the mixture was filtered through a plug of flash silica gel (Merck 70-230 mesh (13 cm x 2 cm diameter). The filtrate was concentrated in vacuo (30°C, 1.9 kPa (14 mmHg), then 20°C; 1.3 Pa (0.01 mmHg)) to afford 3-cyclopentylidenemethylpyridine (54 g, 0.34 mol,) as an oil which was used without further purification. N.M.R. (CDCl₃) 1.6-1.95 (m, 4H); 2.4-2.65 (m, 4H); 6.26-6.34 (m, 1H); 7.16-7.25 (ddd, 1H); 7.56-7.65 (ddd, 1H); 8.52-8.52 (d, 1H).

### REFERENCE EXAMPLE 4

A 4:1 mixture of (±)-cis and trans-2-methoxy-1-(3-pyridyl)cyclohexanol (2g, 10 mmol), toluene and phosphorus pentoxide (3.4g, 24 mmol) was heated at reflux for 5 hours. The mixture was then filtered and the precipitate was portioned between 2M sodium hydroxide solution (80 ml) and diethyl ether (25ml). The aqueous layer was extracted with ether (3x25 ml) and the combined organic extracts were dried over sodium sulphate. Concentration in vacuo afforded a crude oil which was purified by flash chromatography to give 2-(3-pyridyl)cyclohexanone (0.7g, 4mmol).

### REFERENCE EXAMPLE 5

To a solution of 2.5M n-butyl lithium in hexane (13.2ml, 33 mmol) at -78°C (was added diethyl ether (15 ml) followed by a solution of 3-bromopyridine (4.7g, 30 mmol) in ether (90 ml) over a period of 10 minutes. After 1 hour at -78°C a solution of (±)-2-methoxycyclohexanone (3.84g, 30 mmol) in ether (20 ml) was added dropwise during 10 minutes. After 2 hours at -78°C and 30 minutes at 0°C the reaction mixture was warmed to 20°C and then poured onto ice (150g). The mixture was extracted with ether (2 x 50 ml) and then the combined organic extracts were extracted with 1N hydrochloric acid (50 ml). This aqueous extract was washed with ether (20 ml) and then treated with 2M sodium hydroxide solution (25 ml) and extracted with ether (3 x 100 ml). The organic extracts were combined, washed with brine then dried over anhydrous sodium sulphate. Concentration in vacuo afforded (±)-2-methoxy-1-(3-pyridyl)- cyclohexanol (5.0g, 24 mmol) as a 4:1 mixture of cis and trans isomers; N.M.R. (CDCl₃), 1.2 - 2.14 (c), 2.24 - 2.44 (m), 2.90 - 3.28 (c); 3.48 - 3.60 (m), 7.18 - 7.30 (m); 7.78 - 7.96 (m); 8.40 - 8.48 (m); 8.62 - 8.72 (m); 8.78 - 8.82 (m).

### EXAMPLE 2

### Compound B

A solution of (±)-2-(3-quinolinyl)cyclohexanone (0.78g, 3.5 mmol) in tetrahydrofuran (10ml) at -5°C was treated with potassium t.-butoxide (0.43g, 3.9mmol) in one portion. After 25 minutes at -5°C the deep red mixture was treated dropwise during 1 minute with a solution of methyl isothiocyanate (0.28g, 3.9 mmol) in tetrahydrofuran (2 ml). After 4 hours at 0°C the reaction mixture was partitioned between saturated aqueous ammonium chloride solution (50 ml) and chloroform (50 ml). The aqueous layer was extracted again with chloroform (50 ml). The combined organic extracts were then dried over sodium sulphate and concentrated in vacuo (20°C; 1.9 kPa (14mmHg)) to give a crude oil which was purified by flash chromatography over silica gel eluting with ethyl acetate to give (±)-N-methyl-2-oxo-1-(3-quinolinyl)cyclohexanecarbothioamide (0.1g, 0.33 mmol), m.p. 235-236°C N.M.R. (CDCl₃) 1.7-2.18 (c, 4H), 2.46-2.64 (m, 2H) 2.72-2.90 (m, 1H), 2.96-3.16 (m, 1H), 3.16-3.22 (d, 3H), 7.50-7.62 (ddd, 1H), 7.66-7.76 (ddd, 1H), 7.76-8.02 (dd, 1M), 8.0 (d, 1M), 8.04-8.12 (dd, 1H), 8.78-8.80 (d, 1H), 8.92-9.18 (broad singlet, 1H). Calculated for C₁₇H₁₈N₂OS: C,68.4; H, 6.08; N, 9.4; S, 10.7%: Found C, 68.0; H, 5.8; N, 9.0; S, 10.4%.

### REFERENCE EXAMPLE 6

A solution of (±)-trans-1-[3-quinolinyl)bromomethyl] cyclopentanol (1.1g, 3.6 mmol) in tetrahydrofuran (20 ml) at 0°C was treated, dropwise during 2 minutes, with a solution of silver perchlorate (893 mg, 4.3 mmol) in tetrahydrofuran (5 ml). After 1 hour at 0°C a mixture of saturated brine solution (20 ml) and saturated aqueous sodium bicarbonate solution (20 ml) was added to the reaction mixture. Ethyl acetate (40 ml) was then added and the resulting mixture was filtered through diatomaceous earth. The aqueous layer was removed and extracted with ethyl acetate (50ml). The combined organic extracts were washed with brine (30 ml), dried over sodium sulphate then concentrated in vacuo to afford (±)-2-(3-quinolinyl)cyclohexanone (0.8g, 3.5 mmol) as an oil which solidified on standing, melting point 114-115°C. N.M.R. (CDCl₃) 1.72 - 2.68 (c, 8H) 3.74 - 3.88 (dd, 1H), 7.46 - 7.56 (ddd, 1H), 7.62 - 7.70 (ddd, 1H), 7.72 - 7.82 (dd, 1H), 7.88 - 7.96 (d, 1H), 8.04 - 8.12 (d, 1H), 8.68 (d, 1H).

### REFERENCE EXAMPLE 7

A mixture of 3-cyclopentylidenemethylquinoline (3g, 14 mmol), water (100 ml), dimethyl sulphoxide (50 ml), acetone (100 ml) and concentrated sulphuric acid (3.4 ml, 35 mmol) at 5°C was treated with 1,3-dibromo-5,5-dimethylhydantoin (4.0g, 14 mmol). After 5 minutes at 5°C the mixture was stirred for 2 hours at 20°C. The mixture was filtered and washed with ethyl acetate (2 x 50 ml). The aqueous phase was then treated with saturated aqueous sodium bicarbonate solution (50 ml) and extracted with ethyl acetate (100 ml). The organic phase was then washed with water (50 ml) and brine (50 ml) then dried over sodium sulphate. Concentration in vacuo afforded a crude oil which was purified by flash chromatography eluting with a mixture of diethyl ether and hexane (50:50) and then ether. The product so obtained was then recrystallised from cyclohexane to give (±)-trans-1-[(3-quinolinyl)bromomethyl]cyclopentanol (1.1g, 3.6 mmol). N.M.R. 1.44-1.85 (c, 4H), 1.8-2.06 (c, 4H) 5.06 (s, 1H), 7.44-7.56 (ddd, 1H), 7.60-7.72 (ddd, 1H), 7.74-8.02 (dd, 1H) 8.04-8.12 (dd, 1H), 8.38-8.40 (d, 1H), 9.0 (d, 1H).

### REFERENCE EXAMPLE 8

A solution of 2-chloro-3-cyclopentylidenemethylquinoline (7.6g, 31.3 mmol) in glacial acetic acid (80 ml) at 60°C was treated with zinc powder (4.0g, 62.6 mmol). After stirring at 60°C for 3 hours, the reaction mixture was cooled and then treated dropwise with 2M aqueous sodium hydroxide solution (330 ml); the temperature being kept below 20°C throughout. The resulting mixture was then extracted with ethyl acetate (2 x 250 ml). The combined organic extracts were dried over sodium sulphate then concentrated in vacuo (30°C, 1.9 kPa (14mm Hg)) to give a crude red oil (8g) which was extracted with hot pentane (2 x 200 ml). Concentration of the combined extracts in vacuo (20°C, 1.9 kPa (14 mm Hg)) afforded 3-cyclopentylidenemethylquinoline (4g, 31 mmol) which was used without further purification. N.M.R. (CDCl₃) 1.6-1.96 (m, 4H); 2.5-2.72 (m, 4H); 6.50 (m, 1H), 7.46-7.58 (ddd, 1H) 7.60-7.68 (ddd, 1H), 7.76-7.80 (dd, 1H); 8.0-8.08 (c, 2H).

### REFERENCE EXAMPLE 9

A suspension of cyclopentyltriphenylphosphonium bromide (4.1g, 10 mmol) in tetrahydrofuran (50 ml) at 0°C was treated with potassium t.-butoxide (1.1g, 10 mmol) portionwise during 5 minutes. After 1 hour at 0°C the deep red mixture was treated with 2-chloroquinoline-3- carbaldehyde (1.9g, 10 mmol).

After 4 hours at 0°C hexane (250 ml) followed by brine (50 ml) was added to the reaction mixture. The organic layer was removed and dried over sodium sulphate. After concentration in vacuo (30°C, 1.9 kPa (14mm Hg)) the crude oil was recrystallised from hexane to give 2-chloro-3-cyclopentylidenemethylquinoline (1.7g, 7 mmol) m.p. 84-86°C; N.M.R. (CDCl₃); 1.64-1.90 (m, 4H); 2.44-2.66 (m, 4H); 6.52 (m, 1H); 7.44-7.56 (ddd, 1H); 7.58-7.68 (ddd, 1H); 7.70-7.78 (dd, 1H); 7.92-8.00 (dd, 1H); 8.04 (s, 1H). Calculated for C₁₅H₁₄ClN, C, 73.9; 14.11; Cl 14.5%; Found C, 74.3; H 5.8; Cl, 14.6, N, 5.7%.

### REFERENCE EXAMPLE 10

A mixture of 3:1-cis : trans- (±)-2-methoxy-1-(3-quinolinyl)cyclohexan-1-ol (1.35 g, 5.3 mmol) and 40% w/v sulphuric acid (25 ml) was refluxed for 1 hour. The cooled mixture was basified with 1.OM sodium carbonate solution (200 ml) and the mixture extracted with ethyl acetate (3 x 125 ml). The combined organic extracts were washed with brine (30 ml) then dried over sodium sulphate. Concentration in vacuo afforded a crude oil (1.4 g) which was recrystallised from a 4:1 mixture of hexane and ethyl acetate (20 ml) to give (±)-2-(3-quinolinyl)cyclohexanone (0.53 g, 2.3 mmol), melting point 114-115°C.

### REFERENCE EXAMPLE 11

A solution 2.5 M n-butyllithium in hexane (18 ml) in ether (30 ml) at -78°C was treated dropwise with a solution of 3-bromoquinoline (4.7 g, 22.5 mmol) in ether (30 ml). After 1 hour at -78°C a solution of (±)-2-methoxycyclohexanone (5.8 g, 45 mmol) in ether (30 ml) was added dropwise during 35 minutes to the reaction mixture, which was maintained at -78°C for 2 hours then at 0°C for 1 hour and then warmed to 20°C during 1 hour. The reaction mixture was poured onto ice (50 g) and water (50 ml) and the resulting aqueous layer extracted with ether (3 x 50 ml). The combined organic extracts were treated with 2N HCl (75 ml) and the organic phase discarded. The aqueous layer was washed with ether (2 x 30 ml) then basified with 2N sodium hydroxide (75 ml). The aqueous layer was then extracted with ether (4 x 50 ml). The combined organic extracts were washed with brine (30 ml), dried over sodium sulphate and then concentrated in vacuo to afford a crude oil which was recrystallised from a 4:1 mixture of hexane and ethyl acetate (60 ml) to give a 3:1 mixture of (±)-cis and trans 2-methoxy-1-(3-quinolinylcyclohexan-1-ol (3.2 g, 12 mmol), melting point 114-115°C. H n.m.r. (CDCl₃) essential features 3.06 (singlet, trans- OMe), 3.12 (singlet, cis- OMe).

### EXAMPLE 3

### Compound C

A stirred solution of (±)-2-(3-pyridyl)cycloheptanone (1.0 g, 5.3 mmol) in anhydrous tetrahydrofuran (10 ml) under argon at -5°C was treated with potassium t-butoxide (0.65 g, 5.8 mmol).

After 25 minutes at -5°C a solution of methyl isothiocyanate (0.42 g, 5.8 mmol) in tetrahydrofuran (1 ml) was added during 2 minutes.

After 3 hours at -5°C the solution was warmed to 20°C during 3 hours and then poured into a saturated aqueous ammonium chloride solution (50 ml). The mixture was extracted with chloroform (2 x 50 ml). The combined extracts were dried over sodium sulphate then concentrated in vacuo to afford a crude oil (1.7 g) which was purified by flash chromatography, eluting with a mixture of ethyl acetate and hexane (9:1) to afford (±)-N-methyl-2-oxo-1-(3-pyridyl)cycloheptanecarbothioamide (530 mg, 2.0 mmol), melting point 144-146°C; N.M.R. (CDCl₃); 1.25-2.05 (C, 6H); 2.24-2.42 (m, 1H); 2.58-2.74 (m, 1H); 2.76-2.94 (m, 1H); 3.14 (d, 3H); 3.36-3.54 (m, 1H), 7.22-7.32 (m, 1H); 7.58-7.64 (m, 1H), 8.42-8.54 (m, 2H), 8.54-8.62 (broad singlet, 1H). Found C, 63.9; H, 6.9; N, 10.6; s, 12.4%; C₁₄H₁₈N₂OS requires C, 64.1; H, 6.9; N, 10.7; S, 12.2%.

### REFERENCE EXAMPLE 12

A stirred solution of (±)-trans-1-[(3-pyridyl)bromomethyl]cyclohexanol (4.2g, 15.5 mmol) in tetrahydrofuran (50 ml) at 0°C was treated dropwise with a solution of silver perchlorate (3.9g, 18.8 mmol) in tetrahydrofuran (20 ml) during 20 minutes. After 3 hours at 0°C the mixture was treated with saturated aqueous brine solution (50 ml) followed by saturated aqueous sodium bicarbonate solution (50 ml). Ethyl acetate (50 ml) was then added and the mixture was filtered through diatomaceous earth. The organic phase was removed and the aqueous phase was extracted with ethyl acetate (50 ml). The combined organic extracts were washed with brine (20 ml) then dried over sodium sulphate. Concentration in vacuo followed by flash chromatography over silica gel eluting with ethyl acetate afforded (±)-2-(3-pyridyl)cycloheptanone (0.4g, 2.1 mmol) as an oil. N.M.R. (CDCl₃); 1.32-2.20 (m, 8H); 2.56-2.70 (m, 2H), 3.34-3.86 (dd, 1H), 7.22-7.32 (m, 1H), 7.54-7.62 (m, 1H), 8.4-8.54 (m, 2H). Found C, 76.4; H, 8.3; N, 7.3%: C₁₂H₁₅NO requires C, 76.2; H, 8.0; N, 7.4%.

### REFERENCE EXAMPLE 13

A mixture of 3-cyclohexylidenemethylpyridine (1.6g, 9.3 mmol) and 1M sulphuric acid (25 ml) at -10°C was added 1,3-dibromo-5,5-dimethylhydantoin (3.2g, 11 mmol) during 15 minutes. After stirring at 0°C for 1 hour the reaction mixture was filtered then washed with ethyl acetate (20 ml). The aqueous phase was treated with sodium bicarbonate (5g) and then extracted with a 2:1 mixture of hexane and ethyl acetate (20 ml), and then ethyl acetate (20 ml). The combined organic extracts were washed with water (3 x 5 ml) then with brine (5 ml) and dried over sodium sulphate. Concentration in vacuo (30°C; 1.9 kPa (14mm Hg)) afforded a crude oil (1.5g) which was purified by flash chromatography over silica gel eluting with ethyl acetate to give (±)-trans-1-[(3-pyridyl)bromomethyl]cyclohexanol (0.5g, 1.8 mmol) m.p. 120-122°C. N.M.R. (CDCl₃) 1.02-2.12 (c, 11H), 4.96 (s, 1H), 7.22-7.34, (m, 1H), 7.90-8.02 (m, 1H), 8.46-8.56 (dd, 1H), 8.56-8.62 (d, 1H) Found C, 53.4; H, 6.0; Br, 29.5; N, 5.1: C₁₂H₁₅BrN0 requires C, 53.4; H, 6.0; Br, 29.6; N, 5.2.

### EXAMPLE 4

### Compounds D and E

A stirred solution of (±)-N-methyl-2-(3-pyridyl)- cyclohexanecarbothioamide (1.7g, 6.9 mmol) in methanol (4 ml) at 0°C was treated with sodium borohydride (262mg, 6.9 mmol). After 10 minutes at 0°C the mixture was warmed to 20°C and a solution formed. It was then cooled to 0°C and stirred for a further 40 minutes. The reaction mixture was then treated with brine (10 ml) and water (30 ml) and extracted with ethyl acetate (2 x 50 ml). The combined organic extracts were washed with brine (10ml) then dried over anhydrous sodium sulphate. After concentration in vacuo (30°C; 1.9 kPa (14 mmHg)) the product was fractionally recrystallised from cyclohexane: ethyl acetate (6:1) (10 ml) to afford (±)-trans-N-methyl-2-hydroxy-1-(3-pyridyl)cyclohexanecarbothioamide (1.4g, 5.6 mmol) [m.p. 171-172°c. N.M.R. (CDCl₃) 1.24-2.02 (m, 6H); 2.08-2.28 (dt, 1H) 2.46-2.60 (m, 1H); 3.06-3.12 (d, 3H); 3.54-3.78 (br.s, 1H); 4.70-4.86 (m, 1H); 7.26-7.28 (m, 1H); 7.44-7.70 (br.s, 1H); 8.20-8.28 (dt,1H); 8.48-8.58 (dd, 1H); 8.92-8.96 (d, 1H). Calculated for C₁₃H₁₈N₂OS; C, 62.4; H, 7.3; N, 11.2; S, 12.8 Found; C, 62.3; H, 7.3; N, 11.4; 12.7.] and (±)-cis-N-methyl-2-hydroxy-1-(3-pyridyl)cyclohexanecarbothioamide (0.2g, 0.8 mmol). [m.p. 169-172°C. NMR (CDCl₃) 1.32-2.06 (complex, 7H); 2.88-3.04 (m, 1H); 3.22-3.30 (d, 3H); 4.36-4.48 (dd, 1H); 5.46-6.06 (broad singlet, 14); 7.14-7.26 (m, 1H); 7.81-7.92 (m, 1H); 8.16-8.24 (dd, 1H); 8.52-8.60 (m, 1H). Calculated for C₁₃H₁₈N₂OS; C, 62.4; H, 7.3; N, 11.2; S, 12.8. Found C, 62.0; H, 7.3; N, 11.1; S, 12.7.

### EXAMPLE 5

### Compound F

A suspension of (±)-N-methyl-2-oxo-1-(3-pyridyl) cyclohexanecarbothioamide (370 mg, 1.5 mmol) in pyridine (3 ml) at 20°C was treated with hydroxylamine hydrochloride (210 mg, 3.0 mmol). After 4 days at 20°C the mixture was poured into water (50 ml) which was then extracted with ethyl acetate (2 x 20 ml). The combined organic extracts were washed successively with water (10 ml), and then brine (10 ml) then dried over anhydrous sodium sulphate. After concentration in vacuo (30°C; 1.9 kPa (14 mmHg)) the crude product was recrystallised from propan-2-ol to give (±)-anti-N-methyl-2-hydroxyimino-1-(3-pyridyl)cyclohexanecarbothioamide (220 mg, 0.84 mmol), m.p. 181-183°C, N.M.R. (D6-dimethylsulphoxide) 1.26-1.82 (m, 4H); 2.08-2.38 (m, 2H); 2.86-3.20 (m, 5H); 7.24-7.34 (m, 1H); 7.58-7.68 (m, 1H); 8.32-8.40 (m, 1H); 8.42-8.50 (m, 1H); 9.44-9.60 (broad multiplet, 1H); 10.90 (singlet, 1H)s. Calculated for C₁₃H₁₇N₃OS, C, 59.3; H, 6.5; N, 15.96%; S, 12.17; Found: C, 59.7; H, 6.63; N, 16.0; S, 12.3%.

### EXAMPLE 6

### Compound G

A suspension of (±)-N-methyl-2-oxo-1-(3-pyridyl)cyclohexanecarbothioamide (0.5 g, 2 mmol) in pyridine (5 ml) at 20°C was treated with O-methylhydroxylamine hydrochloride (0.34 g, 4 mmol). After stirring for 48 hours at 20°C the mixture was treated with another portion of O-methylhydroxylamine hydrochloride (0.68 g, 8 mmol) and then warmed to 40°C. After 2 hours the solution was poured into water (50 ml) and the resulting mixture was extracted with ethyl acetate (3 x 50 ml). The combined organic extracts were washed with water (20 ml) then dried over sodium sulphate. Concentration in vacuo afforded a crude oil (0.6 g) which was recrystallised from isopropanol to give (±)-anti-N-methyl-2-methoxyimino-1-(3-pyridyl)cyclohexanecarbothioamide (0.3 g, 1.1 mmol), melting point 150-151°C. H n.m.r. (CDCl₃) 1.50-2.06 (C, 4H), 2.14-2.26 (C, 2H), 2.74-3.02 (C, 2H), 3.18-3.24 (d, 3H), 3.84 (s, 3H), 7.16-7.28 (m, 1H), 7.56-7.64 (m, 1H), 8.42-8.48 (m, 1H), 8.48-8.70 (C, 2H); found : C, 60.1; H, 6.8; N, 14.9; s, 11.6% C₁₄H₁₉N₃OS; requires : C, 60.6; H, 6.9; N, 15.2; S, 11.6%.

### EXAMPLE 7

### Compound H

A solution of (±)-N-methyl-2-oxo-(3-pyridyl)cyclohexanecarbothioamide (0.5 g, 2 mmol) in pyridine (5 ml) at 60°C was treated with O-benzylhydroxylamine hydrochloride (0.65 g, 4 mmol) and the resulting mixture was then stirred for 24 hours at 60°C. The reaction mixture was partitioned between water (50 ml) and ethyl acetate (25 ml) and the aqueous layer was then extracted with ethyl acetate (2 x 25 ml). The combined organic extracts were washed with brine (50 ml) then dried over magnesium sulphate. Concentration in vacuo afforded a crude oil which was recrystallised from ethyl acetate to give (±)-anti-N-methyl-2-benzyloxyimino-1-(3-pyridyl)cyclohexanone (0.57 g, 1.6 mmol), melting point 128-130°C. H n.m.r. (CDCl₃) 1.48-2.15 (c, 4H), 2.16-2.40 (m, 2H), 2.8-2.96 (m, 2H), 2.96-3.04 (d, 3H), 5.02-5.08 (s, 2H), 7.12-7.22 (c, 3H), 7.32-7.44 (c, 3H), 7.44-7.56 (m, 2H), 8.16-8.38 (broad singlet, 1H), 8.38-8.52 (c, 2H). Found : C, 67.7; H, 6.5; N, 11.9; S, 9.0% C₂₀H₂₃N₃OS requires C, 68.0; H, 6.6; N, 11.9; S, 9.1%.

### EXAMPLE 8

### Compound I

A solution of (±)-N-methyl-2-oxo-1-(3-quinolinyl)cyclohexanecarbothioamide (0.4 g, 1.3 mmol) in pyridine (5 ml) at 20°C was treated with hydroxylamine hydrochloride (0.19 g, 2.3 mmol). After 24 hours at 20°C the solution was partitioned between ethyl acetate (75 ml) and water (75 ml). The aqueous layer was extracted with ethyl acetate (3 x 75 ml) and the combined organic extracts were washed with water (15 ml) then dried over sodium sulphate. Concentration in vacuo afforded a crude oil (0.55 g) which was recyrstallised from isopropanol to give (±)-anti-N-methyl-2-hydroxyimino-(3-quinolinyl)cyclohexanecarbothioamide (0.25 g, 0.8 mmol), melting point 185-186°C. H n.m.r. (CDCl₃) 1.48-2.46 (c, 6H), 2.96-3.08 (m, 1H), 3.08-3.18 (d, 3H), 3.18-3.38 (m, 1H), 7.44-7.56 (dt, 1H), 7.60-7.70 (dt, 1H), 7.70-7.80 (dd, 1H), 7.96-8.06 (m, 2H), 8.52-8.68 (broad multiplet) 10.90 (s, 1H). Found : C, 64.8; H, 6.1; N, 13.3; S, 9.9%; C₁₇H₁₉N₃OS requires C, 65.2; H, 6.1; N, 13.4; S, 10.2%.

### EXAMPLE 9

A solution of (2S,2'S)-anti-2-(methoxymethyl)-1-[2'-(3-quinolyl)-2'-methylthiocarbamoylcyclohexylideneamino]pyrrolidine (117 mg, 0.28 mmol; prepared in Example 10) in acetone (1 ml), water (0.8 ml) and glacial acetic acid (0.5 ml) was heated at 60 °C for 13 hours. The reaction mixture was cooled to 20 °C and the precipitated solid was filtered off and washed with acetone (2 x 1 ml). Recrystallisation from methanol afforded the (S)-isomer of N-methyl-2-oxo-1-(3-quinolinyl)cyclohexanecarbothioamide (41 mg, 0.14 mmol), m.p. 256-257°C. H n.m.r. (CDCl₃) 1.71-2.08 (c, 4H); 2.57 (m, 2H); 2.8 (m, 1H); 3.08 (m, 1H); 3.17 (d, 3H); 7.55 (dt, 1H); 7.66-7.82 (c, 2H); 7.99 (d, 1H); 8.08 (d, 1H); 8.79 (d, 1H); 8.94-9.14 (broad singlet, 1H). Found C, 68.9; H, 6.3; N, 9.3% C₁₇H₁₈N₂OS requires C, 68.4; H, 6.1; N, 9.4%).

### EXAMPLE 10

### Compound J

A vigorously stirred solution of a 50:50 mixture of 2S-(+)-2-(methoxymethyl)-1-[2-(3-quinolinyl)-cyclohexylideneamino]pyrrolidine and 2S-(-)-2-(methoxymethyl)-1-[2-(3-quinolinyl)-cyclohexylideneamino]pyrrolidine (2.6 g, 7.8 mmol) in tetrahydrofuran (30 ml) at -78°C was treated with a 1.6 M solution of n-butyllithium in hexane (5.9 ml, 9.4 mmol). After 30 minutes at -78°C the reaction mixture was warmed to 0°C and diethylether (30 ml) was added. After 15 minutes at 0°C the reaction mixture was cooled to -78°C then treated with methyl isothiocyanate (730 mg, 10 mmol). The reaction mixture was stirred at -78°C for 1 hour then warmed slowly to 20°C over 3 hours. Saturated aqueous ammonium chloride solution (20 ml) was added to the solution, followed by the addition of ethyl acetate (50 ml). The aqueous layer was extracted with ethyl acetate (2 x 25 ml) and the combined organic extracts were dried over sodium sulphate. Concentration in vacuo afforded a crude oil which when treated with cyclohexane (40 ml) precipitated a crude solid. Recrystallisation from a 5:1 mixture of cyclohexane and ethyl acetate gave (2S,2'S)-anti-2-(methoxymethyl)-1-[2'-(3-quinolinyl)-2'-methylthiocarbamoylcyclohexylideneamino]pyrrolidine (1.1 g, 2.7 mmol), melting point 166-168°C. H n.m.r. (CDCl₃) 1.48-2.10 (c, 8H); 2.16-2.76 (c, 4H); 3.14 (d, 3H), 3.17-3.8 (c, 3H); 3.41 (s, 3H); 3.49 (d, 2H); 7.50 (dt, 1H); 7.66 (dt, 1H); 7.78 (dd, 1H); 8.00-8.10 (c, 2H); 8.86 (d, 1H); 10.7 (broad singlet, 1H). Found C, 67.6%; H, 7.5%; N, 13.6%; S, 7.8% C₂₃H₃₀N₄OS requires C, 67.3; H, 7.4; N, 13.7; S, 7.8%.

The absolute configuration was confirmed by X-ray crystallography.

### REFERENCE EXAMPLE 14

A mixture of (±)-2-(3-quinolinyl)cyclohexanone (0.5 g, 2.2 mmol), (S)-(-)-1-amino-2-(methoxymethyl)pyrrolidine ('SAMP')(0.28 g, 2.2 mmol) and p-toluenesulphonic acid (10 mg) was refluxed in toluene (15 ml) for 2.5 hours. The toluene was removed in vacuo (40°C/1.9 kPa (14 mmHg),) to give a crude oil which was partitioned between water (20 ml) and ethyl acetate (25 ml). The aqueous layer was extracted with ethyl acetate (2 x 25 ml) and the combined organic extracts were dried over sodium sulphate then concentrated in vacuo to give (2S)-(+)-2-(methoxymethyl)-1-[2-(3-quinolinyl)cyclohexylideneamino]pyrrolidine and (2S)-(-)-2-(methoxymethyl)-1-[2-(3-quinolinyl)cyclohexylideneamino]pyrrolidine as a 50:50 mixture of diastereoisomers (0.69 g, 2 mmol).

### EXAMPLE 11

A solution of (2R)-anti-2-(methoxymethyl)-1-[2'-(3-quinolinyl)-2'-methylthiocarbamoylcyclohexylideneamino]pyrrolidine (122 mg, 0.3 mmol; prepared in Example 12) in acetone (1 ml), water (0.8 ml) and glacial acetic acid (0.5 ml) was heated at 60°C for 13 hours. The reaction mixture was cooled to 20°C and the precipitated solid was filtered off and washed with acetone (2 x 1 ml). Recrystallisation from methanol afforded the (R)-isomer, by inference, of N-methyl-2-oxo-1-(3-quinolinyl)cyclohexanecarbothioamide (39 mg, 0.13 mmol), m.p. 255-256°C. H n.m.r. (CDCl₃) 1.71-2.08 (c, 4H); 2.57 (m, 2H); 2.8 (m, 1H); 3.08 (m, 1H); 3.17 (d, 3H); 7.55 (dt, 1H); 7.66-7.82 (c, 2H); 7.99 (d, 1H); 8.08 (d, 1H); 8.79 (d, 1H); 8.94-9.14 (broad singlet, 1H). Found C, 68.1; H, 6.0; N, 9.4%; C₁₇H₁₈N₂OS requires C, 68.4; H, 6.1; N, 9.4%.

### EXAMPLE 12

A solution of a 50:50 mixture of 2(R)-(+)-2-(methoxymethyl)-1-[2-(3-quinolinyl)cyclohexylideneamino]pyrrolidine and 2(R)-(-)-2-(methoxymethyl)-1-[2-(3-quinolinyl)cyclohexylideneamino]pyrrolidine (2.6 g, 7.8 mmol) in tetrahydrofuran (30 ml) was added dropwise to a solution of 1.6 M n-butyllithium in hexane (5.2 ml) containing tetrahydrofuran (20 ml) at -78°C. After 1.5 hours at -78°C the reaction mixture was treated with a solution of methyl isothiocyanate (0.68 g, 9.3 mmol). After 15 minutes at -78°C the solution was warmed slowly to 0°C over 2 hours. After 30 minutes at 0°C aqueous saturated ammonium chloride (30 ml) was added to the reaction mixture which was extracted with chloroform (2 x 50 ml). The combined organic extracts were washed with brine (20 ml) then dried over sodium sulphate. Concentration in vacuo afforded a crude oil (3.2 g) which was recrystallised from a 5:1 mixture cyclohexane and ethyl acetate to give (2R )-anti-2-(methoxymethyl)-1-[2'-(3-quinolinyl)-2'-methylthiocarbamoylcyclohexylideneamino]pyrrolidine (0.67 g, 1.6 mmol) m.p. 163-165°C. H n.m.r. (CDCl₃) 1.48-2.10 (c, 8H); 2.16-2.76 (c, 4H); 3.14 (d, 3H); 3.17-3.8 (c, 3H); 3.41 (s, 3H); 3.49 (d, 2H); 7.50 (dt, 1H); 7.66 (dt, 1H); 7.78 (dd, 1H); 8.00-8.10 (c, 2H); 8.86 (d, 1H); 10.7 (broad singlet, 1H). Found C, 67.4; H, 7.5; N, 13.6; S, 7.7% C₂₃H₃₀N₄OS requires C, 67.3; H, 7.4; N, 13.7; S, 7.8%.

By inference, this was the (2'R)-isomer.

### REFERENCE EXAMPLE 15

A mixture of (±)-2-(3-quinolinyl)cyclohexanone (1.75 g, 7.8 mmol), (R)-(+)-1-amino-2-(methoxymethyl)pyrrolidine ('RAMP')(1.0 g, 7.8 mmol) and p-toluenesulphonic acid (20 mg) was refluxed in toluene (40 ml) for 1.5 hours using a Dean-Stark apparatus. The toluene was removed in vacuo at 40°C/1.9 kPa (14 mmHg) and then at 20°C/0.013 kPa (0.1 mmHg) to give a crude oil (2.6 g) containing (2R)-(+)-2-(methoxymethyl)-1-[2-(3-quinolinyl)cyclohexylideneamino]pyrrolidine and (2R)-(-)-2-(methoxymethyl)-1-[2-(3-quinolinyl)cyclohexylideneamino]pyrrolidine as a 50:50 mixture of diastereoisomers. This mixture was used as such without further purification.

### EXAMPLE 13

### Compound K

A 2.5M solution of n-butyllithium in hexane (47ml) was added dropwise to a stirred solution of (2S)-2-methoxymethyl-1-[2-(3-pyridyl)-cyclohexylideneamino]pyrrolidine (50:50 mixture of diastereoisomers) (30.73g) in dry tetrahydrofuran (410 ml) at -75°C under argon during 15 minutes, to give a dark red solution. After 30 minutes, a solution of methyl isothiocyanate (8.63g) in tetrahydrofuran (65ml) was added during 10 minutes. The solution was allowed to warm to to 0°C during 45 minutes, maintained at this temperature for 1 hour and then at 20°C for 1 hour, giving a yellow solution. This solution was quenched with saturated aqueous ammonium chloride solution (270ml). The organic layer was separated and the aqueous layer extracted with ether(3x50ml). The combined extracts were washed with brine (2x25ml), dried over magnesium sulphate and evaporated (40°C/0.027 kPa (0.2mmHg)) to give crude (2S)-anti-2-(methoxymethyl)-1-[2'-(3-pyridyl)-2'-methylthiocarbamoylcyclohexylideneamino]pyrrolidine, a semi-crystalline oil (39.81g)

By inference, this was the (2'S)-isomer.

### EXAMPLE 14

A solution of (2S)-anti-2-methoxymethyl-1-[2'-(3-pyridyl)-2'-methylthiocarbamoylcyclohexylideneamino]pyrrolidine (prepared in Example 13), in 2M aqueous hydrochloric acid (400ml) was stirred at 60°C for 12 hours. The solution was washed with methylene chloride (3x150ml). The aqueous phase was brought to pH 8 with 2M aqueous sodium hydroxide solution. The precipitate was extracted into methylene chloride (500ml + 3x150ml). The combined extracts were washed with water (3x50ml), dried over magnesium sulphate and evaporated. The residual solid was washed with ethyl acetate (50ml) and recrystallised from methanol to give the (S)-isomer, by inference, of N-methyl-2-oxo-1-(3-pyridyl)cyclohexanecarbothioamide, colourless crystals (12.04g), m.p. 193-194°C,[α]³⁰ -83° (CHCl₃).

### REFERENCE EXAMPLE 16

By proceeding in a similar manner to that hereinbefore described in Reference Example 15, there was prepared (2S)-2-methoxymethyl-1-[2-(3-pyridyl)cyclohexylideneamino]pyrrolidine (50:50 mixture of diastereoisomers), a yellow oil.

### EXAMPLE 15

By proceeding in a similar manner to that hereinbefore described in Example 7, there was prepared the (S)-isomer by inference of anti-N-methyl-2-benzyloxyimino-1-(3-pyridyl)cyclohexanecarbothioamide, colourless crystals, m.p.129-130°C, [α]²⁸-59.4° (CHCl₃), from N-methyl-2-oxo-1-(3-pyridyl)cyclohexanecarbothioamide (prepared in Example 14).

### EXAMPLE 16

### Compound L

A stirred suspension of (±)-N-methyl-2-oxo-1-(3-pyridyl)cyclohexanecarbothioamide (0.5g) and 2-dimethylaminoethoxyamine hydrochloride (0.37g) in ethanol (5ml) and pyridine (1ml) was refluxed for 24 hours The reaction mixture was evaporated and the residual gum dissolved in water (15ml) and washed with chloroform (4x7.5ml). The aqueous phase was brought to pH 14 with 2M aqueous sodium hydroxide solution and then extracted with chloroform (4x10ml). The combined extracts were dried over magnesium sulphate, and evaporated. The residual oil was triturated with water to give (±)-anti-N-methyl-2-(2-dimethylaminoethoxyimino)-1-(3-pyridyl)cyclohexanecarbothioamide, a colourless solid (0.55g), m.p. 93-95°C.

### EXAMPLE 17

### Compound M

A stirred suspension of (±)-N-methyl-2-oxo-1-(3-pyridyl)cyclohexanecarbothioamide (1.24g) and 2-aminoethoxyamine dihydrochloride (0.78g) in ethanol (12.5ml) and pyridine (2.5ml) was refluxed for 11 hours. The reaction mixture was evaporated and the residual gum dissolved in water (15ml) and washed with chloroform (4x10ml). The aqueous phase was brought to pH 14 with 2M aqueous sodium hydroxide solution and then extracted with chloroform (4x10ml). The combined extracts were dried over magnesium sulphate, and evaporated. The residual oil was purified by flash chromatography on silica eluting with chloroform/methanol/triethylamine: 95/5/2 to give a colourless oil (0.93g). A solution of this oil (0.72g) in ethanol (10ml) was treated with a solution of citric acid (1.1g) in ethanol (10ml). Addition of ether precipitated a solid which was washed with further ether to give (±)-anti-N-methyl-2-(2-aminoethoxyimino)-1-(3-pyridyl)cyclohexanecarbothioamide citrate (1.15g), a colourless solid, m.p. 68-70°C.

### EXAMPLE 18

### Compounds N, O, P and Q

A stirred suspension of (±)-N-methyl-2-oxo-1-(3-pyridyl)cyclohexanecarbothioamide (2.48g) and methyl aminooxyacetate (1.1g) in ethanol (25ml) and pyridine (5ml) was refluxed for 24 hours. The solution was evaporated, the residue dissolved in chloroform (100ml), and washed with water (3x75ml). The chloroform phase was dried over magnesium sulphate and evaporated. The residual oil was purified by flash chromatography, eluting with ethyl acetate to give (±)-anti-N-methyl-2-methoxycarbonylmethoxyimino-1-(3-pyridyl) cyclohexanecarbothioamide (2.35g), a colourless solid, m.p. 129-131°C.

By proceeding in a similar manner, there were prepared (±)-anti-N-methyl-2-carbamoylmethoxyimino-1-(3-pyridyl)cyclohexanecarbothioamide, colourless solid, m.p. 168-169°C, after purification by flash chromatography on silica, eluting with ethyl acetate,followed by trituration with ether; (±)-anti-N-methyl-2-(2,3-dihydroxypropoxyimino)-1-(3-pyridyl)cyclohexanecarbothioamide, a yellow gum, after purification by flash chromatography on silica eluting with ethyl acetate/methanol:9/1; (±)-anti-N-methyl-2-(2-hydroxyethoxyimino)-1-(3-pyridyl)cyclohexanecarbothioamide, a yellow oil, after purification by flash chromatography on silica eluting with ethyl acetate.

### EXAMPLE 19

### Compound R

A vigorously stirred solution of 2-(6-chloropyrid-3-yl)cyclohexanone (0.36g 1.7mmol) in anhydrous tetrahydrofuran (10ml) under argon at -15°C was treated with potassium t-butoxide (0.19g, 1.7mmol). The resulting mixture was stirred for 2 hours at room temperature before the addition of a solution of methyl isothiocyanate (0.12g, 1.7mmol) in dry tetrahydrofuran (2ml). The mixture was stirred for a further 48 hours at room temperature, concentrated in vacuo and the residue treated with water (30ml) and extracted with ethyl acetate (50ml). The organic extract was dried over magnesium sulphate and then concentrated in vacuo (30°C; 1.9 kPa (14mmHg)) to give a yellow oil which was purified by flash chromatography over silica gel eluting with ethyl acetate: hexane 4:6 to give (±)-N-methyl-2-oxo-1-(6-chloropyrid-3-yl)cyclohexanecarbothioamide (0.25g) m.p. 172-3°C. NMR (CDCl₃) 1.64-2.14(m,4H); 2.4-2.65(m,2H); 2.7-2.8(m,2H); 3.14-3.16(d,3H); 7.26-7.34 (ddd,1H); 7.6-7.66(dd,1H); 8.2(d,1H); 8.84-9.06 (broad singlet, 1H). Calculated for C₁₃H₁₅ClN₂OS; C,55.2%; H,5.35%; N,9.91%, Cl,12.5%; Found C,55.3%; H,5.3%; N,9.8%;Cl,12.6%.

### EXAMPLE 20

### Compound S

A 33% w/v solution of methylamine in ethanol (5.7ml) was added dropwise to a stirred solution of 2-(5-bromopyrid-3-yl)-2-methylthiothiocarbonylcyclohexanone (1.33g, 3.86mmol) in ethanol (25ml) and dichloromethane (25ml) at 0-5°C. The solution was stirred at room temperature for 5 hours and concentrated in vacuo at 30-35°C. The residue was purified by flash chromatography over silica gel eluting with ethyl acetate: hexane 4:6 to give (±)-N-methyl-2-oxo-1-(5-bromopyrid-3-yl)cyclohexanecarbothioamide (0.57g) m.p. 201-3°C. NMR(CDCl₃) 1.68-2.16(m,4H); 2.46-2.6(m,2H); 2.66-2.8(m,2H); 3.17-3.17-3.19 (d,3H); 7.74-7.78(dd,1H); 8.38-8.39(d,1H), 8.58-8.59(d,1H); 8.8-9.0(broad singlet). Calculated for c₁₃H₁₅BrN₂OS; C,47.7%; H,4.6%; N,8.6%. Found C, 48.2%; H,4.7%; N,8.5%.

### REFERENCE EXAMPLE 17

To a solution of 2-(5-bromopyrid-3-yl)cyclohexanone (1.28g, 5.05mmol) in dry tetrahydrofuran (35ml) at -40°C was added portionwise with stirring potassium tert. butoxide (0.61g, 5.4mmol). The reaction mixture was stirred for 1 hour at -40°C and a solution of carbon disulphide (0.5ml) in dry tetrahydrofuran (5ml) was added and the reaction mixture allowed to warm up to -20°C over 20 minutes. A solution of methyl iodide (1.03g) in dry tetrahydrofuran (5ml) was added dropwise and the solution stirred at room temperature for 6 hours. The reaction mixture was concentrated in vacuo, treated with water (25ml) and extracted with dichloromethane (2 x 25ml). The extract was washed with water, dried over magnesium sulphate and concentrated to give 2-(5-bromopyrid-3-yl)-2-methylthiothiocarbonylcyclohexanone as an orange oil 1.33g. NMR (CDCl₃) 1.6-2.1(m,4H); 2.26-2.35(m,2H); 2.6 (s,3H); 2.65-2.9(m.1H); 3.4-3.5(m,1H), 7.84-7.86(dd,1H); 8.54-8.6(dd,2H).

### REFERENCE EXAMPLE 18

A solution of 3,5-dibromopyridine (30.45g, 0.13mol) in anhydrous ether (400ml) was added dropwise to a stirred solution of n-butyllithium (1.6M in hexane, 88.3ml) and anhydrous ether (40ml) over 0.5 hours at -70°C. The pale yellow suspension was stirred at -70°C for 1 hour, and a solution of 2-methoxycyclohexanone (16.5g, 0.13mol) in anhydrous ether (60ml) was added dropwise. The reaction mixture was stirred at -70°C for 2 hours and allowed to warm to room temperature. The reaction mixture was poured onto ice/water (1l); sodium chloride (50g) was added and the ether layer separated. The aqueous layer was extracted with ether (2 x 200ml). The combined ether layers were extracted with 2N hydrochloric acid (2 x 250ml). The acid extract was washed with ether (200ml), basified with 4N aqueous sodium hydroxide solution at room temperature (pH11), sodium chloride (50g) was added and the precipitated oil was extracted with ether (2 x 400ml). The combined extracts were dried over magnesium sulphate and concentrated in vacuo to give 2-methoxy-1-(5-bromopyrid-3-yl)cyclohexanol (30.6g). The cyclohexanol was added in small portions to stirred sulphuric acid (114ml). The reaction exothermed to 50°C, but no external cooling was applied. When the addition was completed after 20 minutes the red/brown solution was stirred at room temperature for 4 hours. The reaction mixture was poured onto ice/water (1l) and basified to pH8 with sodium carbonate. The product was extracted with ether (2x300ml). The ether extracts were combined, dried with magnesium sulphate and evaporated to give a pale orange oil. The oil was purified by flash chromatography over silica gel eluting with hexane/ethyl acetate 6.4 to give 2-(5-bromopyrid-3-yl)cyclohexanone (15.1g) m.p. 67-9°C. NMR(CDCl₃). 1.6-2.1(m,4H); 2.1-2.4(m,2H); 2.4-2.6 (m,2H); 3.6-3.66(dd,1H); 7.6(s,1H); 8.18(s,1H); 8.38(s,1H).

### EXAMPLE 21

### Compounds T and U

By proceeding in a similar manner to that hereinbefore described in Example 1 but replacing methyl isothiocyanate by ethyl isothiocyanate there was prepared (±)-N-ethyl-2-oxo-1-(3-pyridyl)cyclohexanecarbothioamide, a colourless solid, m.p. 126-130°C, after being purified by trituration with diethyl ether.

By proceeding in a similar manner to that hereinbefore described in Example 2 but replacing methyl isothiocyanate by ethyl isothiocyanate there was prepared (±)-N-ethyl-2-oxo-1-(3-quinolinyl)cyclohexanecarbothioamide, m.p. 156-158°C.

### EXAMPLE 22

### Compound W

A mixture of (±)-N-methyl-2-oxo-1-(3-quinolinyl)cyclohexanecarbothioamide (2.98g, 10mmol), 1,1-diethylhydrazine monohydrochloride (2.49g., 20mmol) and pyridine (15ml) was stirred and heated at 65-70°C for 31 hours, further quantities of 1,1-diethylhydrazine monohydrochloride (2.49g, 20mmol) being added after 20 hours and 31 hours during this period. The reaction mixture was then diluted with di-isopropyl ether (20ml) and pyridine (5ml), stirred at 100-150°C for 52 hours under reflux, added to ice (100g) and the pH of the mixture adjusted to 8 by the addition of sodium hydrogen carbonate. The crude product was extracted using dichloromethane (80ml; 3x20ml), the combined extracts washed with saturated brine solution (2x20ml) and dried over a mixture of magnesium sulphate and charcoal. The solvent was removed in vacuo to give a brown gum, which was purified by flash chomatography over silica gel, using a mixture of toluene: acetone (9:1) as eluting solvent, followed by recrystallisation twice from a mixture of petroleum ether (b.p. 60-80°C): diethyl ether (2:1). This procedure afforded (±)-N-anti-methyl-2-diethylhydrazono-1-(3-quinolinyl)cyclohexanecarbothioamide (1.00g, 2.7mmol) as a peach solid, melting point 117-118°C.

NMR-(CDCl₃); 1.04-1.15 (t,6H), 1.60-1.90 (c,4H), 1.92-2.06(c,1H), 2.30-2.62 (qq,2H), 2.66-2.82 (m,3H), 2.82-2.98 (m,1H), 3.06-3.21 (dm,4H), 7.44-7.56 (m,1H), 7.60-7.79 (m,2H), 8.00-8.08 (m,2H), 8.84-8.88 (d,1H), 10.94-11.08 (broad singlet, 1H).

Microanalysis; found; C,68.3; H,7.6; N,15.2; S,9.0%. C₂₁H₂₈N₄S requires; C,68.4; H,7.7; N 15.2; S,8.7%.

### EXAMPLE 23

### Compounds X to AK

By proceeding in a similar manner to that hereinbefore described in Example 7, there were prepared:-
(±)-anti-N-methyl-2-benzyloxyimino-1-(3-quinolinyl)cyclohexanecarbothioamide, m.p. 158-160°C;
(±)-anti-N-methyl-2-methoxyimino-1-(3-quinolinyl)cyclohexanecarbothioamide, m.p. 179-181°C;
(±)-anti-N-methyl-2-ethoxyimino-1-(3-quinolinyl)cyclohexanecarbothioamide, m.p. 190-192°C;
(±)-anti-N-methyl-2-butoxyimino-1-(3-quinolinyl)cyclohexanecarbothioamide, m.p. 165-167°C;
(±)-anti-N-methyl-2-(3-isopropylamino-2-hydroxypropoxyimino)-1-(3-quinolinyl)cyclohexanecarbothioamide, m.p. 50°C;
(±)-anti-N-methyl-2-t.butoxyimino-1-(3-quinolinyl)cyclohexanecarbothioamide, m.p. 158-160°C;
(±)-anti-N-methyl-2-prop-2-enoxyimino-1-(3-quinolinyl)cyclohexanecarbothioamide, m.p. 162-165°C;
(±)-anti-N-methyl-2-naphth-2-ylmethoxyimino-1-(3-quinolinyl)cyclohexanecarbothioamide, m.p. 180-183°C;
(±)-anti-N-methyl-2-phenethyloxyimino-1-(3-quinolinyl)cyclohexanecarbothioamide, m.p. 145-147°C;
(±)-anti-N-methyl-2-naphth-1-ylmethoxyimino-1-(3-quinolinyl)cyclohexanecarbothioamide, m.p. 184-187°C;
(±)-anti-N-methyl-2-(3-t.butylamino-2-hydroxypropoxyimino)-1-(3-quinolinyl)cyclohexanecarbothioamide hemihydrate, m.p. 65°C;
(±)-anti-N-methyl-2-isopropoxyimino-1-(3-quinolinyl)cyclohexanecarbothioamide, m.p. 208-210°C;
(±)-anti-N-methyl-2-(4-hydroxybenzyloxyimino)-1-(3-quinolinyl)cyclohexanecarbothioamide hydrate, m.p. 190-191°C;
(±)-anti-N-methyl-2-(4-fluorobenzyloxyimino)-1-(3-quinolinyl)cyclohexanecarbothioamide, m.p. 143-144°C.

### EXAMPLE 24

### Compounds AL to AY

By proceeding in a similar manner to that hereinbefore described in Example 7, there were prepared:-
(±)-anti-N-methyl-2-(4-fluorobenzyloxyimino)-1-(3-pyridyl)cyclohexanecarbothioamide, a white powder; m.p. 110-11°C.
(±)-anti-N-methyl-2-(2,3,4,5,6-pentafluorobenzyloxyimino)-1-(3-pyridyl)cyclohexanecarbothioamide, m.p. 113-115°C;
(±)-anti-N-methyl-2-isopropoxyimino-1-(3-pyridyl)cyclohexanecarbothioamide, m.p. 168-170°C;
(±)-anti-N-methyl-2-t.butoxyimino-1-(3-pyridyl)cyclohexanecarbothioamide, m.p. 128-130°C;
(±)-anti-N-methyl-2-[4-(3-t.butylamino-2-hydroxypropoxy)benzyloxyimino]-1-(3-pyridyl)cyclohexanecarbothioamide, m.p. 39-43°C;
(±)-anti-N-methyl-2-(4-hydroxybenzyloxyimino)-1-(3-pyridyl)cyclohexanecarbothioamide, m.p. 169-171°C;
(±)-anti-N-methyl-2-(3-fluorobenzyloxyimino)-1-(3-pyridyl)cyclohexanecarbothioamide, m.p. 117-120°C
(±)-anti-N-methyl-2-(2-fluorobenzyloxyimino)-1-(3-pyridyl)cyclohexanecarbothioamide, m.p. 118-121°C
(±)-anti-N-methyl-2-(3-pyridylmethoxyimino)-1-(3-pyridyl)cyclohexanecarbothioamide, m.p. 150-152°C;
(±)-anti-N-methyl-2-(4-nitrobenzyloxyimino)-1-(3-pyridyl)cyclohexanecarbothioamide, m.p. 121-125°C;
(±)-anti-N-methyl-2-(4-cyanobenzyloxyimino)-1-(3-pyridyl)cyclohexanecarbothioamide, m.p. 125-136°C;
(±)-anti-N-methyl-2-(3,4-difluorobenzyloxyimino)-1-(3-pyridyl)cyclohexanecarbothioamide, m.p. 123-125°C;
(±)-anti-N-methyl-2-(4-methoxybenzyloxyimino)-1-(3-pyridyl)cyclohexanecarbothioamide, m.p. 101-103°C;
(±)-anti-N-methyl-2-(2-trifluoromethylbenzyloxyimino)-1-(3-pyridyl)cyclohexanecarbothioamide hemihydrate, m.p. 133-136°C.

### EXAMPLE 25

By proceeding in a similar manner to Example 15, there was prepared:-
the (R)-isomer, by inference, of anti-N-methyl-2-benzyloxyimino-1-(3-pyridyl)cyclohexanecarbothioamide, m.p. 115°C, from N-methyl-2-oxo-1-(3-pyridyl)cyclohexanecarbothioamide (prepared in Example 29);
the (R)-isomer, by inference, of anti-N-methyl-2-(4-fluorobenzyloxyimino)-1-(3-pyridyl)cyclohexanecarbothioamide, m.p. 89°C, from N-methyl-2-oxo-1-(3-pyridyl)cyclohexanecarbothioamide (prepared in Exampled 29);
the (S)-isomer, by inference, of anti-N-methyl-2-(4-fluorobenzyloxyimino)-1-(3-pyridyl)-cyclohexanecarbothioamide, m.p. 104-106°C from N-methyl-2-oxo-1-(3-pyridyl)cyclohexanecarbothioamide (prepared in Example 14).

### EXAMPLE 26

### Compound AZ

By proceeding in a similar manner to Example 1 but replacing (±)-2-(3-pyridyl)cyclohexanone by (±)-2-(4-isoquinolinyl)cyclohexanone there was prepared:-
(±)-N-methyl-2-oxo-1-(4-isoquinolinyl)cyclohexanecarbothioamide, m.p. 216-218°C.
(±)-2-(4-Isoquinolinyl)cyclohexanone may be prepared in a similar manner to that hereinbefore described in Reference Example 4.

### EXAMPLE 27

### Compound BA

By proceeding in a similar manner to Example 7 there was prepared:-
(±)-anti-N-methyl-2-benzyloxyimino-1-(4-isoquinolinyl)cyclohexanecarbothioamide 0.75 hydrate, m.p. 166-168°C.

### EXAMPLE 28

### Compound BB

By proceeding in a similar manner to Example 1 but replacing (±)-2-(3-pyridyl)cyclohexanone by (±)-2-(5-pyrimidyl)cyclohexanone there was prepared:-
(±)-N-methyl-2-oxo-1-(5-pyrimidyl)cyclohexanecarbothioamide, m.p. 145-148°C.
(±)-2-(5-Pyrimidyl)cyclohexanone may be prepared in a similar manner to that hereinbefore described in Reference Example 4.

### EXAMPLE 29

By proceeding in a similar manner to Example 14, there was prepared:-
the (R)-isomer, by inference, of N-methyl-2-oxo-1-(3-pyridyl)cyclohexanecarbothioamide, m.p. 185°C.

### EXAMPLE 30

### Compound BC

By proceeding in a similar manner to Example 1 but replacing (±)-2-(3-pyridyl)cyclohexanone by (±)-2-(3-pyridyl)cyclopentanone there was prepared:-
(±)-N-methyl-2-oxo-1-(3-pyridyl)cyclopentanecarbothioamide, m.p. 131-132°C.
(±)-2-(3-Pyridyl)cyclopentanone may be prepared in a similar manner to that hereinbefore described in Reference Example 4.

### EXAMPLE 31

### Compounds BD, BE and BF

By proceeding in a similar manner to that hereinbefore described in Example 7, there were prepared:-
(±)-anti-N-methyl-2-(4-fluorobenzyloxyimino)-1-(3-pyridyl)cyclopentanecarbothioamide, m.p. 105-106°C;
(±)-anti-N-methyl-2-methoxyimino-1-(3-pyridyl)cyclopentanecarbothioamide, m.p. 123-124°C;
(±)-anti-N-methyl-2-benzyloxyimino-1-(3-pyridyl)cyclopentanecarbothioamide, m.p. 121-122°C.

### EXAMPLE 32

### Compound V

To a solution of (±)-anti-2-dimethylhydrazono-1-(3-quinolinyl)cyclohexane (1.34g, 5 mmol) in dry tetrahydrofuran (10ml) at -78°C under an atmosphere of argon was added a solution of 2.5M n-butyl lithium in hexane (4.0ml, 10mmol). After stirring at the same temperature for 30 minutes the reaction mixture was treated with a solution of methyl isothiocyanate (0.73g, 10mmol) in dry tetrahydrofuran (5ml) and stirred for a further 30 minutes at -78°C. Finally the mixture was stirred for 2 hours as the temperature was allowed to rise to 25°C and for 18 hours thereafter. The resultant pale brown solution was treated with a 1% v/v aqueous solution of acetic acid, the crude product extracted using dichloromethane (3x50ml) and the combined extracts washed with saturated brine solution (40ml). These extracts were dried over magnesium sulphate and the solvent removed in vacuo to give a red oil, which was purified by flash chromatography over silica gel, using ethyl acetate as eluting solvent, followed by recrystallisation from petroleum ether (b.p. 60-80°C) to afford (±)-N-anti-methyl-2-dimethylhydrazono-1-(3-quinolinyl)cyclohexanecarbothioamide (0.55g., 1.6mmol) as a white solid, melting point 140-142°C.

NMR (CDCl₃); 1.62-1.88(c,4H), 1.88-2.09 (c,1H), 2.40-2.60(s,s,6H), 2.62-2.80 (m,2H), 2.92-3.10 (c,1H) 3.10-3.22 (d,3H), 7.48-7.58 (m,1H), 7.62-7.80 (m,2H), 7.98-8.08 (m,2H), 8.82-8.90 (d,1H), 10.44-10.60 (broad singlet, 1H).

Microanalysis: found; C,66.9; H,7.2; N,16.3; S,9.5%.

C₁₉H₂₄N₄S requires; C,67.0; H,7.1; N,16.5; S,9.4%.
(±)-anti-2-Dimethylhydrazono-1-(3-quinolinyl)cyclohexane was prepared as follows:-
To a warm solution of (±)-2-(3-quinolyl)cyclohexanone (9.00g, 40mmol) in ethanol (30ml.) was added 1,1-dimethylhydrazine (3.61g, 60mmol) followed by concentrated hydrochloric acid (4 drops). The solution was allowed to stand at 25°C for 3 days, added to ice (150g.) and the pH of the mixture adjusted to 9 by the addition of a M aqueous sodium carbonate solution. The crude product was extracted using diethyl ether (3x75ml), the combined extracts washed with saturated brine solution (50ml) and dried over magnesium sulphate. Removal of the solvent in vacuo yielded a yellow oil (11.00g), which was extracted several times with hot portions (40ml) of n-pentane, the extracts being separated by decantation from insoluble material. The combined, concentrated extracts were kept at 0°C for several hours to give a solid (10.24g), which was further purified by recrystallisation from n-pentane (40ml) to afford (±)-anti-2-dimethylhydrazono-1-(3-quinolyl)cyclohexane (7.90g, 29.5mmol) as a pale cream solid, melting point 64-66°C.

NMR (CDCl₃); 1.66-1.96 (c,4H), 2.08-2.26 (c,1H), 2.26-2.40 (c,1H), 2.44-2.50 (s,6H), 2.59-2.71 (c,2H), 3.78-3.88 (t,1H), 7.50-7.62 (m,1H), 7.64-7.77 (m,1H), 7.78-7.86 (m,1H), 7.95-8.04 (s,1H), 8.08-8.15 (m,1H), 8.88-8.94 (d,1H).

| | | | |
|---|---|---|---|
| Microanalysis: found: | C,76.3; | H,8.0; | N,15.6%. |
| C₁₇H₂₁N₃ requires: | C,76.4; | H,7.9; | N,15.7%. |

### REFERENCE EXAMPLE 19

A stirred solution of 2-(4-fluorobenzyloxyimino) -1-pyrid-3-ylcyclohexane (150mg, 0.5mmol) in tetrahydrofuran (2ml) at -78°C was treated with 1.5M butyl lithium in hexane (0.44ml, 0.65mmol) over 5 minutes. After 30 minutes at -78°C the solution was treated with carbon disulphide (57mg, 0.75mmol) in tetrahydrofuran (0.5ml). The temperature of the reaction mixture was then allowed to rise, over 30 minutes to 0°C, and was then treated with a solution of methyl iodide (107mg, 0.75mmol) in tetrahydrofuran (0.5ml) over 10 minutes. The reaction mixture was allowed to warm to 20°C over 30 minutes, and then concentrated in vacuo. The crude oil was dissolved in dichloromethane (10ml) and washed successively with water (2x2ml), and saturated brine (2ml) then dried over magnesium sulphate. The solution was filtered then concentrated in vacuo to give an amber oil which was purified by flash chromatography over silica gel eluting with ethyl acetate to yield methyl 2-(4-fluorobenzyloxyimino)-1-pyrid-3-ylcyclohexanecarbodithioate (102mg).

### EXAMPLE 33

### Compound AL

A stirred solution of methyl 2-(4-fluorobenzyloxyimino)-1-pyrid-3-ylcyclohexanecarbodithioate (20mg, 0.05mmol) in ethanol (1ml) at 20°C was treated with a 33% w/v solution of methylamine in ethanol (100µl, 0.8mmol). After 70 hours at 20°C the mixture was concentrated in vacuo and the resulting oil recrystallised from cyclohexane to give (±)-anti-N-methyl-2-(4-fluorobenzyloxyimino)-1-(3-pyridyl)cyclohexanecarbothioamide (18mg).

The NMR characteristics were the same as those of the product described in Example 24.

### REFERENCE EXAMPLE 20

A mixture of (±)-2-(3-pyridyl)cyclopentanone (2.0g) and benzyloxyamine hydrochloride (1.98g) in anhydrous pyridine was stirred at 60°C for 4.5 hours. The pyridine was removed in vacuo (40°C, 0.027 kPa (0.2mmHg)), the residue was dissolved in chloroform (75ml), washed with water (4x75ml), dried over magnesium sulphate and evaporated. The residual oil was purified by flash chromatography on silica, eluting with chloroform/methanol (100/1) to give (±)-2-benzyloxyimino-1-(3-pyridyl)cyclopentane (mixture of syn and anti isomers, 1:3), a pale yellow oil (2.91g).

### EXAMPLE 34

### Compound BF

A stirred solution of (±)-2-benzyloxyimino-1-(3-pyridyl)cyclopentane (mixture of syn and anti isomers) (2.66g) in tetrahydrofuran (35ml), under argon, at -78°C, was treated with 2.5M butyl lithium in hexane (4.6ml) during 7 minutes at -70°C. After stirring at -70°C for 35 minutes, the dark red solution was treated with methyl isothiocyanate (0.84g) in tetrahydrofuran (6ml). The solution was allowed to warm to 0°C during 45 minutes and maintained at 0°C for 1 hour. The solution was partitioned between saturated ammonium chloride solution (40ml) and ethyl acetate (40ml). The organic layer was separated and the aqueous layer extracted with ethyl acetate (40ml). The combined organic layers were washed with water (3x40ml) dried over magnesium sulphate and evaporated. The residual solid was purified by flash chromatography on silica, eluting with ethyl acetate/toluene (1:2) to give (±)-anti-N-methyl-2-benzyloxyimino-1-(3-pyridyl)cyclopentanecarbothioamide, a colourless solid (1.72g), m.p. 117-118.5°C.

The NMR characteristics were the same as those of the product described in Example 31.

The present invention includes within its scope pharmaceutical compositions which comprise a compound of general formula (I) or a pharmaceutically acceptable salt thereof, in association with a pharmaceutically acceptable carrier or coating. In clinical practice the compounds of the present invention may be administered rectally, but are preferably administered parenterally, by inhalation if appropriate, or, more preferably, orally.

Solid compositions for oral administration include compressed tablets, pills, powders and granules. In such solid compositions, one or more of the active compounds is, or are, admixed with at least one inert diluent such as starch, sucrose or lactose. The compositions may also comprise, as is normal practice, additional substances other than inert diluents, e.g. lubricating agents, such as magnesium stearate.

Liquid compositions for oral administration include pharmaceutically acceptable emulsions, solutions, suspensions, syrups and elixirs containing inert diluents commonly used in the art such as water and liquid paraffin. Besides inert diluents such compositions may comprise adjuvants, such as wetting, and suspending agents, and sweetening, flavouring, perfuming and preserving agents. The compositions according to the invention for oral administration also include capsules of absorbable material such as gelatin, containing one or more of the active substances with or without the addition of diluents or excipients.

Preparations according to the invention for parenteral administration include sterile aqueous, aqueous-organic, and organic solutions, suspensions and emulsions. Examples of organic solvents or suspensing media are propylene glycol, polyethylene glycol, vegetable oils such as olive oil and injectable organic esters such as ethyl oleate. The compositions may also contain adjuvant such as stabilising, preserving, wetting, emulsifying and dispersing agents. They may be sterilized by, for example, filtration through a bacteria-retaining filter, by incorporation in the compositions of sterilizing agents, by irradiation or by heating. They may also be manufactured in the form of sterile solid compositions, which can be dissolved in sterile water or some other sterile injectable medium immediately before use.

Compositions for inhalation may be sterile aqueous solutions which are then nebulised or dry powders formulated in accordance with known methods.

Solid compositions for rectal administration include suppositories formulated in accordance with known methods and containing one or more of the compounds of formula (I) or a pharmaceutically acceptable salt thereof.

The percentage of active ingredient in the compositions of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration and the duration of the treatment, and the condition of the patient. In the adult, the doses are generally from 0.001 to 50 mg/kg body weight per day by oral administration. By inhalation, either as a nebulised solution or as a formulated dry powder, the preferred daily dosage is from 0.001 to 5 mg/kg body weight. The compounds may also be applied topically for inhibition of head hair loss associated with male pattern baldness, the preferred daily dosage being from 0.1 to 10 mg/kg body weight applied, for example, in 5ml portions two or three times per day.

The following Example illustrates pharmaceutical compositions according to the present invention.

### COMPOSITION EXAMPLE

No. 2 size gelatin capsules each containing:-(±)-N-methyl-2-oxo-1-(3-pyridyl)cyclohexane

| | |
|---|---|
| carbothioamide | 20 mg |
| lactose | 100 mg |
| starch | 60 mg |
| dextrin | 40 mg |
| magnesium stearate | 1 mg |

were prepared in accordance with the usual procedure.

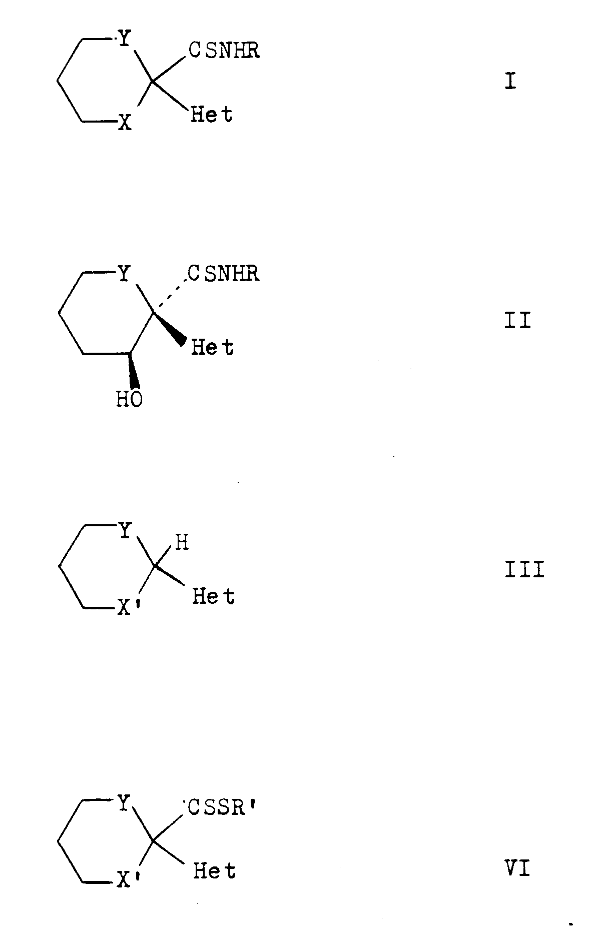

## Claims (Claims for the following Contracting State(s): AT, BE, CH, DE, FR, GB, IT, LI, LU, NL, SE)

1. A thioformamide derivative of the general formula: 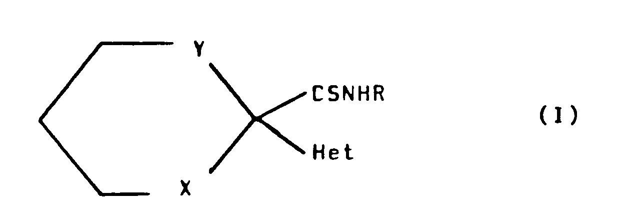 wherein R represents a straight- or branched-chain alkyl radical containing from 1 to 4 carbon atoms, Het represents an aromatic heterocyclic radical containing one or two nitrogen atoms (optionally substituted by a straight- or branched-chain alkyl or alkoxy radical containing 1 to 4 carbon atoms or by a halogen atom), selected from pyrid-3-yl, isoquinolin-4-yl, tetrahydroquinolin-3-yl, quinolin-3-yl, pyridazin-4-yl, pyrimid-5-yl, thiazol-5-yl, thieno[2,3-b]pyridin-5-yl, pyrazin-2-yl, indol-3-yl and thieno[3,2-b]pyridin-6-yl, Y represents an ethylene or methylene radical, or a valency bond, and X represents a carbonyl or hydroxymethylene group or a group of the formula: 〉C=NOR¹, 〉C=NN(R¹)₂ or 〉C=NN(R¹)CON(R¹)₂ in which the symbols R¹, which may be the same or different, each represents the hydrogen atom or a straight- or branched-chain alkyl radical containing from 1 to 4 carbon atoms which is unsubstituted or substituted by one or more substituents selected from C₂₋₄-alkenyl, carboxy, C₂₋₅-alkoxycarbonyl, hydroxy, C₁₋₄-alkoxy, carbamoyl (unsubstituted or substituted by one or two C₁₋₄-alkyl groups), amino, C₁₋₄-alkylamino and di-C₁₋₄-alkylamino groups or represents a benzyl, phenethyl, 1-naphthylmethyl, 2-naphthylmethyl or pyrid-3-ylmethyl radical each of which may be substituted on the ring by one or more halogen atoms or hydroxy, C₁₋₄-alkyl, C₁₋₄-alkoxy (alkoxy being unsubstituted or substituted as defined for alkyl groups represented by R¹) cyano, nitro, trifluoromethyl, carboxy, C₁₋₄-alkylamino, C₂₋₅-alkanoylamino, C₂₋₅-alkoxycarbonyl groups or two R¹ substituents on the same nitrogen atom may together form a straight- or branched-chain alkylene radical containing from 4 to 6 carbon atoms in the chain which is unsubstituted or substituted as defined for alkyl groups represented by R¹, and pharmaceutically acceptable salts thereof.

2. A compound according to claim 1 wherein Y represents a methylene radical or a valency bond.

3. A compound according to claim 1 or 2 wherein X represents the carbonyl group or a group of the formula 〉C=NOR¹ wherein R¹ is as defined in claim 1.

4. A compound according to claim 1, 2 or 3 wherein R¹ represents a straight- or branched-chain alkyl radical containing from 1 to 4 carbon atoms which is unsubstituted or substituted as defined in claim 1, or represents a benzyl, phenethyl, 1-naphthylmethyl, 2-naphthylmethyl or pyrid-3-yl-methyl radical each of which may be substituted on the ring as defined in claim 1.

5. A compound according to any one of the preceding claims wherein Het represents 3-pyridyl, 6-chloropyrid-3-yl, 5-bromopyrid-3-yl, 3-quinolinyl, 4-isoquinolinyl or 5-pyrimidyl.

6. A compound according to any one of claims 1, 2, 4 and 5 of the general formula: 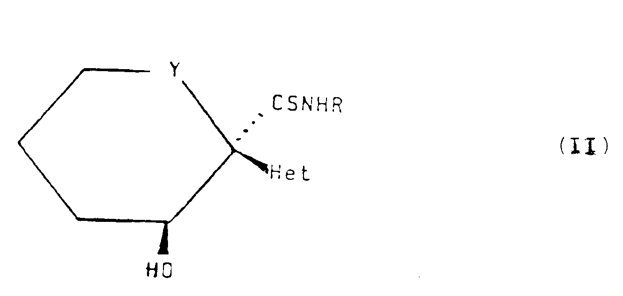 wherein R, Het and Y are as defined in any one of claims 1 to 5 in which the hydroxy group is in the trans position relative to the group -CSNHR.

7. A compound according to claim 1 which is:
(±)-N-methyl-2-oxo-1-(3-pyridyl)cyclohexanecarbothioamide
(±)-N-methyl-2-oxo-1-(3-quinolinyl)cyclohexanecarbothioamide
(±)-N-methyl-2-oxo-1-(3-pyridyl)cycloheptanecarbothioamide
(±)-trans-N-methyl-2-hydroxy-1-(3-pyridyl)cyclohexanecarbothioamide
(±)-cis-N-methyl-2-hydroxy-1-(3-pyridyl)cyclohexanecarbothioamide
(±)-anti-N-methyl-2-hydroximino-1-(3-pyridyl)cyclohexanecarbothioamide
(±)-anti-N-methyl-2-methoxyimino-1-(3-pyridyl)cyclohexanecarbothioamide
(±)-anti-N-methyl-2-benzyloxyimino-1-(3-pyridyl)cyclohexanecarbothioamide
(±)-anti-N-methyl-2-hydroxyimino-1-(3-quinolinyl)cyclohexanecarbothioamide
(2S)-anti-2-(methoxymethyl)-1-[2'-(3-quinolinyl)-2'-methylthiocarbamoylcyclohexylideneamino]pyrrolidine
(2S)-anti-2-(methoxymethyl)-1-[2'-(3-pyridyl)-2'-methylthiocarbamoylcyclohexylideneamino]pyrrolidine,
(±)-anti-N-methyl-2-(2-dimethylaminoethoxyimino)-1-(3-pyridyl)cyclohexanecarbothioamide,
(±)-anti-N-methyl-2-(2-aminoethoxyimino)-1-(3-pyridyl) cyclohexanecarbothioamide
(±)-anti-N-methyl-2-methoxycarbonylmethoxyimino-1-(3-pyridyl)cyclohexanecarbothioamide
(±)-anti-N-methyl-2-carbamoylmethoxyimino-1-(3-pyridyl)cyclohexanecarbothioamide,
(±)-anti-N-methyl-2-(2,3-dihydroxypropoxyimino)-1-(3-pyridyl)cyclohexanecarbothioamide
(±)-anti-N-methyl-2-(2-hydroxyethoxyimino)-1-(3-pyridyl)cyclohexanecarbothioamide
(±)-N-methyl-2-oxo-1-(6-chloropyrid-3-yl)cyclohexanecarbothioamide
(±)-N-methyl-2-oxo-1-(5-bromopyrid-3-yl)cyclohexanecarbothioamide
(±)-N-ethyl-2-oxo-1-(3-pyridyl)cyclohexanecarbothioamide
(±)-N-ethyl-2-oxo-1-(3-quinolinyl)cyclohexanecarbothioamide
(±)-anti-N-methyl-2-dimethylhydrazono-1-(3-quinolinyl)cyclohexanecarbothioamide
(±)-anti-N-methyl-2-diethylhydrazono-1-(3-quinolinyl) cyclohexanecarbothioamide
(±)-anti-N-methyl-2-benzyloxyimino-1-(3-quinolinyl)cyclohexanecarbothioamide
(±)-anti-N-methyl-2-methoxyimino-1-(3-quinolinyl)cyclohexanecarbothioamide
(±)-anti-N-methyl-2-ethoxyimino-1-(3-quinolinyl)cyclohexanecarbothioamide
(±)-anti-N-methyl-2-butoxyimino-1-(3-quinolinyl)cyclohexanecarbothioamide
(±)-anti-N-methyl-2-(3-isopropylamino-2-hydroxypropoxyimino)-1-(3-quinolinyl)cyclohexanecarbothioamide
(±)-anti-N-methyl-2-t.butoxyimino-1-(3-quinolinyl)cyclohexanecarbothioamide
(±)-anti-N-methyl-2-prop-2-enoxyimino-1-(3-quinolinyl)cyclohexanecarbothioamide
(±)-anti-N-methyl-2-naphth-2-ylmethoxyimino-1-(3-quinolinyl)cyclohexanecarbothioamide
(±)-anti-N-methyl-2-phenethyloxyimino-1-(3-quinolinyl)cyclohexanecarbothioamide
(±)-anti-N-methyl-2-naphth-1-ylmethoxyimino-1-(3-quinolinyl)cyclohexanecarbothioamide
(±)-anti-N-methyl-2-(3-t.butylamino-2-hydroxypropoxyimino-1-(3-quinolinyl)cyclohexanecarbothioamide
(±)-anti-N-methyl-2-isopropoxyimino-1-(3-quinolinyl)cyclohexanecarbothioamide
(±)-anti-N-methyl-2-(4-hydroxybenzyloxyimino-1-(3-quinolinyl)cyclohexanecarbothioamide
(±)-anti-N-methyl-2-(4-fluorobenzyloxyimino)-1-(3-quinolinyl)cyclohexanecarbothioamide
(±)-anti-N-methyl-2-(4-fluorobenzyloxyimino)-1-(3-pyridyl)cyclohexanecarbothioamide
(±)-anti-N-methyl-2-(2,3,4,5,6-pentafluorobenzyloxyimino)-1-(3-pyridyl)cyclohexanecarbothioamide
(±)-anti-N-methyl-2-isopropoxyimino-1-(3-pyridyl)cyclohexanecarbothioamide
(±)-anti-N-methyl-2-t.butoxyimino-1-(3-pyridyl)cyclohexanecarbothioamide
(±)-anti-N-methyl-2-[4-(3-t.butylamino-2-hydroxypropoxy)benzyloxyimino]-1-(3-pyridyl)cyclohexanecarbothioamide
(±)-anti-N-methyl-2-(4-hydroxybenzyloxyimino)-1-(3-pyridyl)cyclohexanecarbothioamide
(±)-anti-N-methyl-2-(3-fluorobenzyloxyimino)-1-(3-pyridyl)cyclohexanecarbothioamide
(±)-anti-N-methyl-2-(2-fluorobenzyloxyimino)-1-(3-pyridyl)cyclohexanecarbothioamide
(±)-anti-N-methyl-2-(3-pyridylmethoxyimino)-1-(3-pyridyl)cyclohexanecarbothioamide
(±)-anti-N-methyl-2-(4-nitrobenzyloxyimino)-1-(3-pyridyl)cyclohexanecarbothioamide,
(±)-anti-N-methyl-2-(4-cyanobenzyloxyimino)-1-(3-pyridyl)cyclohexanecarbothioamide
(±)-anti-N-methyl-2-(3,4-difluorobenzyloxyimino)-1-(3-pyridyl)cyclohexanecarbothioamide
(±)-anti-N-methyl-2-(4-methoxybenzyloxyimino)-1-(3-pyridyl)cyclohexanecarbothioamide
(±)-anti-N-methyl-2-(2-trifluoromethylbenzyloxyimino)-1-(3-pyridyl)cyclohexanecarbothioamide
(±)-N-methyl-2-oxo-1-(4-isoquinolinyl)cyclohexanecarbothioamide
(±)-anti-N-methyl-2-benzyloxyimino-1-(4-isoquinolinyl)-cyclohexanecarbothioamide
(±)-N-methyl-2-oxo-1-(5-pyrimidyl)cyclohexanecarbothioamide
(±)-N-methyl-2-oxo-1-(3-pyridyl)cyclopentanecarbothioamide
(±)-anti-N-methyl-2-(4-fluorobenzyloxyimino)-1-(3-pyridyl)cyclopentanecarbothioamide
(±)-anti-N-methyl-2-methoxyimino-1-(3-pyridyl)cyclopentanecarbothioamide or
(±)-anti-N-methyl-2-benzyloxyimino-1-(3-pyridyl)cyclopentanecarbothioamide
or a pharmaceutically acceptable salt thereof.

8. A process for the preparation of a thioformamide derivative of general formula (I) depicted in claim 1 which comprises:-
(A) when X represents the carbonyl group or a group of
formula 〉C=NOR¹ or 〉C=NN(R¹)₂ wherein R¹ is as defined in claim 1 and Y, R and Het are as defined in claim 1 the reaction of a compound of the general formula: 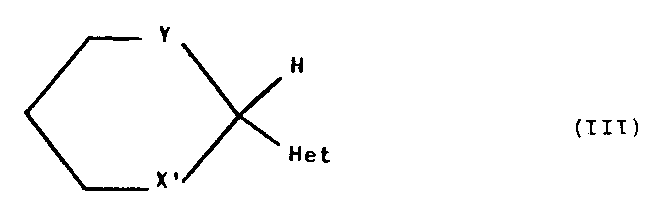 wherein X' represents the carbonyl group or a group of formula 〉C=NOR¹ or 〉C=NN(R¹)₂ as defined in claim 1 and Y and Het are as defined in claim 1 with an isothiocyanate of the general formula:
R-N=C=S (IV)
wherein R represents a straight- or branched-chain alkyl radical containing 1 to 4 carbon atoms;
(B) when X represents the carbonyl group the stereoselective reaction of a mixture of enantiomers of general formula (III) wherein Y and Het are as defined in claim 1 and X' represents the carbonyl group with a chiral auxiliary agent and reaction of the product obtained with a compound of the general formula:
R-N=C=S (IV)
wherein R is as defined in claim 1 followed by removal of the chiral auxiliary agent;
(C) when X represents the hydroxymethylene group and Y, R and Het are as defined in claim 1 the reduction of a compound of general formula (I) wherein X represents the carbonyl group and Y, R and Het are as defined in claim 1;
(D) when X represents a group of the formula 〉C=NOR¹, 〉C=NN(R¹)₂ or 〉C=NN(R¹)CON(R¹)₂ as defined in claim 1 and Y, R and Het are as defined in claim 1 the reaction of a compound of general formula (I) wherein X represents the carbonyl group with a compound of general formula:
NH₂OR¹ (Va)
NH₂N(R¹)₂ (Vb)
or
NH₂N(R¹)CON(R¹)₂ (Vc)
wherein R¹ is as defined in claim 1 or with an acid addition salt thereof;
(E) when R represents the methyl radical and X represents the carbonyl group or a group of the formula 〉C=NOR¹ or 〉C=NN(R¹)₂ as defined in claim 1, and Y and Het are as defined in claim 1 the reaction of methylamine with a dithioester of the general formula: 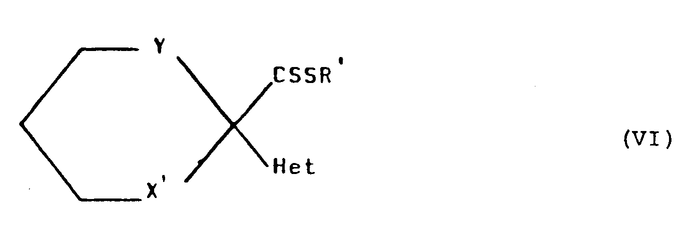 wherein R' represents a straight- or branched-chain alkyl radical containing 1 to 4 carbon atoms, or a benzyl or carboxymethyl radical, X' is as hereinbefore defined and Y and Het are as defined in claim 1;
optionally followed by the step of converting a thioformamide derivative thus obtained into a pharmaceutically acceptable salt thereof.

9. A pharmaceutical composition which comprises a thioformamide derivative of general formula (I) as defined in claim 1 or a pharmaceutically acceptable salt thereof in association with a pharmaceutically acceptable carrier or coating.

10. A compound of general formula (III) depicted in claim 8 wherein Y and Het are as defined in claim 1 and X' is as defined in claim 8 with the proviso that Y is not a direct bond when Het represents an optionally substituted pyrazin-2-yl group and Y does not represents methylene when Het represents an optionally substituted indol-3-yl group.

## Claims (Claims for the following Contracting State(s): ES)

1. A process for the preparation of a thioformamide derivative of the general formula: 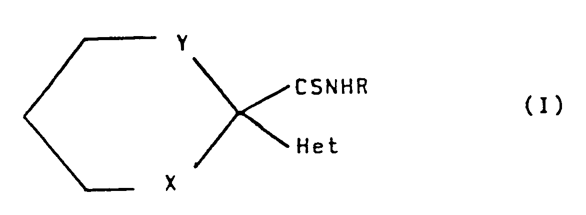 wherein R represents a straight- or branched-chain alkyl radical containing from 1 to 4 carbon atoms, Het represents an aromatic heterocyclic radical containing one or two nitrogen atoms (optionally substituted by a straight- or branched-chain alkyl or alkoxy radical containing 1 to 4 carbon atoms or by a halogen atom), selected from pyrid-3-yl, isoquinolin-4-yl, tetrahydroquinolin-3-yl, quinolin-3-yl, pyridazin-4-yl, pyrimid-5-yl, thiazol-5-yl, thieno[2,3-b]pyridin-5-yl, pyrazin-2-yl, indol-3-yl and thieno[3,2-b]pyridin-6-yl, Y represents an ethylene or methylene radical, or a valency bond, and X represents a carbonyl or hydroxymethylene group or a group of the formula: 〉C=NOR¹, 〉C=NN(R¹)₂ or 〉C=NN(R¹)CON(R¹)₂ in which the symbols R¹, which may be the same or different, each represents the hydrogen atom or a straight- or branched-chain alkyl radical containing from 1 to 4 carbon atoms which is unsubstituted or substituted by one or more substituents selected from C₂₋₄-alkenyl, carboxy, C₂₋₅-alkoxycarbonyl, hydroxy, C₁₋₄-alkoxy, carbamoyl (unsubstituted or substituted by one or two C₁₋₄-alkyl groups), amino, C₁₋₄ alkylamino and di-C₁₋₄-alkylamino groups or represents a benzyl, phenethyl, 1-naphthylmethyl, 2-naphthylmethyl or pyrid-3-ylmethyl radical each of which may be substituted on the ring by one or more halogen atoms or hydroxy, C₁₋₄-alkyl, C₁₋₄-alkoxy (alkoxy being unsubstituted or substituted as defined for alkyl groups represented by R¹) cyano, nitro, trifluoromethyl, carboxy, C₁₋₄-alkylamino, C₂₋₅-alkanoylamino, C₂₋₅-alkoxycarbonyl groups or two R¹ substituents on the same nitrogen atom may together form a straight- or branched-chain alkylene radical containing from 4 to 6 carbon atoms in the chain which is unsubstituted or substituted as defined for alkyl groups represented by R¹, and pharmaceutically acceptable salts thereof, which process comprises
(A) when X represents the carbonyl group or a group of formula 〉C=NOR¹ or 〉C=NN(R¹)₂ wherein R¹, Y, R and Het are as hereinbefore defined the reaction of a compound of the general formula: 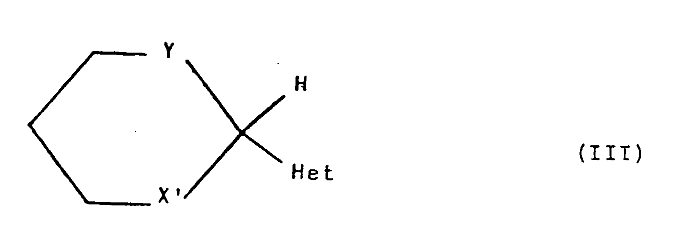 wherein X' represents the carbonyl group or a group of formula 〉C=NOR¹ or 〉C=NN(R¹)₂ as hereinbefore defined and Y and Het are as hereinbefore defined with an isothiocyanate of the general formula:
R-N=C=S (IV)
wherein R represents a straight- or branched-chain alkyl radical containing 1 to 4 carbon atoms;
(B) when X represents the carbonyl group the stereoselective reaction of a mixture of enantiomers of general formula (III) wherein Y and Het are as hereinbefore defined and X' represents the carbonyl group with a chiral auxiliary agent and reaction of the product obtained with a compound of the general formula:
R-N=C=S (IV)
wherein R is as hereinbefore defined followed by removal of the chiral auxiliary agent;
(C) when X represents the hydroxymethylene group and Y, R and Het are as hereinbefore defined the reduction of a compound of general formula (I) wherein X represents the carbonyl group and Y, R and Het are as hereinbefore defined;
(D) when X represents a group of the formula 〉C=NOR¹, 〉C=NN(R¹)₂ or 〉C=NN(R¹)CON(R¹)₂ as hereinbefore defined and Y, R and Het are as hereinbefore defined the reaction of a compound of general formula (I) wherein X represents the carbonyl group with a compound of general formula:
NH₂OR¹ (Va)
NH₂N(R¹)₂ (Vb)
or
NH₂N(R¹)CON(R¹)₂ (Vc)
wherein R¹ is as hereinbefore defined or with an acid addition salt thereof;
(E) when R represents the methyl radical and X represents the carbonyl group or a group of the formula 〉C=NOR¹ or 〉C=NN(R¹)₂ as hereinbefore defined, and Y and Het are as hereinbefore defined the reaction of methylamine with a dithioester of the general formula: 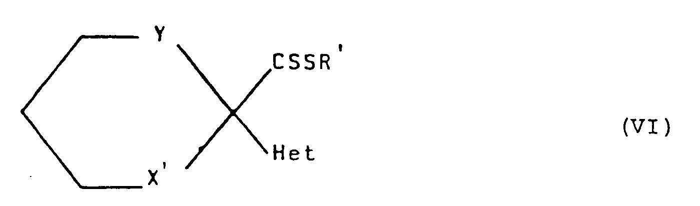 wherein R' represents a straight- or branched-chain alkyl radical containing 1 to 4 carbon atoms, or a benzyl or carboxymethyl radical, and X', Y and Het are as hereinbefore defined;
optionally followed by the step of converting a thioformamide derivative thus obtained into a pharmaceutically acceptable salt thereof.

2. A process according to claim 1 wherein Y represents a methylene radical or a valency bond.

3. A process according to claim 1 or 2 wherein X represents the carbonyl group or a group of the formula 〉C=NOR¹ wherein R¹ is as defined in claim 1.

4. A process according to claim 1, 2 or 3 wherein R¹ represents a straight- or branched-chain alkyl radical containing from 1 to 4 carbon atoms which is unsubstituted or substituted as defined in claim 1, or represents a benzyl, phenethyl, 1-naphthylmethyl, 2-naphthylmethyl or pyrid-3-yl-methyl radical each of which may be substituted on the ring as defined in claim 1.

5. A process according to any one of the preceding claims wherein Het represents 3-pyridyl, 6-chloropyrid-3-yl, 5-bromopyrid-3-yl, 3-quinolinyl, 4-isoquinolinyl or 5-pyrimidyl.

6. A process according to any one of claims 1, 2, 4 and 5 for the preparation of a compound of the general formula: 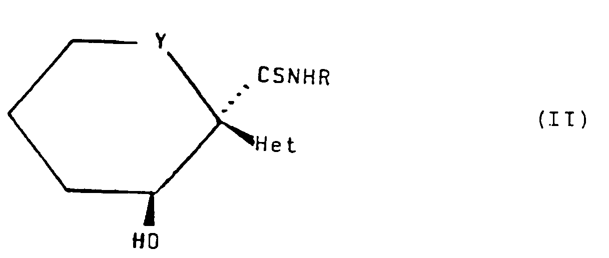 wherein R, Het and Y are as defined in any one of claims 1 to 5 in which the hydroxy group is in the trans position relative to the group -CSNHR.

7. A process according to claim 1 for the preparation of a thioformamide derivative of general formula (I) which is
(±)-N-methyl-2-oxo-1-(3-pyridyl)cyclohexanecarbothioamide
(±)-N-methyl-2-oxo-1-(3-quinolinyl)cyclohexanecarbothioamide
(±)-N-methyl-2-oxo-1-(3-pyridyl)cycloheptanecarbothioamide
(±)-trans-N-methyl-2-hydroxy-1-(3-pyridyl)cyclohexanecarbothioamide
(±)-cis-N-methyl-2-hydroxy-1-(3-pyridyl)cyclohexanecarbothioamide
(±)-anti-N-methyl-2-hydroximino-1-(3-pyridyl)cyclohexanecarbothioamide
(±)-anti-N-methyl-2-methoxyimino-1-(3-pyridyl)cyclohexanecarbothioamide
(±)-anti-N-methyl-2-benzyloxyimino-1-(3-pyridyl)cyclohexanecarbothioamide
(±)-anti-N-methyl-2-hydroxyimino-1-(3-quinolinyl)cyclohexanecarbothioamide
(2S)-anti-2-(methoxymethyl)-1-[2'-(3-quinolinyl)-2'-methylthiocarbamoylcyclohexylideneamino]pyrrolidine
(2S)-anti-2-(methoxymethyl)-1-[2'-(3-pyridyl)-2'-methylthiocarbamoylcyclohexylideneamino]pyrrolidine,
(±)-anti-N-methyl-2-(2-dimethylaminoethoxyimino)-1-(3-pyridyl)cyclohexanecarbothioamide,
(±)-anti-N-methyl-2-(2-aminoethoxyimino)-1-(3-pyridyl) cyclohexanecarbothioamide
(±)-anti-N-methyl-2-methoxycarbonylmethoxyimino-1-(3-pyridyl)cyclohexanecarbothioamide
(±)-anti-N-methyl-2-carbamoylmethoxyimino-1-(3-pyridyl)cyclohexanecarbothioamide,
(±)-anti-N-methyl-2-(2,3-dihydroxypropoxyimino)-1-(3-pyridyl)cyclohexanecarbothioamide
(±)-anti-N-methyl-2-(2-hydroxyethoxyimino)-1-(3-pyridyl)cyclohexanecarbothioamide
(±)-N-methyl-2-oxo-1-(6-chloropyrid-3-yl)cyclohexanecarbothioamide
(±)-N-methyl-2-oxo-1-(5-bromopyrid-3-yl)cyclohexanecarbothioamide
(±)-N-ethyl-2-oxo-1-(3-pyridyl)cyclohexanecarbothioamide
(±)-N-ethyl-2-oxo-1-(3-quinolinyl)cyclohexanecarbothioamide
(±)-anti-N-methyl-2-dimethylhydrazono-1-(3-quinolinyl)cyclohexanecarbothioamide
(±)-anti-N-methyl-2-diethylhydrazono-1-(3-quinolinyl) cyclohexanecarbothioamide
(±)-anti-N-methyl-2-benzyloxyimino-1-(3-quinolinyl)cyclohexanecarbothioamide
(±)-anti-N-methyl-2-methoxyimino-1-(3-quinolinyl)cyclohexanecarbothioamide
(±)-anti-N-methyl-2-ethoxyimino-1-(3-quinolinyl)cyclohexanecarbothioamide
(±)-anti-N-methyl-2-butoxyimino-1-(3-quinolinyl)cyclohexanecarbothioamide
(±)-anti-N-methyl-2-(3-isopropylamino-2-hydroxypropoxyimino)-1-(3-quinolinyl)cyclohexanecarbothioamide
(±)-anti-N-methyl-2-t.butoxyimino-1-(3-quinolinyl)cyclohexanecarbothioamide
(±)-anti-N-methyl-2-prop-2-enoxyimino-1-(3-quinolinyl)cyclohexanecarbothioamide
(±)-anti-N-methyl-2-naphth-2-ylmethoxyimino-1-(3-quinolinyl)cyclohexanecarbothioamide
(±)-anti-N-methyl-2-phenethyloxyimino-1-(3-quinolinyl)cyclohexanecarbothioamide
(±)-anti-N-methyl-2-naphth-1-ylmethoxyimino-1-(3-quinolinyl)cyclohexanecarbothioamide
(±)-anti-N-methyl-2-(3-t.butylamino-2-hydroxypropoxyimino-1-(3-quinolinyl)cyclohexanecarbothioamide
(±)-anti-N-methyl-2-isopropoxyimino-1-(3-quinolinyl)cyclohexanecarbothioamide
(±)-anti-N-methyl-2-(4-hydroxybenzyloxyimino-1-(3-quinolinyl)cyclohexanecarbothioamide
(±)-anti-N-methyl-2-(4-fluorobenzyloxyimino)-1-(3-quinolinyl)cyclohexanecarbothioamide
(±)-anti-N-methyl-2-(4-fluorobenzyloxyimino)-1-(3-pyridyl)cyclohexanecarbothioamide
(±)-anti-N-methyl-2-(2,3,4,5,6-pentafluorobenzyloxyimino)-1-(3-pyridyl)cyclohexanecarbothioamide
(±)-anti-N-methyl-2-isopropoxyimino-1-(3-pyridyl)cyclohexanecarbothioamide
(±)-anti-N-methyl-2-t.butoxyimino-1-(3-pyridyl)cyclohexanecarbothioamide
(±)-anti-N-methyl-2-[4-(3-t.butylamino-2-hydroxypropoxy)benzyloxyimino]-1-(3-pyridyl)cyclohexanecarbothioamide
(±)-anti-N-methyl-2-(4-hydroxybenzyloxyimino)-1-(3-pyridyl)cyclohexanecarbothioamide
(±)-anti-N-methyl-2-(3-fluorobenzyloxyimino)-1-(3-pyridyl)cyclohexanecarbothioamide
(±)-anti-N-methyl-2-(2-fluorobenzyloxyimino)-1-(3-pyridyl)cyclohexanecarbothioamide
(±)-anti-N-methyl-2-(3-pyridylmethoxyimino)-1-(3-pyridyl)cyclohexanecarbothioamide
(±)-anti-N-methyl-2-(4-nitrobenzyloxyimino)-1-(3-pyridyl)cyclohexanecarbothioamide,
(±)-anti-N-methyl-2-(4-cyanobenzyloxyimino)-1-(3-pyridyl)cyclohexanecarbothioamide
(±)-anti-N-methyl-2-(3,4-difluorobenzyloxyimino)-1-(3-pyridyl)cyclohexanecarbothioamide
(±)-anti-N-methyl-2-(4-methoxybenzyloxyimino)-1-(3-pyridyl)cyclohexanecarbothioamide
(±)-anti-N-methyl-2-(2-trifluoromethylbenzyloxyimino)-1-(3-pyridyl)cyclohexanecarbothioamide
(±)-N-methyl-2-oxo-1-(4-isoquinolinyl)cyclohexanecarbothioamide
(±)-anti-N-methyl-2-benzyloxyimino-1-(4-isoquinolinyl)-cyclohexanecarbothioamide
(±)-N-methyl-2-oxo-1-(5-pyrimidyl)cyclohexanecarbothioamide
(±)-N-methyl-2-oxo-1-(3-pyridyl)cyclopentanecarbothioamide
(±)-anti-N-methyl-2-(4-fluorobenzyloxyimino)-1-(3-pyridyl)cyclopentanecarbothioamide
(±)-anti-N-methyl-2-methoxyimino-1-(3-pyridyl)cyclopentanecarbothioamide or
(±)-anti-N-methyl-2-benzyloxyimino-1-(3-pyridyl)cyclopentanecarbothioamide
or a pharmaceutically acceptable salt thereof.

8. A process according to any one of the preceding claims followed by the step of formulating a thioformamide derivative of general formula (I) as defined in claim 1 or a pharmaceutically acceptable salt thereof with a pharmaceutically acceptable carrier or coating.

## Claims (Claims for the following Contracting State(s): GR)

1. A process for the preparation of a thioformamide derivative of the general formula: 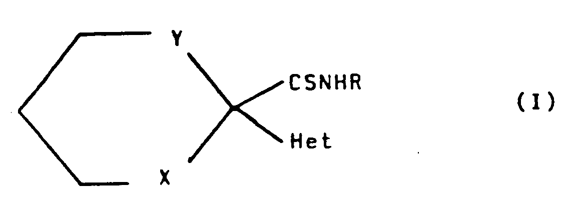 wherein R represents a straight- or branched-chain alkyl radical containing from 1 to 4 carbon atoms, Het represents an aromatic heterocyclic radical containing one or two nitrogen atoms (optionally substituted by a straight- or branched-chain alkyl or alkoxy radical containing 1 to 4 carbon atoms or by a halogen atom), selected from pyrid-3-yl, isoquinolin-4-yl, tetrahydroquinolin-3-yl, quinolin-3-yl, pyridazin-4-yl, pyrimid-5-yl, thiazol-5-yl, thieno[2,3-b]pyridin-5-yl, pyrazin-2-yl, indol-3-yl and thieno[3,2-b]pyridin-6-yl, Y represents an ethylene or methylene radical, or a valency bond, and X represents a carbonyl or hydroxymethylene group or a group of the formula: 〉C=NOR¹, 〉C=NN(R¹)₂ or 〉C=NN(R¹)CON(R¹)₂ in which the symbols R¹, which may be the same or different, each represents the hydrogen atom or a straight- or branched-chain alkyl radical containing from 1 to 4 carbon atoms which is unsubstituted or substituted by one or more substituents selected from C₂₋₄-alkenyl, carboxy, C₂₋₅-alkoxycarbonyl, hydroxy, C₁₋₄-alkoxy, carbamoyl (unsubstituted or substituted by one or two C₁₋₄-alkyl groups), amino, C₁₋₄ alkylamino and di-C₁₋₄-alkylamino groups or represents a benzyl, phenethyl, 1-naphthylmethyl, 2-naphthylmethyl or pyrid-3-ylmethyl radical each of which may be substituted on the ring by one or more halogen atoms or hydroxy, C₁₋₄-alkyl, C₁₋₄-alkoxy (alkoxy being unsubstituted or substituted as defined for alkyl groups represented by R¹) cyano, nitro, trifluoromethyl, carboxy, C₁₋₄-alkylamino, C₂₋₅-alkanoylamino, C₂₋₅-alkoxycarbonyl groups or two R¹ substituents on the same nitrogen atom may together form a straight- or branched-chain alkylene radical containing from 4 to 6 carbon atoms in the chain which is unsubstituted or substituted as defined for alkyl groups represented by R¹, and pharmaceutically acceptable salts thereof, which process comprises
(A) when X represents the carbonyl group or a group of formula 〉C=NOR¹ or 〉C=NN(R¹)₂ wherein R¹, Y, R and Het are as hereinbefore defined the reaction of a compound of the general formula: 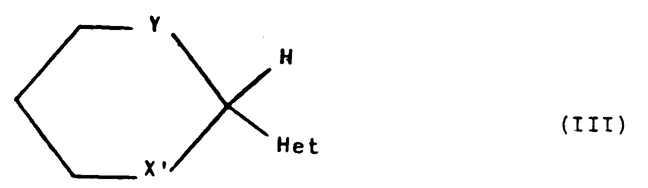 wherein X' represents the carbonyl group or a group of formula 〉C=NOR¹ or 〉C=NN(R¹)₂ as hereinbefore defined and Y and Het are as hereinbefore defined with an isothiocyanate of the general formula:
R-N=C=S (IV)
wherein R represents a straight- or branched-chain alkyl radical containing 1 to 4 carbon atoms;
(B) when X represents the carbonyl group the stereoselective reaction of a mixture of enantiomers of general formula (III) wherein Y and Het are as hereinbefore defined and X' represents the carbonyl group with a chiral auxiliary agent and reaction of the product obtained with a compound of the general formula:
R-N=C=S (IV)
wherein R is as hereinbefore defined followed by removal of the chiral auxiliary agent;
(C) when X represents the hydroxymethylene group and Y, R and Het are as hereinbefore defined the reduction of a compound of general formula (I) wherein X represents the carbonyl group and Y, R and Het are as hereinbefore defined;
(D) when X represents a group of the formula 〉C=NOR¹, 〉C=NN(R¹)₂ or 〉C=NN(R¹)CON(R¹)₂ as hereinbefore defined and Y, R and Het are as hereinbefore defined the reaction of a compound of general formula (I) wherein X represents the carbonyl group with a compound of general formula:
NH₂OR¹ (Va)
NH₂N(R¹)₂ (Vb)
or
NH₂N(R¹)CON(R¹)₂ (Vc)
wherein R¹ is as hereinbefore defined or with an acid addition salt thereof;
(E) when R represents the methyl radical and X represents the carbonyl group or a group of the formula 〉C=NOR¹ or 〉C=NN(R¹)₂ as hereinbefore defined, and Y and Het are as hereinbefore defined the reaction of methylamine with a dithioester of the general formula: 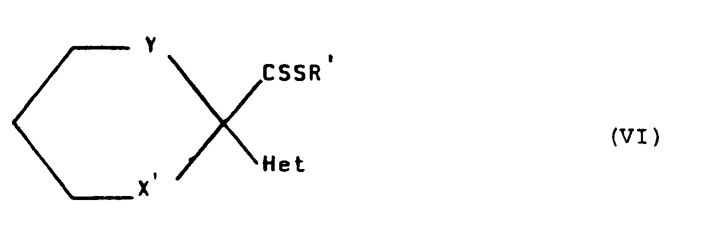 wherein R' represents a straight- or branched-chain alkyl radical containing 1 to 4 carbon atoms, or a benzyl or carboxymethyl radical, and X', Y and Het are as hereinbefore defined;
optionally followed by the step of converting a thioformamide derivative thus obtained into a pharmaceutically acceptable salt thereof.

2. A process according to claim 1 wherein Y represents a methylene radical or a valency bond.

3. A process according to claim 1 or 2 wherein X represents the carbonyl group or a group of the formula 〉C=NOR¹ wherein R¹ is as defined in claim 1.

4. A process according to claim 1, 2 or 3 wherein R¹ represents a straight- or branched-chain alkyl radical containing from 1 to 4 carbon atoms which is unsubstituted or substituted as defined in claim 1, or represents a benzyl, phenethyl, 1-naphthylmethyl, 2-naphthylmethyl or pyrid-3-yl-methyl radical each of which may be substituted on the ring as defined in claim 1.

5. A process according to any one of the preceding claims wherein Het represents 3-pyridyl, 6-chloropyrid-3-yl, 5-bromopyrid-3-yl, 3-quinolinyl, 4-isoquinolinyl or 5-pyrimidyl.

6. A process according to any one of claims 1, 2, 4 and 5 for the preparation of a compound of the general formula: 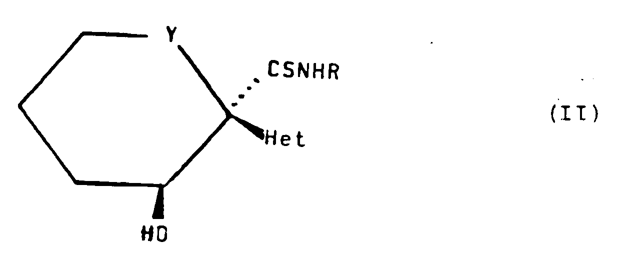 wherein R, Het and Y are as defined in any one of claims 1 to 5 in which the hydroxy group is in the trans position relative to the group -CSNHR.

7. A process according to claim 1 for the preparation of a thioformamide derivative of general formula (I) which is
(±)-N-methyl-2-oxo-1-(3-pyridyl)cyclohexanecarbothioamide
(±)-N-methyl-2-oxo-1-(3-quinolinyl)cyclohexanecarbothioamide
(±)-N-methyl-2-oxo-1-(3-pyridyl)cycloheptanecarbothioamide
(±)-trans-N-methyl-2-hydroxy-1-(3-pyridyl)cyclohexanecarbothioamide
(±)-cis-N-methyl-2-hydroxy-1-(3-pyridyl)cyclohexanecarbothioamide
(±)-anti-N-methyl-2-hydroximino-1-(3-pyridyl)cyclohexanecarbothioamide
(±)-anti-N-methyl-2-methoxyimino-1-(3-pyridyl)cyclohexanecarbothioamide
(±)-anti-N-methyl-2-benzyloxyimino-1-(3-pyridyl)cyclohexanecarbothioamide
(±)-anti-N-methyl-2-hydroxyimino-1-(3-quinolinyl)cyclohexanecarbothioamide
(2S)-anti-2-(methoxymethyl)-1-[2'-(3-quinolinyl)-2'-methylthiocarbamoylcyclohexylideneamino]pyrrolidine
(2S)-anti-2-(methoxymethyl)-1-[2'-(3-pyridyl)-2'-methylthiocarbamoylcyclohexylideneamino]pyrrolidine,
(±)-anti-N-methyl-2-(2-dimethylaminoethoxyimino)-1-(3-pyridyl)cyclohexanecarbothioamide,
(±)-anti-N-methyl-2-(2-aminoethoxyimino)-1-(3-pyridyl) cyclohexanecarbothioamide
(±)-anti-N-methyl-2-methoxycarbonylmethoxyimino-1-(3-pyridyl)cyclohexanecarbothioamide
(±)-anti-N-methyl-2-carbamoylmethoxyimino-1-(3-pyridyl)cyclohexanecarbothioamide,
(±)-anti-N-methyl-2-(2,3-dihydroxypropoxyimino)-1-(3-pyridyl)cyclohexanecarbothioamide
(±)-anti-N-methyl-2-(2-hydroxyethoxyimino)-1-(3-pyridyl)cyclohexanecarbothioamide
(±)-N-methyl-2-oxo-1-(6-chloropyrid-3-yl)cyclohexanecarbothioamide
(±)-N-methyl-2-oxo-1-(5-bromopyrid-3-yl)cyclohexanecarbothioamide
(±)-N-ethyl-2-oxo-1-(3-pyridyl)cyclohexanecarbothioamide
(±)-N-ethyl-2-oxo-1-(3-quinolinyl)cyclohexanecarbothioamide
(±)-anti-N-methyl-2-dimethylhydrazono-1-(3-quinolinyl)cyclohexanecarbothioamide
(±)-anti-N-methyl-2-diethylhydrazono-1-(3-quinolinyl) cyclohexanecarbothioamide
(±)-anti-N-methyl-2-benzyloxyimino-1-(3-quinolinyl)cyclohexanecarbothioamide
(±)-anti-N-methyl-2-methoxyimino-1-(3-quinolinyl)cyclohexanecarbothioamide
(±)-anti-N-methyl-2-ethoxyimino-1-(3-quinolinyl)cyclohexanecarbothioamide
(±)-anti-N-methyl-2-butoxyimino-1-(3-quinolinyl)cyclohexanecarbothioamide
(±)-anti-N-methyl-2-(3-isopropylamino-2-hydroxypropoxyimino)-1-(3-quinolinyl)cyclohexanecarbothioamide
(±)-anti-N-methyl-2-t.butoxyimino-1-(3-quinolinyl)cyclohexanecarbothioamide
(±)-anti-N-methyl-2-prop-2-enoxyimino-1-(3-quinolinyl)cyclohexanecarbothioamide
(±)-anti-N-methyl-2-naphth-2-ylmethoxyimino-1-(3-quinolinyl)cyclohexanecarbothioamide
(±)-anti-N-methyl-2-phenethyloxyimino-1-(3-quinolinyl)cyclohexanecarbothioamide
(±)-anti-N-methyl-2-naphth-1-ylmethoxyimino-1-(3-quinolinyl)cyclohexanecarbothioamide
(±)-anti-N-methyl-2-(3-t.butylamino-2-hydroxypropoxyimino-1-(3-quinolinyl)cyclohexanecarbothioamide
(±)-anti-N-methyl-2-isopropoxyimino-1-(3-quinolinyl)cyclohexanecarbothioamide
(±)-anti-N-methyl-2-(4-hydroxybenzyloxyimino-1-(3-quinolinyl)cyclohexanecarbothioamide
(±)-anti-N-methyl-2-(4-fluorobenzyloxyimino)-1-(3-quinolinyl)cyclohexanecarbothioamide
(±)-anti-N-methyl-2-(4-fluorobenzyloxyimino)-1-(3-pyridyl)cyclohexanecarbothioamide
(±)-anti-N-methyl-2-(2,3,4,5,6-pentafluorobenzyloxyimino)-1-(3-pyridyl)cyclohexanecarbothioamide
(±)-anti-N-methyl-2-isopropoxyimino-1-(3-pyridyl)cyclohexanecarbothioamide
(±)-anti-N-methyl-2-t.butoxyimino-1-(3-pyridyl)cyclohexanecarbothioamide
(±)-anti-N-methyl-2-[4-(3-t.butylamino-2-hydroxypropoxy)benzyloxyimino]-1-(3-pyridyl)cyclohexanecarbothioamide
(±)-anti-N-methyl-2-(4-hydroxybenzyloxyimino)-1-(3-pyridyl)cyclohexanecarbothioamide
(±)-anti-N-methyl-2-(3-fluorobenzyloxyimino)-1-(3-pyridyl)cyclohexanecarbothioamide
(±)-anti-N-methyl-2-(2-fluorobenzyloxyimino)-1-(3-pyridyl)cyclohexanecarbothioamide
(±)-anti-N-methyl-2-(3-pyridylmethoxyimino)-1-(3-pyridyl)cyclohexanecarbothioamide
(±)-anti-N-methyl-2-(4-nitrobenzyloxyimino)-1-(3-pyridyl)cyclohexanecarbothioamide,
(±)-anti-N-methyl-2-(4-cyanobenzyloxyimino)-1-(3-pyridyl)cyclohexanecarbothioamide
(±)-anti-N-methyl-2-(3,4-difluorobenzyloxyimino)-1-(3-pyridyl)cyclohexanecarbothioamide
(±)-anti-N-methyl-2-(4-methoxybenzyloxyimino)-1-(3-pyridyl)cyclohexanecarbothioamide
(±)-anti-N-methyl-2-(2-trifluoromethylbenzyloxyimino)-1-(3-pyridyl)cyclohexanecarbothioamide
(±)-N-methyl-2-oxo-1-(4-isoquinolinyl)cyclohexanecarbothioamide
(±)-anti-N-methyl-2-benzyloxyimino-1-(4-isoquinolinyl)-cyclohexanecarbothioamide
(±)-N-methyl-2-oxo-1-(5-pyrimidyl)cyclohexanecarbothioamide
(±)-N-methyl-2-oxo-1-(3-pyridyl)cyclopentanecarbothioamide
(±)-anti-N-methyl-2-(4-fluorobenzyloxyimino)-1-(3-pyridyl)cyclopentanecarbothioamide
(±)-anti-N-methyl-2-methoxyimino-1-(3-pyridyl)cyclopentanecarbothioamide or
(±)-anti-N-methyl-2-benzyloxyimino-1-(3-pyridyl)cyclopentanecarbothioamide
or a pharmaceutically acceptable salt thereof.

8. A process according to any one of the preceding claims followed by the step of formulating a thioformamide derivative of general formula (I) as defined in claim 1 or a pharmaceutically acceptable salt thereof with a pharmaceutically acceptable carrier or coating.

9. A compound of general formula (III) depicted in claim 1 wherein X', Y and Het are as defined in claim 1 with the proviso that Y is not a direct bond when Het represents an optionally substituted pyrazin-2-yl group and Y does not represent methylene when Het represents an optionally substituted indol-3-yl group.

## Patentansprüche (Patentansprüche für folgende(n) Vertragsstaat(en): AT, BE, CH, DE, FR, GB, IT, LI, LU, NL, SE)

1. Thioformamidderivat der allgemeinen Formel: 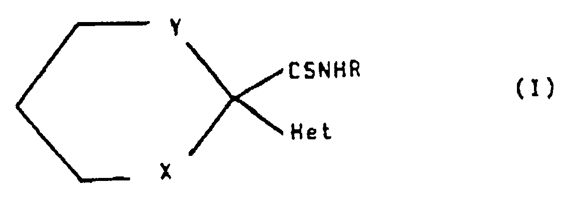 worin bedeuten:
R einen gerad- oder verzweigtkettigen Alkylrest mit 1 bis 4 Kohlenstoffatom(en),
Het einen aromatischen heterocyclischen Rest mit einem oder zwei Stickstoffatom(en) (gegebenenfalls substituiert durch einen gerad- oder verzweigtkettigen Alkyl- oder Alkoxyrest mit 1 bis 4 Kohlenstoffatom(en) oder ein Halogenatom), ausgewählt aus Pyrid-3-yl, Isochinolin-4-yl, Tetrahydrochinolin-3-yl, Chinolin-3-yl, Pyridazin-4-yl, Pyrimid-5-yl, Thiazol-5-yl, Thieno[2,3-b]pyridin-5-yl, Pyrazin-2-yl, Indol-3-yl und Thieno[3,2-b]pyridin-6-yl,
Y einen Ethylen- oder Methylenrest oder eine Valenzbindung und
X eine Carbonyl- oder Hydroxymethylengruppe oder eine Gruppe der Formel: 〉C=NOR¹, 〉C=NN(R¹)₂ oder 〉C=NN(R¹)CON(R¹)₂, worin die Symbole R¹, die gleich oder verschieden sein können, jeweils für ein Wasserstoffatom oder einen gerad- oder verzweigtkettigen Alkylrest mit 1 bis 4 Kohlenstoffatom(en), der nicht substituiert ist oder durch einen oder mehrere Substituenten, ausgewählt aus C₂₋₄-Alkenyl-, Carboxy-, C₂₋₅-Alkoxycarbonyl-, Hydroxy-, C₁₋₄-Alkoxy-, Carbamoyl-(unsubstituiert oder substituiert durch eine oder zwei C₁₋₄-Alkylgruppe(n)), Amino-, C₁₋₄-Alkylamino- und di-C₁₋₄-Alkylaminogruppen, substituiert ist, oder für einen Benzyl-, Phenethyl-, 1-Naphthylmethyl-, 2-Naphthylmethyl- oder Pyrid-3-ylmethylrest, jeweils gegebenenfalls substituiert am Ring durch ein oder mehrere Halogenatom(e) oder Hydroxy-, C₁₋₄-Alkyl-, C₁₋₄-Alkoxy (wobei Alkoxy gegebenenfalls durch die für die durch R¹ dargestellten Alkylgruppen definierten Substituenten substituiert sein kann), Cyano-, Nitro-, Trifluormethyl-, Carboxy-, C₁₋₄-Alkylamino-, C₂₋₅-Alkanoylamino-, C₂₋₅-Alkoxycarbonylgruppe(n), stehen oder zwei R¹-Substituenten am selben Stickstoffatom zusammen einen gerad- oder verzweigtkettigen Alkylenrest mit 4 bis 6 Kohlenstoffatomen in der Kette, die gegebenenfalls durch die für die durch R¹ dargestellten Alkylgruppen definierten Substituenten substituiert ist, bilden können,
und pharmazeutisch akzeptable Salze davon.

2. Verbindung nach Anspruch 1, worin Y für einen Methylenrest oder eine Valenzbindung steht.

3. Verbindung nach Anspruch 1 oder 2, worin X für die Carbonylgruppe oder eine Gruppe der Formel 〉C=NOR¹, worin R¹ die in Anspruch 1 angegebene Bedeutung besitzt, steht.

4. Verbindung nach Anspruch 1, 2 oder 3, worin R¹ für einen gerad- oder verzweigtkettigen Alkylrest mit 1 bis 4 Kohlenstoffatom(en), der gegebenenfalls entsprechend der Definition in Anspruch 1 substituiert ist, oder einen Benzyl-, Phenethyl-, 1-Naphthylmethyl-, 2-Napthylmethyl- oder Pyrid-3-ylmethylrest, jeweils gegebenenfalls entsprechend der Definition in Anspruch 1 am Ring substituiert, steht.

5. Verbindung nach einem der vorhergehenden Ansprüche, worin Het für 3-Pyridyl, 6-Chlorpyrid-3-yl, 5-Brompyrid-3-yl, 3-Chinolinyl, 4-Isochinolinyl oder 5-Pyrimidyl steht.

6. Verbindung nach einem der Ansprüche 1, 2, 4 und 5 der allgemeinen Formel 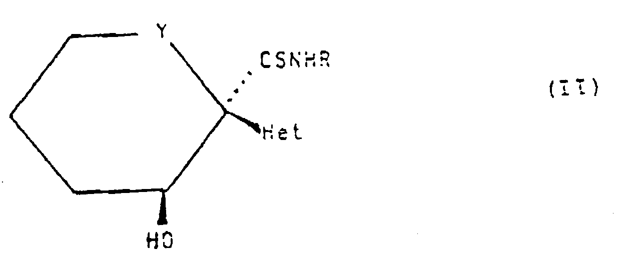 worin R, Het und Y die in einem der Ansprüche 1 bis 5 angegebene Bedeutung besitzen, bei der sich die Hydroxygruppe in trans-Stellung relativ zu der -CSNHR-Gruppe befindet.

7. Verbindung nach Anspruch 1, nämlich:
(±)-N-Methyl-2-oxo-1-(3-pyridyl)cyclohexancarbothioamid
(±)-N-Methyl-2-oxo-1-(3-chinolinyl)-cyclohexancarbothioamid
(±)-N-Methyl-2-oxo-1-(3-pyridyl)cycloheptancarbothioamid
(±)-trans-N-Methyl-2-hydroxy-1-(3-pyridyl)cyclohexancarbothioamid
(±)-cis-N-Methyl-2-hydroxy-1-(3-pyridyl)cyclohexancarbothioamid
(±)-anti-N-Methyl-2-hydroximino-1-(3-pyridyl)cyclohexancarbothioamid
(±)-anti-N-Methyl-2-methoxyimino-1-(3-pyridyl)cyclohexancarbothioamid
(±)-anti-N-Methyl-2-benzyloxyimino-1-(3-pyridyl)-cyclohexancarbothioamid
(±)-anti-N-Methyl-2-hydroxyimino-1-(3-chinolinyl)cyclohexancarbothioamid
(2S)-anti-2-(methoxymethyl)-1-[2'-(3-chinolinyl)-2'-methylthiocarbamoylcyclohexylidenamino]pyrrolidin
(2S)-anti-2-(Methoxymethyl)-1-[2'-(3-pyridyl)-2'-methylthiocarbamoylcyclohexylidenamino]pyrrolidin
(±)-anti-N-Methyl-2-(2-dimethylaminoethoxyimino)-1-(3-pyridyl)cyclohexancarbothioamid
(±)-anti-N-Methyl-2-(2-aminoethoxyimino)-1-(3-pyridyl)cyclohexancarbothioamid
(±)-anti-N-Methyl-2-methoxycarbonylmethoxyimino-1-(3-pyridyl)cyclohexancarbothioamid
(±)-anti-N-Methyl-2-carbamoylmethoxyimino-1-(3-pyridyl)cyclohexancarbothioamid
(±)-anti-N-Methyl-2-(2,3-dihydroxypropoxyimino)-1-(3-pyridyl)cyclohexancarbothioamid
(±)-anti-N-Methyl-2-(2-hydroxyethoxyimino)-1-(3-pyridyl)cyclohexancarbothioamid
(±)-N-Methyl-2-oxo-1-(6-chloropyrid-3-yl)-cyclohexancarbothioamid
(±)-N-Methyl-2-oxo-1-(5-bromopyrid-3-yl)-cyclohexancarbothioamid
(±)-N-Ethyl-2-oxo-1-(3-pyridyl)cyclohexancarbothioamid
(±)-N-Ethyl-2-oxo-1-(3-chinolinyl)-cyclohexancarbothioamid
(±)-anti-N-Methyl-2-dimethylhydrazono-1-(3-chinolinyl)-cyclohexancarbothioamid
(±)-anti-N-Methyl-2-diethylhydrazono-1-(3-chinolinyl)-cyclohexancarbothioamid
(±)-anti-N-Methyl-2-benzyloxyimino-1-(3-chinolinyl)-cyclohexancarbothioamid
(±)-anti-N-Methyl-2-methoxyimino-1-(3-chinolinyl)-cyclohexancarbothioamid
(±)-anti-N-Methyl-2-ethoxyimino-1-(3-chinolinyl)-cyclohexancarbothioamid
(±)-anti-N-Methyl-2-butoxyimino-1-(3-chinolinyl)-cyclohexancarbothioamid
(±)-anti-N-Methyl-2-(3-isopropylamino-2-hydroxy-propoxyimino)-1-(3-chinolinyl)cyclohexancarbothioamid
(±)-anti-N-Methyl-2-tert.-butoxyimino-1-(3-chinolinyl)cyclohexancarbothioamid
(±)-anti-N-Methyl-2-prop-2-enoxyimino-1-(3-chinolinyl)cyclohexancarbothioamid
(±)-anti-N-Methyl-2-naphth-2-ylmethoxyimino-1-(3-chinolinyl)cyclohexancarbothioamid
(±)-anti-N-Methyl-2-phenethyloxyimino-1-(3-chinolinyl)cyclohexancarbothioamid
(±)-anti-N-Methyl-2-naphth-1-ylmethoxyimino-1-(3-chinolinyl)cyclohexancarbothioamid
(±)-anti-N-Methyl-2-(3-tert.-butylamino-2-hydroxypropoxyimino-1-(3-chinolinyl)cyclohexancarbothioamid
(±)-anti-N-Methyl-2-isopropoxyimino-1-(3-chinolinyl)-cyclohexancarbothioamid
(±)-anti-N-Methyl-2-(4-hydroxybenzyloxyimino-1-(3-chinolinyl)cyclohexancarbothioamid
(±)-anti-N-Methyl-2-(4-fluorbenzyloxyimino)-1-(3-chinolinyl)cyclohexancarbothioamid
(±)-anti-N-Methyl-2-(4-fluorbenzyloxyimino-1-(3-pyridyl)cyclohexancarbothioamid
(±)-anti-N-Methyl-2-(2,3,4,5,6-pentafluorbenzyloxyimino)-1-(3-pyridyl)cyclohexancarbothioamid
(±)-anti-N-Methyl-2-isopropoxyimino-1-(3-pyridyl)-cyclohexancarbothioamid
(±)-anti-N-Methyl-2-tert.-butoxyimino-1-(3-pyridyl)-cyclohexancarbothioamid
(±)-anti-N-Methyl-2-[4-(3-tert.-butylamino-2-hydroxypropoxy)benzyloxyimino]-1-(3-pyridyl)cyclohexancarbo thioamid
(±)-anti-N-Methyl-2-(4-hydroxybenzyloxyimino)-1-(3-pyridyl)cyclohexancarbothioamid
(±)-anti-N-Methyl-2-(3-fluorbenzyloxyimino)-1-(3-pyridyl)cyclohexancarbothioamid
(±)-anti-N-Methyl-2-(2-fluorbenzyloxyimino)-1-(3-pyridyl)cyclohexancarbothioamid
(±)-anti-N-Methyl-2-(3-pyridylmethoxyimino)-1-(3-pyridyl)cyclohexancarbothioamid
(±)-anti-N-Methyl-2-(4-nitrobenzyloxyimino)-1-(3-pyridyl)cyclohexancarbothioamid
(±)-anti-N-Methyl-2-(4-cyanobenzyloxyimino)-1-(3-pyridyl)cyclohexancarbothioamid
(±)-anti-N-Methyl-2-(3,4-difluorbenzyloxyimino)-1-(3-pyridyl)cyclohexancarbothioamid
(±)-anti-N-Methyl-2-(4-methoxybenzyloxyimino)-1-(3-pyridyl)cyclohexancarbothioamid
(±)-anti-N-Methyl-2-(2-trifluormethylbenzyloxyimino)-1-(3-pyridyl)cyclohexancarbothioamid
(±)-N-Methyl-2-oxo-1-(4-isochinolinyl)cyclohexancarbothioamid
(±)-anti-N-Methyl-2-benzyloxyimino-1-(4-isochinolinyl)-cyclohexancarbothioamid
(±)-N-Methyl-2-oxo-1-(5-pyrimidyl)cyclohexancarbothioamid
(±)-N-Methyl-2-oxo-1-(3-pyridyl)cyclopentancarbothioamid
(±)-anti-N-Methyl-2-(4-fluorbenzyloxyimino)-1-(3-pyridyl)cyclopentancarbothioamid
(±)-anti-N-Methyl-2-methoxyimino-1-(3-pyridyl)-cyclopentancarbothioamid oder
(±)-anti-N-Methyl-2-benzyloxyimino-1-(3-pyridyl)-cyclopentancarbothioamid
oder ein pharmazeutisch akzeptables Salz davon.

8. Verfahren zur Herstellung eines Thioformamidderivats der allgemeinen Formel (I) entsprechend Anspruch 1, umfassend:
(A) wenn X für die Carbonylgruppe oder eine Gruppe der Formel 〉C=NOR¹ oder 〉C=NN(R¹)₂ mit R¹ in der in Anspruch 1 angegebenen Bedeutung steht und Y, R und Het die in Anspruch 1 angegebene Bedeutung besitzen, die Umsetzung einer Verbindung der allgemeinen Formel: 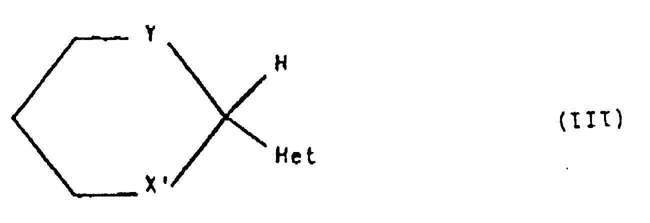 worin X' für die Carbonylgruppe oder eine Gruppe der Formel 〉C=NOR¹ oder 〉C=NN(R¹)₂ entsprechend der Definition in Anspruch 1 steht und Y und Het die in Anspruch 1 angebenene Bedeutung besitzen, mit einem Isothiocyanat der allgemeinen Formel:
R-N=C=S (IV)
worin R für einen gerad- oder verzweigtkettigen Alkylrest mit 1 bis 4 Kohlenstoffatom(en) steht;
(B) wenn X für die Carbonylgruppe steht, die stereoselektive Umsetzung eines Enantiomerengemisches der allgemeinen Formel (III), worin Y und Het die in Anspruch 1 angegebene Bedeutung besitzen und X' für die Carbonylgruppe steht, mit einem chiralen Hilfsstoff und Umsetzung des erhaltenen Produkts mit einer Verbindung der allgemeinen Formel:
R-N=C=S (IV)
worin R die in Anspruch 1 angegebene Bedeutung besitzt, und anschließende Entfernung des chiralen Hilfsstoffs;
(C) wenn X für die Hydroxymethylengruppe steht und Y, R und Het die in Anspruch 1 angegebene Bedeutung besitzen, die Reduktion einer Verbindung der allgemeinen Formel (I), worin X für die Carbonylgruppe steht und Y, R und Het die in Anspruch 1 angegebene Bedeutung besitzen;
(D) wenn X für eine Gruppe der Formel 〉C=NOR¹, 〉C=NN(R¹)₂ oder 〉C=NN(R¹)CON(R¹)₂ entsprechend der Definition in Anspruch 1 steht und Y, R und Het die in Anspruch 1 angegebene Bedeutung besitzen, die Umsetzung einer Verbindung der allgemeinen Formel (I), worin X für die Carbonylgruppe steht, mit einer Verbindung der allgemeinen Formel:
NH₂OR¹ (Va)
NH₂N(R¹)₂ (Vb)
oder
NH₂N(R¹)CON(R¹)₂ (Vc)
worin R¹ die in Anspruch 1 angegebene Bedeutung besitzt, oder mit einem Säureadditionssalz davon;
(E) wenn R für den Methylrest steht und X die Carbonylgruppe oder eine Gruppe der Formel 〉C=NOR¹ oder 〉C=NN(R¹)₂ entsprechend der Definition in Anspruch 1 darstellt und Y und Het die in Anspruch 1 angegebene Bedeutung besitzen, die Umsetzung von Methylamin mit einem Dithioester der allgemeinen Formel: 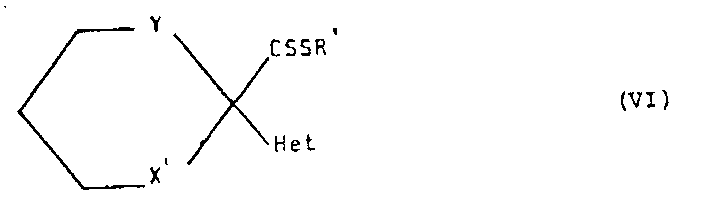 worin R' für einen gerad- oder verzweigtkettigen Alkylrest mit 1 bis 4 Kohlenstoffatom(en) oder einen Benzyl- oder Carboxymethylrest steht, X' die oben angegebene Bedeutung besitzt und Y und Het die in Anspruch 1 angegebene Bedeutung besitzen;
gegebenenfalls gefolgt von der Stufe eines Umwandelns eines so erhaltenen Thioformamidderivats in ein pharmazeutisch akzeptables Salz davon.

9. Pharmazeutische Zubereitung, umfassend ein Thioformamidderivat der allgemeinen Formel 1 entsprechend der Definition in Anspruch 1 oder ein pharmazeutisch akzeptables Salz davon in Verbindung mit einem pharmazeutisch akzeptablen Träger oder Überzug.

10. Verbindung der allgemeinen Formel (III) entsprechend Anspruch 8, worin Y und Het die in Anspruch 1 angegebene Bedeutung besitzen und X' die in Anspruch 8 angegebene Bedeutung besitzt, wobei gilt, daß Y nicht für eine direkte Bindung steht, wenn Het eine gegebenenfalls substituierte Pyrazin-2-yl-Gruppe bedeutet, und Y nicht für Methylen steht, wenn Het eine gegebenenfalls substituierte Indol-3-yl-Gruppe darstellt.

## Patentansprüche (Patentansprüche für folgende(n) Vertragsstaat(en): ES)

1. Verfahren zur Herstellung eines Thioformamidderivats der allgemeinen Formel: 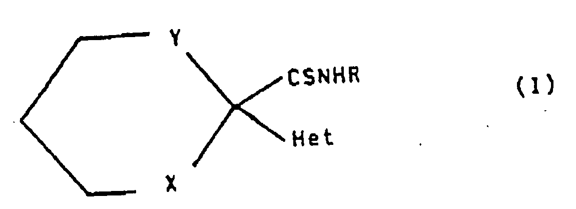 worin bedeuten:
R einen gerad- oder verzweigtkettigen Alkylrest mit 1 bis 4 Kohlenstoffatom(en),
Het einen aromatischen heterocyclischen Rest mit einem oder zwei Stickstoffatom(en) (gegebenenfalls substituiert durch einen gerad- oder verzweigtkettigen Alkyl- oder Alkoxyrest mit 1 bis 4 Kohlenstoffatom(en) oder ein Halogenatom), ausgewählt aus Pyrid-3-yl, Isochinolin-4-yl, Tetrahydrochinolin-3-yl, Chinolin-3-yl, Pyridazin-4-yl, Pyrimid-5-yl, Thiazol-5-yl, Thieno[2,3-b]pyridin-5-yl, Pyrazin-2-yl, Indol-3-yl und Thieno[3,2-b]pyridin-6-yl,
Y einen Ethylen- oder Methylenrest oder eine Valenzbindung und
X eine Carbonyl- oder Hydroxymethylengruppe oder eine Gruppe der Formel: 〉C=NOR¹, 〉C=NN(R¹)₂ oder 〉C=NN(R¹)CON(R¹)₂, worin die Symbole R¹, die gleich oder verschieden sein können, jeweils für ein Wasserstoffatom oder einen gerad- oder verzweigtkettigen Alkylrest mit 1 bis 4 Kohlenstoffatom(en), der nicht substituiert ist oder durch einen oder mehrere Substituenten, ausgewählt aus C₂₋₄-Alkenyl-, Carboxy-, C₂₋₅-Alkoxycarbonyl-, Hydroxy-, C₁₋₄-Alkoxy-, Carbamoyl-(unsubstituiert oder substituiert durch eine oder zwei C₁₋₄-Alkylgruppe(n)), Amino-, C₁₋₄-Alkylamino- und di-C₁₋₄-Alkylaminogruppen, substituiert ist, oder für einen Benzyl-, Phenethyl-, 1-Naphthylmethyl-, 2-Naphthylmethyl- oder Pyrid-3-ylmethylrest, jeweils gegebenenfalls substituiert am Ring durch ein oder mehrere Halogenatom(e) oder Hydroxy-, C₁₋₄-Alkyl-, C₁₋₄-Alkoxy (wobei Alkoxy gegebenenfalls durch die für die durch R¹ dargestellten Alkylgruppen definierten Substituenten substituiert sein kann), Cyano-, Nitro-, Trifluormethyl-, Carboxy-, C₁₋₄-Alkylamino-, C₂₋₅-Alkanoylamino-, C₂₋₅-Alkoxycarbonylgruppe(n), stehen oder zwei R¹-Substituenten am selben Stickstoffatom zusammen einen gerad- oder verzweigtkettigen Alkylenrest mit 4 bis 6 Kohlenstoffatomen in der Kette, die gegebenenfalls durch die für die durch R¹ dargestellten Alkylgruppen definierten Substituenten substituiert ist, bilden können,
sowie pharmazeutisch akzeptabler Salze davon, umfassend:
(A) wenn X für die Carbonylgruppe oder eine Gruppe der Formel 〉C=NOR¹ oder 〉C=NN(R¹)₂ steht, wobei R¹, Y, R und Het die oben angegebene Bedeutung besitzen, die Umsetzung einer Verbindung der allgemeinen Formel: 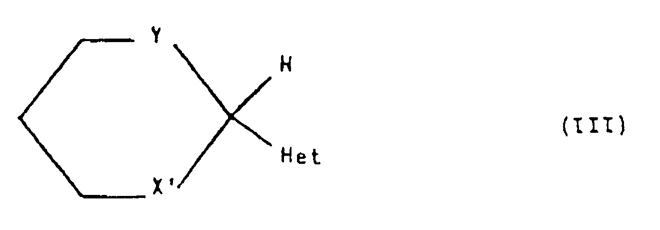 worin X' für die Carbonylgruppe oder eine Gruppe der Formel 〉C=NOR¹ oder 〉C=NN(R¹)₂ entsprechend der oben angegebenen Definition steht und Y und Het die oben angegebene Bedeutung besitzen, mit einem Isothiocyanat der allgemeinen Formel:
R-N=C=S (IV)
worin R für einen gerad- oder verzweigtkettigen Alkylrest mit 1 bis 4 Kohlenstoffatom(en) steht;
(B) wenn X für die Carbonylgruppe steht, die stereoselektive Umsetzung eines Enantiomerengemisches der allgemeinen Formel (III), worin Y und Het die oben angegebene Bedeutung besitzen und X' für die Carbonylgruppe steht, mit einem chiralen Hilfsstoff und Umsetzung des erhaltenen Produkts mit einer Verbindung der allgemeinen Formel:
R-N=C=S (IV)
worin R die oben angegebene Bedeutung besitzt, und anschließende Entfernung des chiralen Hilfsstoffs;
(C) wenn X für die Hydroxymethylengruppe steht und Y, R und Het die oben angegebene Bedeutung besitzen, die Reduktion einer Verbindung der allgemeinen Formel (I), worin X für die Carbonylgruppe steht und Y, R und Het die oben angegebene Bedeutung besitzen;
(D) wenn X für eine Gruppe der Formel 〉C=NOR¹, 〉C=NN(R¹)₂ oder 〉C=NN(R¹)CON(R¹)₂ entsprechend der obigen Definition steht und Y, R und Het die oben angegebene Bedeutung besitzen, die Umsetzung einer Verbindung der allgemeinen Formel (I), worin X für die Carbonylgruppe steht, mit einer Verbindung der allgemeinen Formel:
NH₂OR¹ (Va)
NH₂N(R¹)₂ (Vb)
oder
NH₂N(R¹)CON(R¹)₂ (Vc)
worin R¹ die oben angegebene Bedeutung besitzt, oder mit einem Säureadditionssalz davon;
(E) wenn R für den Methylrest steht und X die Carbonylgruppe oder eine Gruppe der Formel 〉C=NOR¹ oder 〉C=NN(R¹)₂ entsprechend der obigen Definition darstellt und Y und Het die oben angegebene Bedeutung besitzen, die Umsetzung von Methylamin mit einem Dithioester der allgeeinen Formel: 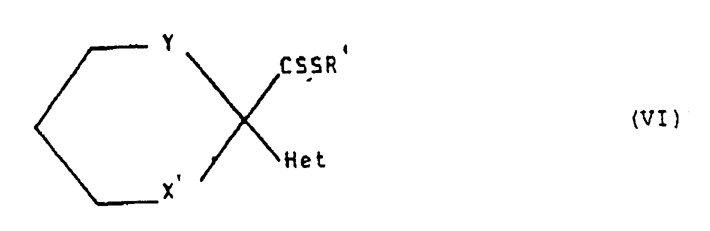 worin R' für einen gerad- oder verzweigtkettigen Alkylrest mit 1 bis 4 Kohlenstoffatom(en) oder einen Benzyl- oder Carboxymethylrest steht und X', Y und Het die oben angegebene Bedeutung besitzen;
gegebenenfalls gefolgt von der Stufe eines Umwandelns eines so erhaltenen Thioformamidderivats in ein pharmazeutisch akzeptables Salz davon.

2. Verfahren nach Anspruch 1, wobei Y für einen Methylenrest oder eine Valenzbindung steht.

3. Verfahren nach Anspruch 1 oder 2, wobei X für die Carbonylgruppe oder eine Gruppe der Formel 〉C=NOR¹ mit R¹ in der in Anspruch 1 angegebenen Bedeutung steht.

4. Verfahren nach Anspruch 1, 2 oder 3, wobei R¹ für einen gerad- oder verzweigtkettigen Alkylrest mit 1 bis 4 Kohlenstoffatom(en), der gegebenenfalls entsprechend der Definition in Anspruch 1 substituiert ist, oder einen Benzyl-, Phenetyl-, 1-Naphthylmethyl-, 2-Naphthylmethyl- oder Pyrid-3-yl-methylrest, der jeweils gegebenenfalls entsprechend der Definition in Anspruch 1 substituiert ist, steht.

5. Verfahren nach einem der vorhergehenden Ansprüche, worin Het für 3-Pyridyl, 6-Chlorpyrid-3-yl, 5-Brompyrid-3-yl, 3-Chinolinyl, 4-Isochinolinyl oder 5-Pyrimidyl steht.

6. Verfahren nach einem der Ansprüche 1, 2, 4 und 5 zur Herstellung einer Verbindung der allgemeinen Formel: 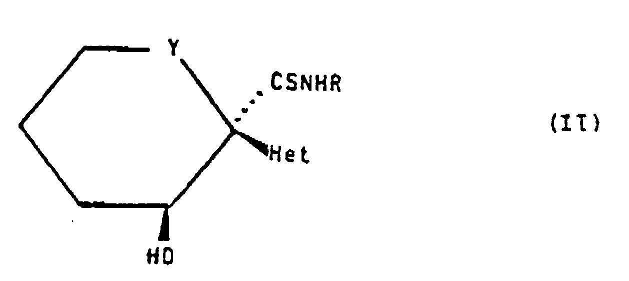 worin R, Het und Y die in einem der Ansprüche 1 bis 5 angegebene Bedeutung besitzen, bei der sich die Hydroxygruppe in trans-Stellung relativ zu der -CSNHR-Gruppe befindet.

7. Verfahren nach Anspruch 1 zur Herstellung eines Thioformamidderivats der allgemeinen Formel (I), nämlich:
(±)-N-Methyl-2-oxo-1-(3-pyridyl)cyclohexancarbothioamid
(±)-N-Methyl-2-oxo-1-(3-chinolinyl)-cyclohexancarbothioamid
(±)-N-Methyl-2-oxo-1-(3-pyridyl)cycloheptancarbothioamid
(±)-trans-N-Methyl-2-hydroxy-1-(3-pyridyl)cyclohexancarbothioamid
(±)-cis-N-Methyl-2-hydroxy-1-(3-pyridyl)cyclohexancarbothioamid
(±)-anti-N-Methyl-2-hydroximino-1-(3-pyridyl)cyclohexancarbothioamid
(±)-anti-N-Methyl-2-methoxyimino-1-(3-pyridyl)cyclohexancarbothioamid
(±)-anti-N-Methyl-2-benzyloxyimino-1-(3-pyridyl)-cyclohexancarbothioamid
(±)-anti-N-Methyl-2-hydroxyimino-1-(3-chinolinyl)cyclohexancarbothioamid
(2S)-anti-2-(methoxymethyl)-1-[2'-(3-chinolinyl)-2'-methylthiocarbamoylcyclohexylidenamino]pyrrolidin
(2S)-anti-2-(Methoxymethyl)-1-[2'-(3-pyridyl)-2'-methylthiocarbamoylcyclohexylidenamino]pyrrolidin
(±)-anti-N-Methyl-2-(2-dimethylaminoethoxyimino)-1-(3-pyridyl)cyclohexancarbothioamid
(±)-anti-N-Methyl-2-(2-aminoethoxyimino)-1-(3-pyridyl)cyclohexancarbothioamid
(±)-anti-N-Methyl-2-methoxycarbonylmethoxyimino-1-(3-pyridyl)cyclohexancarbothioamid
(±)-anti-N-Methyl-2-carbamoylmethoxyimino-1-(3-pyridyl)cyclohexancarbothioamid
(±)-anti-N-Methyl-2-(2,3-dihydroxypropoxyimino)-1-(3-pyridyl)cyclohexancarbothioamid
(±)-anti-N-Methyl-2-(2-hydroxyethoxyimino)-1-(3-pyridyl)cyclohexancarbothioamid
(±)-N-Methyl-2-oxo-1-(6-chloropyrid-3-yl)-cyclohexancarbothioamid
(±)-N-Methyl-2-oxo-1-(5-bromopyrid-3-yl)-cyclohexancarbothioamid
(±)-N-Ethyl-2-oxo-1-(3-pyridyl)cyclohexancarbothioamid
(±)-N-Ethyl-2-oxo-1-(3-chinolinyl)-cyclohexancarbothioamid
(±)-anti-N-Methyl-2-dimethylhydrazono-1-(3-chinolinyl)-cyclohexancarbothioamid
(±)-anti-N-Methyl-2-diethylhydrazono-1-(3-chinolinyl)-cyclohexancarbothioamid
(±)-anti-N-Methyl-2-benzyloxyimino-1-(3-chinolinyl)-cyclohexancarbothioamid
(±)-anti-N-Methyl-2-methoxyimino-1-(3-chinolinyl)-cyclohexancarbothioamid
(±)-anti-N-Methyl-2-ethoxyimino-1-(3-chinolinyl)-cyclohexancarbothioamid
(±)-anti-N-Methyl-2-butoxyimino-1-(3-chinolinyl)-cyclohexancarbothioamid
(±)-anti-N-Methyl-2-(3-isopropylamino-2-hydroxy-propoxyimino)-1-(3-chinolinyl)cyclohexancarbothioamid
(±)-anti-N-Methyl-2-tert.-butoxyimino-1-(3-chinolinyl)-cyclohexancarbothioamid
(±)-anti-N-Methyl-2-prop-2-enoxyimino-1-(3-chinolinyl)-cyclohexancarbothioamid
(±)-anti-N-Methyl-2-naphth-2-ylmethoxyimino-1-(3-chinolinyl)cyclohexancarbothioamid
(±)-anti-N-Methyl-2-phenethyloxyimino-1-(3-chinolinyl)-cyclohexancarbothioamid
(±)-anti-N-Methyl-2-naphth-1-ylmethoxyimino-1-(3-chinolinyl)cyclohexancarbothioamid
(±)-anti-N-Methyl-2-(3-tert.-butylamino-2-hydroxypropoxyimino-1-(3-chinolinyl)cyclohexancarbothioamid
(±)-anti-N-Methyl-2-isopropoxyimino-1-(3-chinolinyl)-cyclohexancarbothioamid
(±)-anti-N-Methyl-2-(4-hydroxybenzyloxyimino-1-(3-chinolinyl)cyclohexancarbothioamid
(±)-anti-N-Methyl-2-(4-fluorbenzyloxyimino)-1-(3-chinolinyl)cyclohexancarbothioamid
(±)-anti-N-Methyl-2-(4-fluorbenzyloxyimino-1-(3-pyridyl)cyclohexancarbothioamid
(±)-anti-N-Methyl-2-(2,3,4,5,6-pentafluorbenzyloxyimino)-1-(3-pyridyl)cyclohexancarbothioamid
(±)-anti-N-Methyl-2-isopropoxyimino-1-(3-pyridyl)-cyclohexancarbothioamid
(±)-anti-N-Methyl-2-tert.-butoxyimino-1-(3-pyridyl)-cyclohexancarbothioamid
(±)-anti-N-Methyl-2-[4-(3-tert.-butylamino-2-hydroxypropoxy)benzyloxyimino]-1-(3-pyridyl)cyclohexancarbothioamid
(±)-anti-N-Methyl-2-(4-hydroxybenzyloxyimino)-1-(3-pyridyl)cyclohexancarbothioamid
(±)-anti-N-Methyl-2-(3-fluorbenzyloxyimino)-1-(3-pyridyl)cyclohexancarbothioamid
(±)-anti-N-Methyl-2-(2-fluorbenzyloxyimino)-1-(3-pyridyl)cyclohexancarbothioamid
(±)-anti-N-Methyl-2-(3-pyridylmethoxyimino)-1-(3-pyridyl)cyclohexancarbothioamid
(±)-anti-N-Methyl-2-(4-nitrobenzyloxyimino)-1-(3-pyridyl)cyclohexancarbothioamid
(±)-anti-N-Methyl-2-(4-cyanobenzyloxyimino)-1-(3-pyridyl)cyclohexancarbothioamid
(±)-anti-N-Methyl-2-(3,4-difluorbenzyloxyimino)-1-(3-pyridyl)cyclohexancarbothioamid
(±)-anti-N-Methyl-2-(4-methoxybenzyloxyimino)-1-(3-pyridyl)cyclohexancarbothioamid
(±)-anti-N-Methyl-2-(2-trifluormethylbenzyloxyimino)-1-(3-pyridyl)cyclohexancarbothioamid
(±)-N-Methyl-2-oxo-1-(4-isochinolinyl)cyclohexancarbothioamid
(±)-anti-N-Methyl-2-benzyloxyimino-1-(4-isochinolinyl)-cyclohexancarbothioamid
(±)-N-Methyl-2-oxo-1-(5-pyrimidyl)cyclohexancarbothioamid
(±)-N-Methyl-2-oxo-1-(3-pyridyl)cyclopentancarbothioamid
(±)-anti-N-Methyl-2-(4-fluorbenzyloxyimino)-1-(3-pyridyl)cyclopentancarbothioamid
(±)-anti-N-Methyl-2-methoxyimino-1-(3-pyridyl)-cyclopentancarbothioamid oder
(±)-anti-N-Methyl-2-benzyloxyimino-1-(3-pyridyl)-cyclopentancarbothioamid
oder eines pharmazeutisch akzeptablen Salzes davon.

8. Verfahren nach einem der vorhergehenden Ansprüche, gefolgt von der Stufe einer Formulierung eines Thioformamidderivats der allgemeinen Formel (I) entsprechend der Definition in Anspruch 1 oder eines pharmazeutisch akzeptablen Salzes davon mit einem pharmazeutisch akzeptablen Träger oder Überzug.

## Patentansprüche (Patentansprüche für folgende(n) Vertragsstaat(en): GR)

1. Verfahren zur Herstellung eines Thioformamidderivats der allgemeinen Formel: 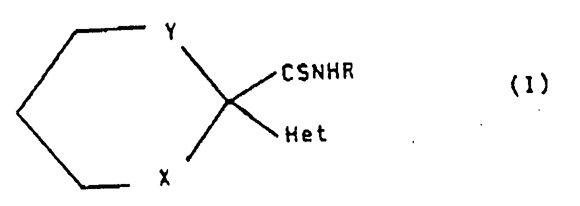 worin bedeuten:
R einen gerad- oder verzweigtkettigen Alkylrest mit 1 bis 4 Kohlenstoffatom(en),
Het einen aromatischen heterocyclischen Rest mit einem oder zwei Stickstoffatom(en) (gegebenenfalls substituiert durch einen gerad- oder verzweigtkettigen Alkyl- oder Alkoxyrest mit 1 bis 4 Kohlenstoffatom(en) oder ein Halogenatom), ausgewählt aus Pyrid-3-yl, Isochinolin-4-yl, Tetrahydrochinolin-3-yl, Chinolin-3-yl, Pyridazin-4-yl, Pyrimid-5-yl, Thiazol-5-yl, Thieno[2,3-b]pyridin-5-yl, Pyrazin-2-yl, Indol-3-yl und Thieno[3,2-b]pyridin-6-yl,
Y einen Ethylen- oder Methylenrest oder eine Valenzbindung und
X eine Carbonyl- oder Hydroxymethylengruppe oder eine Gruppe der Formel: 〉C=NOR¹, 〉C=NN(R¹)₂ oder 〉C=NN(R¹)CON(R¹)₂, worin die Symbole R¹, die gleich oder verschieden sein können, jeweils für ein Wasserstoffatom oder einen gerad- oder verzweigtkettigen Alkylrest mit 1 bis 4 Kohlenstoffatom(en), der nicht substituiert ist oder durch einen oder mehrere Substituenten, ausgewählt aus C₂₋₄-Alkenyl-, Carboxy-, C₂₋₅-Alkoxycarbonyl-, Hydroxy-, C₁₋₄-Alkoxy-, Carbamoyl-(unsubstituiert oder substituiert durch eine oder zwei C₁₋₄-Alkylgruppe(n)), Amino-, C₁₋₄-Alkylamino- und di-C₁₋₄-Alkylaminogruppen, substituiert ist, oder für einen Benzyl-, Phenethyl-, 1-Naphthylmethyl-, 2-Naphthylmethyl- oder Pyrid-3-ylmethylrest, jeweils gegebenenfalls substituiert am Ring durch ein oder mehrere Halogenatom(e) oder Hydroxy-, C₁₋₄-Alkyl-, C₁₋₄-Alkoxy (wobei Alkoxy gegebenenfalls durch die für die durch R¹ dargestellten Alkylgruppen definierten Substituenten substituiert sein kann), Cyano-, Nitro-, Trifluormethyl-, Carboxy-, C₁₋₄-Alkylamino-, C₂₋₅-Alkanoylamino-, C₂₋₅-Alkoxycarbonylgruppe(n), stehen oder zwei R¹-Substituenten am selben Stickstoffatom zusammen einen gerad- oder verzweigtkettigen Alkylenrest mit 4 bis 6 Kohlenstoffatomen in der Kette, die gegebenenfalls durch die für die durch R¹ dargestellten Alkylgruppen definierten Substituenten substituiert ist, bilden können,
sowie pharmazeutisch akzeptabler Salze davon, umfassend:
(A) wenn X für die Carbonylgruppe oder eine Gruppe der Formel 〉C=NOR¹ oder 〉C=NN(R¹)₂ steht, wobei R¹, Y, R und Het die oben angegebene Bedeutung besitzen, die Umsetzung einer Verbindung der allgemeinen Formel: 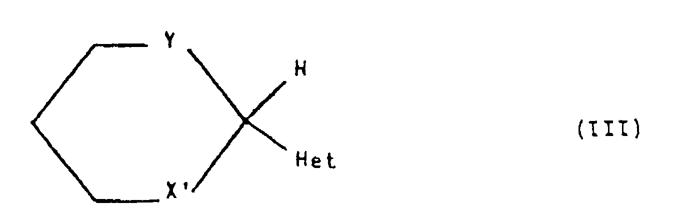 worin X' für die Carbonylgruppe oder eine Gruppe der Formel 〉C=NOR¹ oder 〉C=NN(R¹)₂ entsprechend der oben angegebenen Definition steht und Y und Het die oben angegebene Bedeutung besitzen, mit einem Isothiocyanat der allgemeinen Formel:
R-N=C=S (IV)
worin R für einen gerad- oder verzweigtkettigen Alkylrest mit 1 bis 4 Kohlenstoffatom(en) steht;
(B) wenn X für die Carbonylgruppe steht, die stereoselektive Umsetzung eines Enantiomerengemisches der allgemeinen Formel (III), worin Y und Het die oben angegebene Bedeutung besitzen und X' für die Carbonylgruppe steht, mit einem chiralen Hilfsstoff und Umsetzung des erhaltenen Produkts mit einer Verbindung der allgemeinen Formel:
R-N=C=S (IV)
worin R die oben angegebene Bedeutung besitzt, und anschließende Entfernung des chiralen Hilfsstoffs;
(C) wenn X für die Hydroxymethylengruppe steht und Y, R und Het die oben angegebene Bedeutung besitzen, die Reduktion einer Verbindung der allgemeinen Formel (I), worin X für die Carbonylgruppe steht und Y, R und Het die oben angegebene Bedeutung besitzen;
(D) wenn X für eine Gruppe der Formel 〉C=NOR¹, 〉C=NN(R¹)₂ oder 〉C=NN(R¹)CON(R¹)₂ entsprechend der obigen Definition steht und Y, R und Het die oben angegebene Bedeutung besitzen, die Umsetzung einer Verbindung der allgemeinen Formel (I), worin X für die Carbonylgruppe steht, mit einer Verbindung der allgemeinen Formel:
NH₂OR¹ (Va)
NH₂N(R¹)₂ (Vb)
oder
NH₂N(R¹)CON(R¹)₂ (Vc)
worin R¹ die oben angegebene Bedeutung besitzt, oder mit einem Säureadditionssalz davon;
(E) wenn R für den Methylrest steht und X die Carbonylgruppe oder eine Gruppe der Formel 〉C=NOR¹ oder 〉C=NN(R¹)₂ entsprechend der obigen Definition darstellt und Y und Het die oben angegebene Bedeutung besitzen, die Umsetzung von Methylamin mit einem Dithioester der allgeeinen Formel: 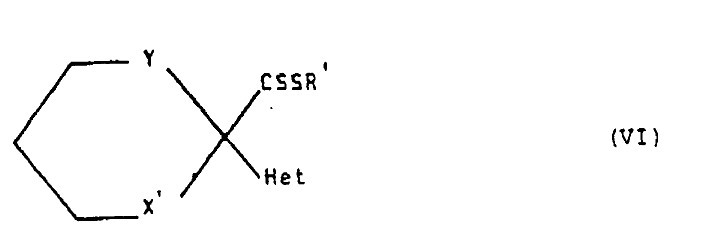 worin R' für einen gerad- oder verzweigtkettigen Alkylrest mit 1 bis 4 Kohlenstoffatom(en) oder einen Benzyl- oder Carboxymethylrest steht und X', Y und Het die oben angegebene Bedeutung besitzen;
gegebenenfalls gefolgt von der Stufe eines Umwandelns eines so erhaltenen Thioformamidderivats in ein pharmazeutisch akzeptables Salz davon.

2. Verfahren nach Anspruch 1, wobei Y für einen Methylenrest oder eine Valenzbindung steht.

3. Verfahren nach Anspruch 1 oder 2, wobei X für die Carbonylgruppe oder eine Gruppe der Formel 〉C=NOR¹ mit R¹ in der in Anspruch 1 angegebenen Bedeutung steht.

4. Verfahren nach Anspruch 1, 2 oder 3, wobei R¹ für einen gerad- oder verzweigtkettigen Alkylrest mit 1 bis 4 Kohlenstoffatom(en), der gegebenenfalls entsprechend der Definition in Anspruch 1 substituiert ist, oder einen Benzyl-, Phenetyl-, 1-Naphthylmethyl-, 2-Naphthylmethyl- oder Pyrid-3-yl-methylrest, der jeweils gegebenenfalls entsprechend der Definition in Anspruch 1 substituiert ist, steht.

5. Verfahren nach einem der vorhergehenden Ansprüche, worin Het für 3-Pyridyl, 6-Chlorpyrid-3-yl, 5-Brompyrid-3-yl, 3-Chinolinyl, 4-Isochinolinyl oder 5-Pyrimidyl steht.

6. Verfahren nach einem der Ansprüche 1, 2, 4 und 5 zur Herstellung einer Verbindung der allgemeinen Formel: 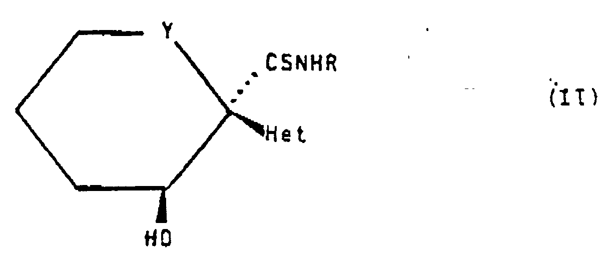 worin R, Het und Y die in einem der Ansprüche 1 bis 5 angegebene Bedeutung besitzen, bei der sich die Hydroxygruppe in trans-Stellung relativ zu der -CSNHR-Gruppe befindet.

7. Verfahren nach Anspruch 1 zur Herstellung eines Thioformamidderivats der allgemeinen Formel (I), nämlich:
(±)-N-Methyl-2-oxo-1-(3-pyridyl)cyclohexancarbothioamid
(±)-N-Methyl-2-oxo-1-(3-chinolinyl)-cyclohexancarbothioamid
(±)-N-Methyl-2-oxo-1-(3-pyridyl)cycloheptancarbothioamid
(±)-trans-N-Methyl-2-hydroxy-1-(3-pyridyl)cyclohexancarbothioamid
(±)-cis-N-Methyl-2-hydroxy-1-(3-pyridyl)cyclohexancarbothioamid
(±)-anti-N-Methyl-2-hydroximino-1-(3-pyridyl)cyclohexancarbothioamid
(±)-anti-N-Methyl-2-methoxyimino-1-(3-pyridyl)cyclohexancarbothioamid
(±)-anti-N-Methyl-2-benzyloxyimino-1-(3-pyridyl)-cyclohexancarbothioamid
(±)-anti-N-Methyl-2-hydroxyimino-1-(3-chinolinyl)cyclohexancarbothioamid
(2S)-anti-2-(methoxymethyl)-1-[2'-(3-chinolinyl)-2'-methylthiocarbamoylcyclohexylidenamino]pyrrolidin
(2S)-anti-2-(Methoxymethyl)-1-[2'-(3-pyridyl)-2'-methylthiocarbamoylcyclohexylidenamino]pyrrolidin
(±)-anti-N-Methyl-2-(2-dimethylaminoethoxyimino)-1-(3-pyridyl)cyclohexancarbothioamid
(±)-anti-N-Methyl-2-(2-aminoethoxyimino)-1-(3-pyridyl)cyclohexancarbothioamid
(±)-anti-N-Methyl-2-methoxycarbonylmethoxyimino-1-(3-pyridyl)cyclohexancarbothioamid
(±)-anti-N-Methyl-2-carbamoylmethoxyimino-1-(3-pyridyl)cyclohexancarbothioamid
(±)-anti-N-Methyl-2-(2,3-dihydroxypropoxyimino)-1-(3-pyridyl)cyclohexancarbothioamid
(±)-anti-N-Methyl-2-(2-hydroxyethoxyimino)-1-(3-pyridyl)cyclohexancarbothioamid
(±)-N-Methyl-2-oxo-1-(6-chloropyrid-3-yl)-cyclohexancarbothioamid
(±)-N-Methyl-2-oxo-1-(5-bromopyrid-3-yl)-cyclohexancarbothioamid
(±)-N-Ethyl-2-oxo-1-(3-pyridyl)cyclohexancarbothioamid
(±)-N-Ethyl-2-oxo-1-(3-chinolinyl)-cyclohexancarbothioamid
(±)-anti-N-Methyl-2-dimethylhydrazono-1-(3-chinolinyl)-cyclohexancarbothioamid
(±)-anti-N-Methyl-2-diethylhydrazono-1-(3-chinolinyl)-cyclohexancarbothioamid
(±)-anti-N-Methyl-2-benzyloxyimino-1-(3-chinolinyl)-cyclohexancarbothioamid
(±)-anti-N-Methyl-2-methoxyimino-1-(3-chinolinyl)-cyclohexancarbothioamid
(±)-anti-N-Methyl-2-ethoxyimino-1-(3-chinolinyl)-cyclohexancarbothioamid
(±)-anti-N-Methyl-2-butoxyimino-1-(3-chinolinyl)-cyclohexancarbothioamid
(±)-anti-N-Methyl-2-(3-isopropylamino-2-hydroxy-propoxyimino)-1-(3-chinolinyl)cyclohexancarbothioamid
(±)-anti-N-Methyl-2-tert.-butoxyimino-1-(3-chinolinyl)-cyclohexancarbothioamid
(±)-anti-N-Methyl-2-prop-2-enoxyimino-1-(3-chinolinyl)-cyclohexancarbothioamid
(±)-anti-N-Methyl-2-naphth-2-ylmethoxyimino-1-(3-chinolinyl)cyclohexancarbothioamid
(±)-anti-N-Methyl-2-phenethyloxyimino-1-(3-chinolinyl)-cyclohexancarbothioamid
(±)-anti-N-Methyl-2-naphth-1-ylmethoxyimino-1-(3-chinolinyl)cyclohexancarbothioamid
(±)-anti-N-Methyl-2-(3-tert.-butylamino-2-hydroxypropoxyimino-1-(3-chinolinyl)cyclohexancarbothioamid
(±)-anti-N-Methyl-2-isopropoxyimino-1-(3-chinolinyl)-cyclohexancarbothioamid
(±)-anti-N-Methyl-2-(4-hydroxybenzyloxyimino-1-(3-chinolinyl)cyclohexancarbothioamid
(±)-anti-N-Methyl-2-(4-fluorbenzyloxyimino)-1-(3-chinolinyl)cyclohexancarbothioamid
(±)-anti-N-Methyl-2-(4-fluorbenzyloxyimino-1-(3-pyridyl)cyclohexancarbothioamid
(±)-anti-N-Methyl-2-(2,3,4,5,6-pentafluorbenzyloxyimino)-1-(3-pyridyl)cyclohexancarbothioamid
(±)-anti-N-Methyl-2-isopropoxyimino-1-(3-pyridyl)-cyclohexancarbothioamid
(±)-anti-N-Methyl-2-tert.-butoxyimino-1-(3-pyridyl)-cyclohexancarbothioamid
(±)-anti-N-Methyl-2-[4-(3-tert.-butylamino-2-hydroxypropoxy)benzyloxyimino]-1-(3-pyridyl)cyclohexancarbothioamid
(±)-anti-N-Methyl-2-(4-hydroxybenzyloxyimino)-1-(3-pyridyl)cyclohexancarbothioamid
(±)-anti-N-Methyl-2-(3-fluorbenzyloxyimino)-1-(3-pyridyl)cyclohexancarbothioamid
(±)-anti-N-Methyl-2-(2-fluorbenzyloxyimino)-1-(3-pyridyl)cyclohexancarbothioamid
(±)-anti-N-Methyl-2-(3-pyridylmethoxyimino)-1-(3-pyridyl)cyclohexancarbothioamid
(±)-anti-N-Methyl-2-(4-nitrobenzyloxyimino)-1-(3-pyridyl)cyclohexancarbothioamid
(±)-anti-N-Methyl-2-(4-cyanobenzyloxyimino)-1-(3-pyridyl)cyclohexancarbothioamid
(±)-anti-N-Methyl-2-(3,4-difluorbenzyloxyimino)-1-(3-pyridyl)cyclohexancarbothioamid
(±)-anti-N-Methyl-2-(4-methoxybenzyloxyimino)-1-(3-pyridyl)cyclohexancarbothioamid
(±)-anti-N-Methyl-2-(2-trifluormethylbenzyloxyimino)-1-(3-pyridyl)cyclohexancarbothioamid
(±)-N-Methyl-2-oxo-1-(4-isochinolinyl)cyclohexancarbothioamid
(±)-anti-N-Methyl-2-benzyloxyimino-1-(4-isochinolinyl)-cyclohexancarbothioamid
(±)-N-Methyl-2-oxo-1-(5-pyrimidyl)cyclohexancarbothioamid
(±)-N-Methyl-2-oxo-1-(3-pyridyl)cyclopentancarbothioamid
(±)-anti-N-Methyl-2-(4-fluorbenzyloxyimino)-1-(3-pyridyl)cyclopentancarbothioamid
(±)-anti-N-Methyl-2-methoxyimino-1-(3-pyridyl)-cyclopentancarbothioamid oder
(±)-anti-N-Methyl-2-benzyloxyimino-1-(3-pyridyl)-cyclopentancarbothioamid
oder eines pharmazeutisch akzeptablen Salzes davon.

8. Verfahren nach einem der vorhergehenden Ansprüche, gefolgt von der Stufe einer Formulierung eines Thioformamidderivats der allgemeinen Formel (I) entsprechend der Definition in Anspruch 1 oder eines pharmazeutisch akzeptablen Salzes davon mit einem pharmazeutisch akzeptablen Träger oder Überzug.

9. Verbindung der allgemeinen Formel (III) nach Anspruch 1, worin X', Y und Het die in Anspruch 1 angegebene Bedeutung besitzen, wobei gilt, daß Y nicht für eine direkte Bindung steht, wenn Het eine gegebenenfalls substituierte Pyrazin-2-yl-Gruppe darstellt, und Y nicht für Methylen steht, wenn Het eine gegebenenfalls substituierte Indol-3-yl-Gruppe darstellt.

## Revendications (Revendications pour l'(les) Etat(s) contractant(s) suivant(s): AT, BE, CH, DE, FR, GB, IT, LI, LU, NL, SE)

1. Un dérivé de thioformamide de formule générale : 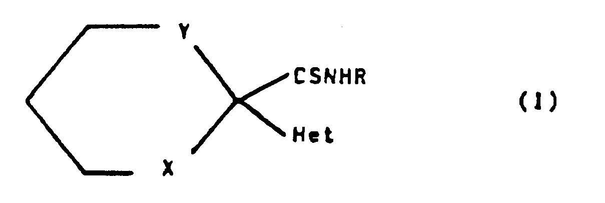 dans laquelle R représente un groupe alkyle à chaîne droite ou ramifiée en C₁-C₄, Hét représente un radical hétérocyclique aromatique contenant 1 ou 2 atomes d'azote (éventuellement substitué par un radical alkyle ou alcoxy à châine droite ou ramifiée en C₁-C₄ ou par un atome d'halogène), choisi parmi les groupes 3-pyridyle, 4-isoquinoléyle, 3-tétrahydroquinoléyle, 3-quinoléyle, 4-pyridazinyle, 5-pyrimidyle, 5-thiazolyle, 5-thiéno[2,3-b]pyridyle, 2-pyrazinyle, 3-indolyle et 6-thiéno[3,2-b]pyridyle, Y représente un radical éthylène ou méthylène ou une liaison de valence et X représente un groupe carbonyle ou hydroxyméthylène ou un groupe de formule : 〉C = NOR¹, 〉C = NN(R¹)₂ ou 〉C = NN(R¹)CON(R¹)₂ dans lesquelles les symboles R¹, qui peuvent être identiques ou différents, représentent chacun un atome d'hydrogène ou un radical alkyle à chaîne droite ou ramifiée en C₁-C₄, non substitué ou substitué par un ou plusieurs substituants choisis parmi les groupes alcényle en C₂-C₄, carboxy, (alcoxy en C₂-C₅)carbonyle, hydroxy, alcoxy en C₁-C₄, carbamoyle (non substitué ou substitué par un ou deux groupes alkyles en C₁-C₄), amino, alkylamino en C₁-C₄ et di(alkyl en C₁-C₄)amino ou représente un radical benzyle, phénéthyle, 1-naphtylméthyle, 2-naphtylméthyle ou 3-pyridylméthyle, chacun d'eux pouvant être substitué au noyau par un ou plusieurs atomes d'halogènes ou groupes hydroxy, alkyles en C₁-C₄, alcoxy en C₁-C₄ (le groupe alcoxy étant substitué ou non comme pour les groupes alkyles représentés par R¹), cyano, nitro, trifluorométhyle, carboxy, alkylamino en C₁-C₄, alcanoylamino en C₂-C₅, (alcoxy en C₂-C₅)carbonyle, ou bien deux substituants R¹ sur le même atome d'azote peuvent former ensemble un radical alkylène à chaîne droite ou ramifiée en C₄-C₆ dans la chaîne, qui est substitué ou non comme défini pour les groupes alkyles représentés par R¹ et ses sels acceptables en pharmacie.

2. Un composé selon la revendication 1, dans lequel Y représente un radical méthylène ou une liaison de valence.

3. Un composé selon la revendication 1 ou 2, dans lequel X représente le groupe carbonyle ou un groupe de formule 〉C = NOR¹ dans laquelle R¹ est défini comme à la revendication 1.

4. Un composé selon la revendication 1, 2 ou 3, dans lequel R¹ représente un radical alkyle à chaîne droite ou ramifiée en C₁-C₄ qui est substitué ou non comme défini dans la revendication 1, ou bien représente un radical benzyle, phénéthyle, 1-naphtylméthyle, 2-naphtylméthyle ou 3-pyridylméthyle, chacun d'eux pouvant être substitué au noyau comme défini à la revendication 1.

5. Un composé selon l'une quelconque des revendications précédentes dans lequel Hét représente un groupe 3-pyridyle, 6-chloro-3-pyridyle, 5-bromo-3-pyridyle, 3-quinoléyle, 4-isoquinoléyle ou 5-pyrimidyle.

6. Un composé selon l'une quelconque des revendications 1, 2, 4 et 5 de formule générale : 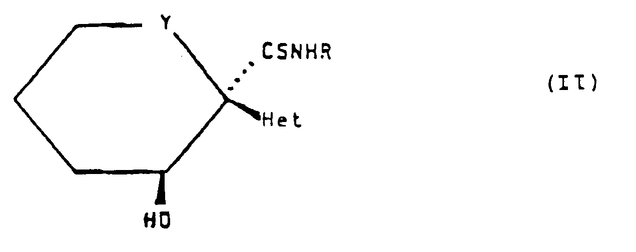 dans laquelle R, Hét et Y sont tels que définis dans l'une quelconque des revendications 1 à 5, dans lequel le groupe hydroxy est dans la position trans par rapport au groupe -CSNHR.

7. Un composé selon la revendication 1, qui est choisi parmi les suivants :
(±)-N-méthyl-2-oxo-1-(3-pyridyl)cyclohexanecarbothioamide,
(±)-N-méthyl-2-oxo-1-(3-quinoléyl)cyclohexanecarbothiamide,
(±)-N-méthyl-2-oxo-1-(3-pyridyl)cycloheptanecarbothioamide,
(±)-trans-N-méthyl-2-hydroxy-1-(3-pyridyl)cyclohexanecarbothioamide,
(±)-cis-N-méthyl-2-hydroxy-1-(3-pyridyl)cyclohexanecarbothiamide,
(±)-anti-N-méthyl-2-hydroxyimino-1-(3-pyridyl)cyclohexanecarbothioamide,
(±)-anti-N-méthyl-2-méthoxyimino-1-(3-pyridyl)cyclohexanecarbothioamide,
(±)-anti--N--méthyl-2-benzyloxyimino-1-(3-pyridyl)cyclohexanecarbothioamide,
(±)-anti-N-méthyl-2-hydroxyimino-1-(3-quinoléyl)cyclohexanecarbothioamide,
(2S)-anti-2-(méthoxyméthyl)-1-[2'-(3-quinoléyl)-2'-méthylthiocarbamoylcyclohexylidèneamino]pyrrolidine,
(2S)-anti-2-(méthoxyméthyl)-1-[2'-(3-pyridyl)-2'-méthylthiocarbamoylcyclohexylidèneamino]-pyrrolidine,
(±)-anti-N-méthyl-2-(2-diméthylaminoéthoxyimino)-1-(3-pyridyl)cyclohexanecarbothioamide,
(±)-anti-N-méthyl-2-(2-aminoéthoxyimino)-1-(3-pyridyl)cyclohexanecarbothioamide,
(±)-anti-N-méthyl-2-méthoxycarbonylméthoxyimino-1-(3-pyridyl)cyclohexanecarbothioamide,
(±)-anti-N―méthyl-2-carbamoylméthoxyimino-1-(3-pyridyl)cyclohexanecarbothioamide,
(±)-anti-N-méthyl-2-(2,3-dihydroxypropoxyimino)-1-(3-pyridyl)cyclohexanecarbothioamide,
(±)-anti-N-méthyl-2-(2-hydroxyéthoxyimino)-1-(3-pyridyl)cyclohexanecarbothioamide,
(±)-N-méthyl-2-oxo-1-(6-chloro-3-pyridyl)cyclohexanecarbothioamide,
(±)-N-méthyl-2-oxo-1-(5-bromo-3-pyridy)cyclohexanecarbothioamide,
(±)-N-éthyl-2-oxo-1-(3-pyridyl)cyclohexanecarbothioamide,
(±)-N-éthyl-2-oxo-1-(3-quinoléyl)cyclohexanecarbothioamide,
(±)-anti-N-méthyl-2-diméthylhydrazono-1-(3-quinoléyl)cyclohexanecarbothioamide,
(±)-anti-N-méthyl-2-diéthylhydrazono-1-(3-quinoléyl)cyclohexanecarbothioamide,
(±)-anti-N-méthyl-2-benzyloxyimino-1-(3-quinoléyl)cyclohexanecarbothioamide,
(±)-anti-N-méthyl-2-méthoxyimino-1-(3-quinoléyl)cyclohexanecarbothioamide,
(±)-anti-N-méthyl-2-éthoxyimino-1-(3-quinoléyl)cyclohexanecarbothioamide,
(±)-anti-N-méthyl-2-butoxyimino-1-(3-quinoléyl)cyclohexanecarbothioamide,
(±)-anti-N-méthyl-2-(3-isopropylamino-2-hydroxypropoxyimino)-1-(3-quinoléyl)cyclohexanecarbothioamide,
(±)-anti-N-méthyl-2-t.butoxyimino-1-(3-quinoléyl)cyclohexanecarbothioamide,
(±)-anti-N-méthyl-2-(2-propényl)oxyimino-1-(3-quinoléyl)cyclohexanecarbothioamide,
(±)-anti-N-méthyl-2-(β-naphtyl)méthoxyimino-1-(3-quinoléyl)cyclohexanecarbothioamide,
(±)-anti-N-méthyl-2-phénéthyloxyimino-1-(3-quinoléyl)cyclohexanecarbothioamide,
(±)-anti-N-méthyl-2-(α-naphtyl)méthoxyimino-1-(3-quinoléyl)cyclohexanecarbothioamide,
(±)-anti-N-méthyl-2-(3-t.butylamino-2-hydroxypropoxyimino)-1-(3-quinoléyl)cyclohexanecarbothioamide,
(±)-anti-N-méthyl-2-isopropoxyimino-1-(3-quinoléyl)cyclohexanecarbothioamide,
(±)-anti-N-méthyl-2-(4-hydroxybenzyloxyimino-1-(3-quinoléyl)cyclohexanecarbothioamide,
(±)-anti-N-méthyl-2-(4-fluorobenzyloxyimino)-1-(3-quinoléyl)cyclohexanecarbothioamide,
(±)-anti-N-méthyl-2-(4-fluorobenzyloxyimino)-1-(3-pyridyl)cyclohexanecarbothioamide,
(±)-anti-N-méthyl-2-(2,3,4,5,6-pentafluorobenzyloxyimino)-1-(3-pyridyl)cyclohexanecarbothioamide,
(±)-anti-N-méthyl-2-isopropoxyimino-1-(3-pyridyl)cyclohexanecarbothioamide,
(±)-anti-N-méthyl-2-t.butoxyimino-1-(3-pyridyl)cyclohexanecarbothioamide,
(±)-anti-N-méthyl-2-[4-(3-t.butylamino-2-hydroxypropoxy)benzyloxyimino]-1-(3-pyridyl)cyclohexanecarbothioamide,
(±)-anti-N-méthyl-2-(4-hydroxybenzyloxyimino)-1-(3-pyridyl)cyclohexanecarbothioamide,
(±)-anti-N-méthyl-2-(3-fluorobenzyloxyimino)-1-(3-pyridyl)cyclohexanecarbothioamide,
(±)-anti-N-méthyl-2-(2-fluorobenzyloxyimino)-1-(3-pyridyl)cyclohexanecarbothioamide,
(±)-anti-N-méthyl-2-(3-pyridylméthoxyimino)-1-(3-pyridyl)cyclohexanecarbothioamide,
(±)-anti-N-méthyl-2-(4-nitrobenzyloxyimino)-1-(3-pyridyl)cyclohexanecarbothioamide,
(±)-anti-N-méthyl-2-(4-cyanobenzyloxyimino)-1-(3-pyridyl)cyclohexanecarbothioamide,
(±)-anti-N-méthyl-2-(3,4-difluorobenzyloxyimino)-1-(3-pyridyl)cyclohexanecarbothioamide,
(±)-anti-N-méthyl-2-(4-méthoxybenzyloxyimino)-1-(3-pyridyl)cyclohexanecarbothioamide,
(±)-anti-N-méthyl-2-(2-trifluorométhylbenzyloxyimino)-1-(3-pyridyl)cyclohexanecarbothioamide,
(±)-N-méthyl-2-oxo-1-(4-isoquinoléyl)cyclohexanecarbothioamide,
(±)-anti-N-méthyl-2--benzyloxyimino-1-(4-isoquinoléyl)cyclohexanecarbothioamide,
(±)-N-méthyl-2-oxo-1-(5-pyrimidyl)cyclohexanecarbothioamide,
(±)-N-méthyl-2-oxo-1-(3-pyridyl)cyclopentanecarbothioamide,
(±)-anti-N-méthyl-2-(4-fluorobenzyloxyimino)-1-(3-pyridyl)cyclopentanecarbothioamide,
(±)-anti-N-méthyl-2-méthoxyimino-1-(3-pyridyl)cyclopentanecarbothioamide et
(±)-anti-N-méthyl-2-benzyloxyimino-1-(3-pyridyl)cyclopentanecarbothioamide et leurs sels acceptables en pharmacie.

8. Un procédé pour la préparation d'un dérivé de thioformamide de formule générale (I) selon la revendication 1, qui comprend :
(A) lorsque X représente le groupe carbonyle ou un groupe de formule 〉C = NOR¹ ou 〉C = NN(R¹)₂ dans lesquelles R¹ est défini comme à la revendication 1 et Y, R et Hét sont définis comme à la revendication 1, la réaction d'un composé de formule générale : 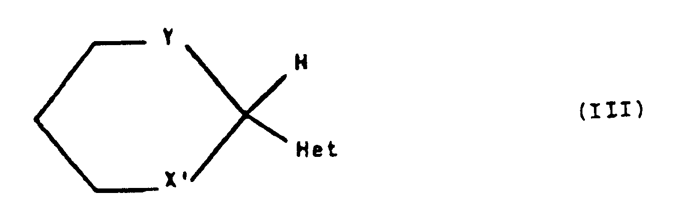 dans laquelle X' représente le groupe carbonyle ou un groupe de formule 〉C = NOR¹ ou 〉C = NN(R¹)₂ défini comme à la revendication 1 et Y et Hét sont définis comme à la revendication 1 avec un isothiocyanate de formule générale :
R - N = C = S (IV)
dans laquelle R représente un radical alkyle à chaîne droite ou ramifiée en C₁-C₄ ;
(B) lorsque X représente le groupe carbonyle, la réaction stéréosélective d'un mélange d'énantiomères de formule générale (III) dans laquelle Y et Hét sont définis comme à la revendication 1 et X' représente le groupe carbonyle avec un agent auxiliaire chiral et la réaction du produit obtenu avec un composé de formule générale :
R - N = C = S (IV)
dans laquelle R est défini comme à la revendication 1, suivie d'élimination de l'agent auxiliaire chiral ;
(C) lorsque X représente le groupe hydroxyéthylène et Y, R et Hét sont définis comme à la revendication 1, la réduction d'un composé de formule générale (I) dans laquelle X représente un groupe carbonyle et Y, R et Hét sont définis comme à la revendication 1 ;
(D) lorsque X représente un groupe de formule 〉C = NOR¹, 〉C = NN(R¹)₂ ou 〉C = NN(R¹)CON(R¹)₂ défini comme à la revendication 1 et Y, R et Hét sont définis comme à la revendication 1, la réaction d'un composé de formule générale (I) dans laquelle X représente le groupe carbonyle avec un composé de formule générale :
NH₂OR¹ (Va)
NH₂N(R¹)₂ (Vb)
ou
NH₂N(R¹)CON(R¹)₂ (Vc)
où R¹ est défini comme à la revendication 1 ou avec un de ses sels d'addition d'acides ;
(E) lorsque R représente un radical méthyle et X représente le groupe carbonyle ou un groupe de formule 〉C = NOR¹ ou 〉C = NN(R¹)₂ défini comme à la revendication 1 et Y et Hét sont définis comme à la revendication 1, la réaction de la méthylamine avec un dithioester de formule générale : 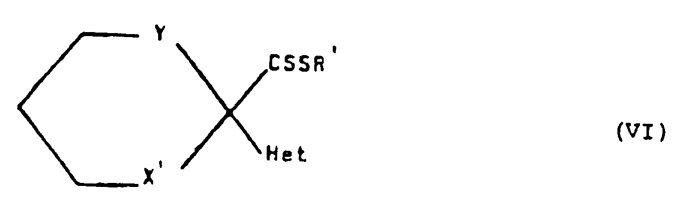 dans laquelle R' représente un radical alkyle à chaîne droite ou ramifiée en C₁-C₄ ou un radical benzyle ou carboxyméthyle, X' est défini comme précédemment et Y et Hét sont définis comme à la revendication 1 ;
éventuellement suivie de l'étape de conversion du dérivé de thioformamide ainsi obtenu en un de ses sels acceptables en pharmacie.

9. Une composition pharmaceutique qui comprend un dérivé de thioformamide de formule générale (I) définie comme à la revendication 1 ou un de ses sels acceptables en pharmacie en association avec un support ou un revêtement acceptable en pharmacie.

10. Un composé de formule générale (III) décrit à la revendication 8, dans lequel Y et Hét sont définis comme à la revendication 1 et X' est défini comme à la revendication 8 avec la condition que Y ne soit pas une liaison directe lorsque Hét représente un groupe 2-pyrazinyle facultativement substitué et que Y ne représente pas le radical méthylène lorsque Hét représente un groupe 3-indolyle facultativement substitué.

## Revendications (Revendications pour l'(les) Etat(s) contractant(s) suivant(s): ES)

1. Un procédé pour la préparation d'un dérivé de thioformamide de formule générale : 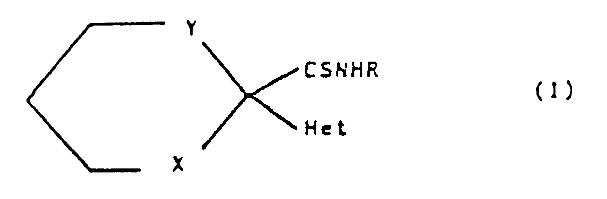 dans laquelle R représente un groupe alkyle à chaîne droite ou ramifiée en C₁-C₄, Hét représente un radical hétérocyclique aromatique contenant 1 ou 2 atomes d'azote (éventuellement substitué par un radical alkyle ou alcoxy à chaîne droite ou ramifiée en C₁-C₄ ou par un atome d'halogène), choisi parmi les groupes 3-pyridyle, 4-isoquinoléyle, 3-tétrahydroquinoléyle, 3-quinoléyle, 4-pyridazinyle, 5-pyrimidyle, 5-thiazolyle, 5-thiéno[2,3-b]pyridyle, 2-pyrazinyle, 3-indolyle et 6-thiéno[3,2-b]pyridyle, Y représente un radical éthylène ou méthylène ou une liaison de valence et X représente un groupe carbonyle ou hydroxyméthylène ou un groupe de formule : 〉C = NOR¹, 〉C = NN(R¹)₂ ou 〉C = NN(R¹)CON(R¹)₂ dans lesquelles les symboles R¹, qui peuvent être identiques ou différents, représentent chacun un atome d'hydrogène ou un radical alkyle à chaîne droite ou ramifiée en C₁-C₄, non substitué ou substitué par un ou plusieurs substituants choisis parmi les groupes alcényle en C₂-C₄, carboxy, (alcoxy en C₂-C₅)-carbonyle, hydroxy, alcoxy en C₁-C₄, carbamoyle (non substitué ou substitué par un ou deux groupes alkyles en C₁-C₄), amino, alkylamino en C₁-C₄ et di(alkyl en C₁-C₄)amino ou représente un radical benzyle, phénéthyle, 1-naphtylméthyle, 2-naphtylméthyle ou 3-pyridylméthyle, chacun d'eux pouvant être substitué au noyau par un ou plusieurs atomes d'halogènes ou groupes hydroxy, alkyles en C₁-C₄, alcoxy en C₁-C₄ (le groupe alcoxy étant substitué ou non comme pour les groupes alkyles représentés par R¹), cyano, nitro, trifluorométhyle, carboxy, alkylamino en C₁-C₄, alcanoylamino en C₂-C₅, (alcoxy en C₂-C₅)carbonyle, ou bien deux substituants R¹ sur le même atome d'azote peuvent former ensemble un radical alkylène à chaîne droite ou ramifiée en C₄-C₆ dans la chaîne, qui est substitué ou non comme défini pour les groupes alkyles représentés par R₁, et de ses sels acceptables en pharmacie, procédé qui comprend :
(A) lorsque X représente le groupe carbonyle ou un groupe de formule 〉C = NOR¹ ou 〉C = NN(R¹)₂ dans lesquelles R¹, Y, R et Hét sont définis comme précédemment, la réaction d'un composé de formule générale : 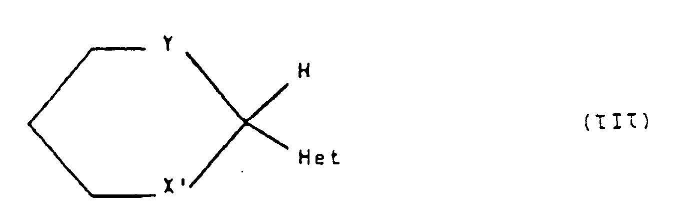 dans laquelle X' représente le groupe carbonyle ou un groupe de formule 〉C = NOR¹ ou 〉C = NN(R¹)₂ défini comme précédemment et Y et Hét sont définis comme précédemment avec un isothiocyanate de formule générale :
R - N = C = S (IV)
dans laquelle R représente un radical alkyle à chaîne droite ou ramifiée en C₁-C₄ ;
(B) lorsque X représente le groupe carbonyle, la réaction stéréosélective d'un mélange d'énantiomères de formule générale (III) dans laquelle Y et Hét sont définis comme précédemment et X' représente le groupe carbonyle avec un agent auxiliaire chiral et la réaction du produit obtenu avec un composé de formule générale :
R - N = C = S (IV)
dans laquelle R est défini comme précédemment suivie d'élimination de l'agent auxiliaire chiral ;
(C) lorsque X représente le groupe hydroxyéthylène et Y, R et Hét sont définis comme précédemment, la réduction d'un composé de formule générale (I) dans laquelle X représente un groupe carbonyle et Y, R et Hét sont définis comme précédemment ;
(D) lorsque X représente un groupe de formule 〉C = NOR¹, 〉C = NN(R¹)₂ ou 〉C = NN(R¹)CON(R¹)₂ défini comme précédemment et Y, R et Hét sont définis comme précédemment, la réaction d'un composé de formule générale (I) dans laquelle X représente le groupe carbonyle avec un composé de formule générale :
NH₂OR¹ (Va)
NH₂N(R¹)₂ (Vb)
ou
NH₂N(R¹)CON(R¹)₂ (Vc)
où R¹ est défini comme précédemment ou avec un de ses sels d'addition d'acides ;
(E) lorsque R représente un radical méthyle et X représente le groupe carbonyle ou un groupe de formule 〉C = NOR¹ ou 〉C = NN(R¹)₂ défini comme précédemment et Y et Hét sont définis comme précédemment, la réaction de la méthylamine avec un dithioester de formule générale : 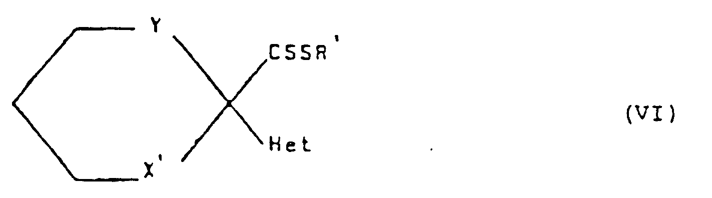 dans laquelle R' représente un radical alkyle à chaîne droite ou ramifiée en C₁-C₄ ou un radical benzyle ou carboxyméthyle, X', Y et Hét sont définis comme précédemment ;
éventuellement suivie de l'étape de conversion du dérivé de thioformamide ainsi obtenu en un de ses sels acceptables en pharmacie.

2. Un procédé selon la revendication 1, dans lequel Y représente un radical méthylène ou une liaison de valence.

3. Un procédé selon la revendication 1 ou 2, dans lequel X représente le groupe carbonyle ou un groupe de formule 〉C = NOR¹ dans laquelle R¹ est défini comme à la revendication 1.

4. Un procédé selon la revendication 1, 2 ou 3, dans lequel R¹ représente un radical alkyle à chaîne droite ou ramifiée en C₁-C₄ qui est substitué ou non comme défini dans la revendication 1, ou bien représente un radical benzyle, phénéthyle, 1-naphtylméthyle, 2-naphtylméthyle ou 3-pyridylméthyle, chacun d'eux pouvant être substitué au noyau comme défini à la revendication 1.

5. Un procédé selon l'une quelconque des revendications précédentes dans lequel Hét représente un groupe 3-pyridyle, 6-chloro-3-pyridyle, 5-bromo-3-pyridyle, 3-quinoléyle, 4-isoquinoléyle ou 5-pyrimidyle.

6. Un procédé selon l'une quelconque des revendications 1, 2, 4 et 5 pour la préparation d'un composé de formule générale : 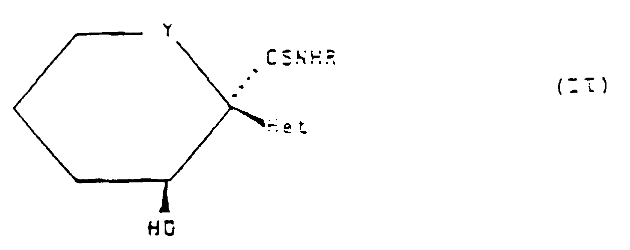 dans laquelle R, Hét et Y sont tels que définis dans l'une quelconque des revendications 1 à 5, dans lequel le groupe hydroxy est dans la position trans par rapport au groupe -CSNHR.

7. Un procédé selon la revendication 1, pour la préparation d'un dérivé de thioformamide choisi parmi les suivants :
(±)-N-méthyl-2-oxo-1-(3-pyridyl)cyclohexanecarbothioamide,
(±)-N-méthyl-2-oxo-1-(3-quinoléyl)cyclohexanecarbothioamide,
(±)-N-méthyl-2-oxo-1-(3-pyridyl)cycloheptanecarbothioamide,
(±)-trans-N-méthyl-2-hydroxy-1-(3-pyridyl)cyclohexanecarbothioamide,
(±)-cis-N-méthyl-2-hydroxy-1-(3-pyridyl)cyclohexanecarbothioamide,
(±)-anti-N-méthyl-2-hydroxyimino-1-(3-pyridyl)cyclohexanecarbothioamide,
(±)-anti-N-méthyl-2-méthoxyimino-1-(3-pyridyl)cyclohexanecarbothioamide,
(±)-anti-N-méthyl-2-benzyloxyimino-1-(3-pyridyl)cyclohexanecarbothioamide,
(±)-anti-N-méthyl-2-hydroxyimino-1-(3-quinoléyl)cyclohexanecarbothioamide,
(2S)-anti-2-(méthoxyméthyl)--1-[2'-(3-quinoléyl)-2'-méthylthiocarbamoylcyclohexylidèneamino]pyrrolidine,
(2S)-anti-2-(méthoxyméthyl)-1-[2'-(3-pyridyl)-2'-méthylthiocarbamoylcyclohexylidèneamino]-pyrrolidine,
(±)-anti-N-méthyl-2-(2-diméthylaminoéthoxyimino)-1-(3-pyridyl)cyclohexanecarbothioamide,
(±)-anti-N-méthyl-2-(2-aminoéthoxyimino)-1-(3-pyridyl)cyclohexanecarbothioamide,
(±)-anti-N-méthyl-2-méthoxycarbonylméthoxyimino-1-(3-pyridyl)cyclohexanecarbothioamide,
(±)-anti-N-méthyl-2-carbamoylméthoxyimino-1-(3-pyridyl)cyclohexanecarbothioamide,
(±)-anti-N-méthyl-2-(2,3-dihydroxypropoxyimino)-1-(3-pyridyl)cyclohexanecarbothioamide,
(±)-anti-N-méthyl-2-(2-hydroxyéthoxyimino)-1-(3-pyridyl)cyclohexanecarbothioamide,
(±)-N-méthyl-2-oxo-1-(6-chloro-3-pyridyl)cyclohexanecarbothioamide,
(±)-N-méthyl-2-oxo-1-(5-bromo-3-pyridyl)cyclohexanecarbothioamide,
(±)-N-éthyl-2-oxo-1-(3-pyridyl)cyclohexanecarbothioamide,
(±)-N-éthyl-2-oxo-1-(3-quinoléyl)cyclohexanecarbothioamide,
(±)-anti-N-méthyl-2-diméthylhydrazono-1-(3-quinoléyl)cyclohexanecarbothioamide,
(±)-anti-N-méthyl-2-diéthylhydrazono-1-(3-quinoléyl)cyclohexanecarbothioamide,
(±)-anti-N-méthyl-2-benzyloxyimino-1-(3-quinoléyl)cyclohexanecarbothioamide,
(±)-anti-N-méthyl-2-méthoxyimino-1-(3-quinoléyl)cyclohexanecarbothioamide,
(±)-anti-N-méthyl-2-éthoxyimino-1-(3-quinoléyl)cyclohexanecarbothioamide,
(±)-anti-N-méthyl-2-butoxyimino-1-(3-quinoléyl)cyclohexanecarbothioamide,
(±)-anti-N-méthyl-2-(3-isopropylamino-2-hydroxypropoxyimino)-1-(3-quinoléyl)cyclohexanecarbothioamide,
(±)-anti-N-méthyl-2-t.butoxyimino-1-(3-quinoléyl)cyclohexanecarbothioamide,
(±)-anti-N-méthyl-2-(2-propényl)oxyimino-1-(3-quinoléyl)cyclohexanecarbothioamide,
(±)-anti-N-méthyl-2-(β-naphtyl)méthoxyimino-1-(3-quinoléyl)cyclohexanecarbothioamide,
(±)-anti-N-méthyl-2-phénéthyloxyimino-1-(3-quinoléyl)cyclohexanecarbothioamide,
(±)-anti-N-méthyl-2-(α-naphtyl)méthoxyimino-1-(3-quinoléyl)cyclohexanecarbothioamide,
(±)-anti-N-méthyl-2-(3―t.butylamino-2-hydroxypropoxyimino-1-(3-quinoléyl)cyclohexanecarbothioamide,
(±)-anti-N-méthyl-2-isopropoxyimino-1-(3-quinoléyl)cyclohexanecarbothioamide,
(±)-anti-N-méthyl-2-(4-hydroxybenzyloxyimino-1-(3-quinoléyl)cyclohexanecarbothioamide,
(±)-anti-N-méthyl-2-(4-fluorobenzloxyimino)-1-(3-quinoléyl)cyclohexanecarbothioamide,
(±)-anti-N-méthyl-2-(4-fluorobenzyloxyimino)-1-(3-pyridyl)cyclohexanecarbothioamide,
(±)-anti-N-méthyl-2-(2,3,4,5,6-pentafluorobenzyloxyimino)-1-(3-pyridyl)cyclohexanecarbothioamide,
(±)-anti-N-méthyl-2-isopropoxyimino-1-(3-pyridyl)cyclohexanecarbothioamide,
(±)-anti-N-methyl-2-t.butoxyimino-1-(3-pyridyl)cyclohexanecarbothioamide,
(±)-anti-N-méthyl-2-[4-(3-t.butylamino-2-hydropropoxy)benzyloxyimino]-1-(3-pyridyl)cyclohexanecarbothioamide,
(±)-anti-N-méthyl-2-(4-hydroxybenzyloxyimino)-1-(3-pyridyl)cyclohexanecarbothioamide,
(±)-anti-N-méthyl-2-(3-fluorobenzyloxyimino)-1-(3-pyridyl)cyclohexanecarbothioamide,
(±)-anti-N-méthyl-2-(2-fluorobenzyloxyimino)-1-(3-pyridyl)cyclohexanecarbothioamide,
(±)-anti-N-méthyl-2-(3-pyridylméthoxyimino)-1-(3-pyridyl)cyclohexanecarbothioamide,
(±)-anti-N-méthyl-2-(4-nitrobenzyloxyimino)-1-(3-pyridyl)cyclohexanecarbothioamide,
(±)-anti-N-méthyl-2-(4-cyanobenzyloxyimino)-1-(3-pyridyl)cyclohexanecarbothioamide,
(±)-anti-N-méthyl-2-(3,4-difluorobenzyloxyimino)-1-(3-pyridyl)cyclohexanecarbothioamide,
(±)-anti-N-méthyl-2-(4-méthoxybenzyloxyimino)-1-(3-pyridyl)cyclohexanecarbothioamide,
(±)-anti-N-méthyl-2-(2-trifiuorométhylbenzyloxyimino)-1-(3-pyridyl)cyclohexanecarbothioamide,
(±)-N-méthyl-2-oxo-1-(4-isoquinoléyl)cyclohexanecarbothioamide,
(±)-anti-N-méthyl-2--benzyloxyimino-1-(4-isoquinoléyl)cyclohexanecarbothioamide,
(±)-N-méthyl-2-oxo-1-(5-pyrimidyl)cyclohexanecarbothioamide,
(±)-N-méthyl-2-oxo-1-(3-pyridyl)cyclopentanecarbothioamide,
(±)-anti-N-méthyl-2-(4-fluorobenzyloxyimino)-1-(3-pyridyl)cyclopentanecarbothioamide,
(±)-anti-N-méthyl-2-méthoxyimino-1-(3-pyridyl)cyclopentanecarbothioamide et
(±)-anti-N-méthyl-2-benzyloxyimino-1-(3-pyridyl)cyclopentanecarbothioamide et leurs sels acceptables en pharmacie.

8. Un procédé selon l'une quelconque des revendications précédentes, suivi de l'étape de formulation d'un dérivé de thioformamide de formule générale (I) définie comme à la revendication 1 ou d'un de ses sels acceptables en pharmacie avec un support ou un revêtement acceptable en pharmacie.

## Revendications (Revendications pour l'(les) Etat(s) contractant(s) suivant(s): GR)

1. Un procédé pour la préparation d'un dérivé de thioformamide de formule générale : 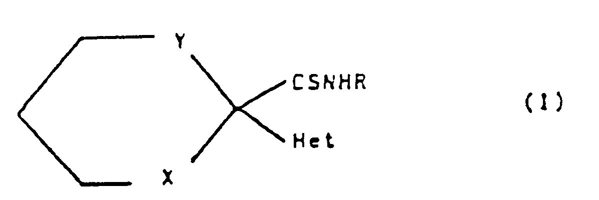 dans laquelle R représente un groupe alkyle à chaîne droite ou ramifiée en C₁-C₄, Hét représente un radical hétérocyclique aromatique contenant 1 ou 2 atomes d'azote (éventuellement substitué par un radical alkyle ou alcoxy à chaîne droite ou ramifiée en C₁-C₄ ou par un atome d'halogène), choisi parmi les groupes 3-pyridyle, 4-isoquinoléyle, 3-tétrahydroquinoléyle, 3-quinoléyle, 4-pyridazinyle, 5-pyrimidyle, 5-thiazolyle, 5-thiéno[2,3-b]pyridyle, 2-pyrazinyle, 3-indolyle et 6-thiéno[3,2-b]pyridyle, Y représente un radical éthylène ou méthylène ou une liaison de valence et X représente un groupe carbonyle ou hydroxyméthylène ou un groupe de formule : 〉C = NOR¹, 〉C = NN(R¹)₂ ou 〉C = NN(R¹)CON(R¹)₂ dans lesquelles les symboles R¹, qui peuvent être identiques ou différents, représentent chacun un atome d'hydrogène ou un radical alkyle à chaîne droite ou ramifiée en C₁-C₄, non substitué ou substitué par un ou plusieurs substituants choisis parmi les groupes alcényle en C₂-C₄, carboxy, (alcoxy en C₂-C₅)-carbonyle, hydroxy, alcoxy en C₁-C₄, carbamoyle (non substitué ou substitué par un ou deux groupes alkyles en C₁-C₄), amino, alkylamino en C₁-C₄ et di(alkyl en C₁-C₄)amino ou représente un radical benzyle, phénéthyle, 1-naphtylméthyle, 2-naphtylméthyle ou 3-pyridylméthyle, chacun d'eux pouvant être substitué au noyau par un ou plusieurs atomes d'halogènes ou groupes hydroxy, alkyles en C₁-C₄, alcoxy en C₁-C₄ (le groupe alcoxy étant substitué ou non comme pour les groupes alkyles représentés par R¹), cyano, nitro, trifluorométhyle, carboxy, alkylamino en C₁-C₄, alcanoylamino en C₂-C₅, (alcoxy en C₂-C₅)carbonyle, ou bien deux substituants R¹ sur le même atome d'azote peuvent former ensemble un radical alkylène à chaîne droite ou ramifiée en C₄-C₆ dans la chaîne, qui est substitué ou non comme défini pour les groupes alkyles représentés par R₁, et de ses sels acceptables en pharmacie, procédé qui comprend :
(A) lorsque X représente le groupe carbonyle ou un groupe de formule 〉C = NOR¹ ou 〉C = NN(R¹)₂ dans lesquelles R¹, Y, R et Hét sont définis comme précédemment, la réaction d'un composé de formule générale : 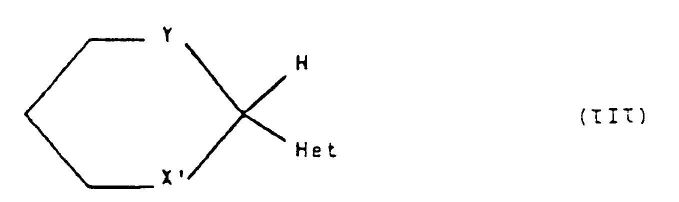 dans laquelle X' représente le groupe carbonyle ou un groupe de formule 〉C = NOR¹ ou 〉C = NN(R¹)₂ défini comme précédemment et Y et Hét sont définis comme précédemment avec un isothiocyanate de formule générale :
R - N = C = S (IV)
dans laquelle R représente un radical alkyle à chaîne droite ou ramifiée en C₁-C₄ ;
(B) lorsque X représente le groupe carbonyle, la réaction stéréosélective d'un mélange d'énantiomères de formule générale (III) dans laquelle Y et Hét sont définis comme précédemment et X' représente le groupe carbonyle avec un agent auxiliaire chiral et la réaction du produit obtenu avec un composé de formule générale :
R - N = C = S (IV)
dans laquelle R est défini comme précédemment suivie d'élimination de l'agent auxiliaire chiral ;
(C) lorsque X représente le groupe hydroxyéthylène et Y, R et Hét sont définis comme précedemment, la réduction d'un composé de formule générale (I) dans laquelle X représente un groupe carbonyle et Y, R et Hét sont définis comme précédemment ;
(D) lorsque X représente un groupe de formule 〉C = NOR¹, 〉C = NN(R¹)₂ ou 〉C = NN(R¹)CON(R¹)₂ défini comme précédemment et Y, R et Hét sont définis comme précédemment, la réaction d'un composé de formule générale (I) dans laquelle X représente le groupe carbonyle avec un composé de formule générale :
NH₂OR¹ (Va)
NH₂N(R¹)₂ (Vb)
ou
NH₂N(R¹)CON(R¹)₂ (Vc)
où R¹ est défini comme précédemment ou avec un de ses sels d'addition d'acides ;
(E) lorsque R représente un radical méthyle et X représente le groupe carbonyle ou un groupe de formule 〉C = NOR¹ ou 〉C = NN(R¹)₂ défini comme précédemment et Y et Hét sont définis comme précédemment, la réaction de la méthylamine avec un dithioester de formule générale : 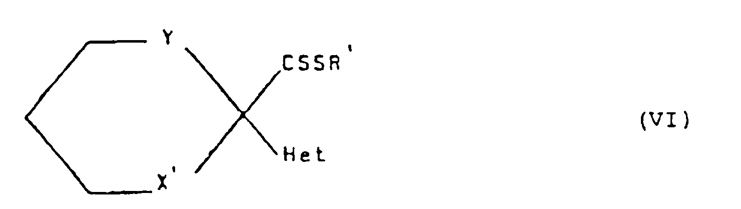 dans laquelle R' représente un radical alkyle à chaîne droite ou ramifiée en C₁-C₄ ou un radical benzyle ou carboxyméthyle, X', Y et Hét sont définis comme précédemment ;
éventuellement suivie de l'étape de conversion du dérivé de thioformamide ainsi obtenu en un de ses sels acceptables en pharmacie.

2. Un procédé selon la revendication 1, dans lequel Y représente un radical méthylène ou une liaison de valence.

3. Un procédé selon la revendication 1 ou 2, dans lequel X représente le groupe carbonyle ou un groupe de formule 〉C = NOR¹ dans laquelle R¹ est défini comme à la revendication 1.

4. Un procédé selon la revendication 1, 2 ou 3, dans lequel R¹ représente un radical alkyle à chaîne droite ou ramifiée en C₁-C₄ qui est substitué ou non comme défini dans la revendication 1, ou bien représente un radical benzyle, phénéthyle, 1-naphtylméthyle, 2-naphtylméthyle ou 3-pyridylméthyle, chacun d'eux pouvant être substitué au noyau comme défini à la revendication 1.

5. Un procédé selon l'une quelconque des revendications précédentes dans lequel Hét représente un groupe 3-pyridyle, 6-chloro-3-pyridyle, 5-bromo-3-pyridyle, 3-quinoléyle, 4-isoquinoléyle ou 5-pyrimidyle.

6. Un procédé selon l'une quelconque des revendications 1, 2, 4 et 5 pour la préparation d'un composé de formule générale : 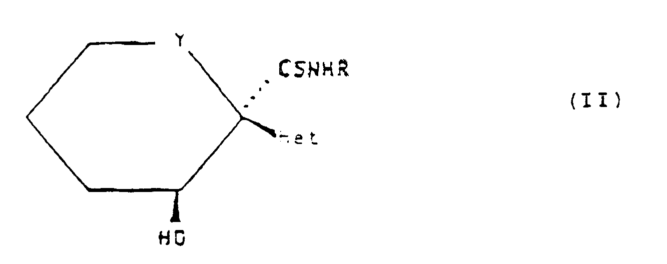 dans laquelle R, Hét et Y sont tels que définis dans l'une quelconque des revendications 1 à 5, dans lequel le groupe hydroxy est dans la position trans par rapport au groupe -CSNHR.

7. Un procédé selon la revendication 1, pour la préparation d'un dérivé de thioformamide choisi parmi les suivants :
(±)-N-méthyl-2-oxo-1-(3-pyridyl)cyclohexanecarbothioamide,
(±)-N-méthyl-2-oxo-1-(3-quinoléyl)cyclohexanecarbothioamide,
(±)-N-méthyl-2-oxo-1-(3-pyridyl)cycloheptanecarbothioamide,
(±)-trans-N-méthyl-2-hydroxy-1-(3-pyridyl)cyclohexanecarbothioamide,
(±)-cis-N-méthyl-2-hydroxy-1-(3-pyridyl)cyclohexanecarbothioamide,
(±)-anti-N-méthyl-2-hydroxyimino-1-(3-pyridyl)cyclohexanecarbothioamide,
(±)-anti-N-méthyl-2-méthoxyimino-1-(3-pyridyl)cyclohexanecarbothioamide,
(±)-anti-N-méthyl-2-benzyloxyimino-1-(3-pyridyl)cyclohexanecarbothioamide,
(±)-anti-N-méthyl-2-hydroxyimino-1-(3-quinoléyl)cyclohexanecarbothioamide,
(2S)-anti-2-(méthoxyméthyl)--1-[2'-(3-quinoléyl)-2'-méthylthiocarbamoylcyclohexylidèneamino]pyrrolidine,
(2S)-anti-2-(méthoxyméthyl)-1-[2'-(3-pyridyl)-2'-méthylthiocarbamoylcyclohexylidèneamino]-pyrrolidine,
(±)-anti-N-méthyl-2-(2-diméthylaminoéthoxyimino)-1-(3-pyridyl)cyclohexanecarbothioamide,
(±)-anti-N-méthyl-2-(2-aminoéthoxyimino)-1-(3-pyridyl)cyclohexanecarbothioamide,
(±)-anti-N-méthyl-2-méthoxycarbonylméthoxyimino-1-(3-pyridyl)cyclohexanecarbothioamide,
(±)-anti-N-méthyl-2-carbamoylméthoxyimino-1-(3-pyridyl)cyclohexanecarbothioamide,
(±)-anti-N-méthyl-2-(2,3-dihydroxypropoxyimino)-1-(3-pyridyl)cyclohexanecarbothioamide,
(±)-anti-N-méthyl-2-(2-hydroxyéthoxyimino)-1-(3-pyridyl)cyclohexanecarbothioamide,
(±)-N-méthyl-2-oxo-1-(6-chloro-3-pyridyl)cyclohexanecarbothioamide,
(±)-N-méthyl-2-oxo-1-(5-bromo-3-pyridyl)cyclohexanecarbothioamide,
(±)-N-éthyl-2-oxo-1-(3-pyridyl)cyclohexanecarbothioamide,
(±)-N-éthyl-2-oxo-1-(3-quinoléyl)cyclohexanecarbothioamide,
(±)-anti-N-méthyl-2-diméthylhydrazono-1-(3-quinoléyl)cyclohexanecarbothioamide,
(±)-anti-N-méthyl-2-diéthylhydrazono-1-(3-quinoléyl)cyclohexanecarbothioamide,
(±)-anti-N-méthyl-2-benzyloxyimino-1-(3-quinoléyl)cyclohexanecarbothioamide,
(±)-anti-N-méthyl-2-méthoxyimino-1-(3-quinoléyl)cyclohexanecarbothioamide,
(±)-anti-N-méthyl-2-éthoxyimino-1-(3-quinoléyl)cyclohexanecarbothioamide,
(±)-anti-N-méthyl-2-butoxyimino-1-(3-quinoléyl)cyclohexanecarbothioamide,
(±)-anti-N-méthyl-2-(3-isopropylamino-2-hydroxypropoxyimino)-1-(3-quinoléyl)cyclohexanecarbothioamide,
(±)-anti-N-méthyl-2-t.butoxyimino-1-(3-quinoléyl)cyclohexanecarbothioamide,
(±)-anti-N-méthyl-2-(2-propényl)oxyimino-1-(3-quinoléyl)cyclohexanecarbothioamide,
(±)-anti-N-méthyl-2-(β-naphtyl)méthoxyimino-1-(3-quinoléyl)cyclohexanecarbothioamide,
(±)-anti-N-méthyl-2-phénéthyloxyimino-1-(3-quinoléyl)cyclohexanecarbothioamide,
(±)-anti-N-méthyl-2-(α-naphtyl)méthoxyimino-1-(3-quinoléyl)cyclohexanecarbothioamide,
(±)-anti-N-méthyl-2-(3-t.butylamino-2-hydroxypropoxyimino-1-(3-quinoléyl)cyclohexanecarbothioamide,
(±)-anti-N-méthyl-2-isopropoxyimino-1-(3-quinoléyl)cyclohexanecarbothioamide,
(±)-anti-N-méthyl-2-(4-hydroxybenzyloxyimino-1-(3-quinoléyl)cyclohexanecarbothioamide,
(±)-anti-N-méthyl-2-(4-fluorobenzyloxyimino)-1-(3-quinoléyl)cyclohexanecarbothioamide,
(±)-anti-N-méthyl-2-(4-fluorobenzyloxyimino)-1-(3-pyridyl)cyclohexanecarbothioamide,
(±)-anti-N-méthyl-2-(2,3,4,5,6-pentafluorobenzyloxyimino-1-(3-pyridyl)cyclohexanecarbothioamide,
(±)-anti-N-méthyl-2-isopropoxyimino-1-(3-pyridyl)cyclohexanecarbothioamide,
(±)-anti-N-méthyl-2-t.butoxyimino-1-(3-pyridyl)cyclohexanecarbothioamide,
(±)-anti-N-méthyl-2-[4-(3-t.butylamino-2-hydroxypropoxy)benzyloxyimino]-1-(3-pyridyl)cyclohexanecarbothioamide,
(±)-anti-N-méthyl-2-(4-hydroxybenzyloxyimino)-1-(3-pyridyl)cyclohexanecarbothioamide,
(±)-anti-N-méthyl-2-(3-fluorobenzyloxyimino)-1-(3-pyridyl)cyclohexanecarbothioamide,
(±)-anti-N-méthyl-2-(2-fluorobenzyloxyimino)-1-(3-pyridyl)cyclohexanecarbothioamide,
(±)-anti-N-méthyl-2-(3-pyridylméthoxyimino)-1-(3-pyridyl)cyclohexanecarbothioamide,
(±)-anti-N-méthyl-2-(4-nitrobenzyloxyimino)-1-(3-pyridyl)cyclohexanecarbothioamide,
(±)-anti-N-méthyl-2-(4-cyanobenzyloxyimino)-1-(3-pyridyl)cyclohexanecarbothioamide,
(±)-anti-N-méthyl-2-(3,4-difluorobenzyloxyimino)-1-(3-pyridyl)cyclohexanecarbothioamide,
(±)-anti-N-méthyl-2-(4-méthoxybenzyloxyimino)-1-(3-pyridyl)cyclohexanecarbothioamide,
(±)-anti-N-méthyl-2-(2-trifluorométhylbenzyloxyimino)-1-(3-pyridyl)cyclohexanecarbothioamide,
(±)-N-méthyl-2-oxo-1-(4-isoquinoléyl)cyclohexanecarbothioamide,
(±)-anti-N-méthyl-2--benzyloxyimino-1-(4-isoquinoléyl)cyclohexanecarbothioamide,
(±)-N-méthyl-2-oxo-1-(5-pyrimidyl)cyclohexanecarbothioamide,
(±)-N-méthyl-2-oxo-1-(3-pyridyl)cyclopentanecarbothioamide,
(±)-anti-N-méthyl-2-(4-fluorobenzyloxyimino)-1-(3-pyridyl)cyclopentanecarbothioamide,
(±)-anti-N-méthyl-2-méthoxyimino-1-(3-pyridyl)cyclopentanecarbothioamide et
(±)-anti-N-méthyl-2-benzyloxyimino-1-(3-pyridyl)cyclopentanecarbothioamide et leurs sels acceptables en pharmacie.

8. Un procédé selon l'une quelconque des revendications précédentes, suivi de l'étape de formulation d'un dérivé de thioformamide de formule générale (I) définie comme à la revendication 1 ou d'un de ses sels acceptables en pharmacie avec un support ou un revêtement acceptable en pharmacie.

9. Un composé de formule générale (III) décrit à la revendication 1, dans lequel X', Y et Hét sont définis comme à la revendication 1 avec la condition que Y ne soit pas une liaison directe lorsque Hét représente un groupe 2-pyrazinyle facultativement substitué et que Y ne représente pas le radical méthylène lorsque Hét représente un groupe 3-indolyle facultativement substitué.
